(19) Europäisches Patentamt
European Patent Office
Office européen des brevets

(11) EP 2 086 643 B1

(12) # EUROPEAN PATENT SPECIFICATION

(45) Date of publication and mention of the grant of the patent:
**23.03.2011 Bulletin 2011/12**

(21) Application number: **07822140.5**

(22) Date of filing: **01.11.2007**

(51) Int Cl.:
***A61P 25/18*** *(2006.01)* ***C07D 471/04*** *(2006.01)*
***C07D 491/052*** *(2006.01)* ***A61K 31/416*** *(2006.01)*
***A61K 31/4162*** *(2006.01)*

(86) International application number:
**PCT/EP2007/061794**

(87) International publication number:
**WO 2008/053031 (08.05.2008 Gazette 2008/19)**

(54) **COMPOUNDS WHICH POTENTIATE AMPA RECEPTOR AND USES THEREOF IN MEDICINE**

VERBINDUNGEN, DIE DEN AMPA-REZEPTOR VERSTÄRKEN, UND DEREN ANWENDUNG IN DER MEDIZIN

COMPOSÉS DE POTENTIALISATION DU RÉCEPTEUR AMPA ET UTILISATIONS EN MÉDECINE

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**HR**

(30) Priority: **03.11.2006 GB 0621967**
**05.09.2007 GB 0717262**

(43) Date of publication of application:
**12.08.2009 Bulletin 2009/33**

(73) Proprietor: **Glaxo Group Limited**
**Greenford**
**Middlesex**
**UB6 0NN (GB)**

(72) Inventors:
- **BRADLEY, Daniel, Marcus**
**Harlow Essex CM19 5AW (GB)**
- **CHAN, Wai, Ngor**
**Harlow Essex CM19 5AW (GB)**
- **THEWLIS, Kevin, Michael**
**Harlow Essex CM19 5AW (GB)**
- **WARD, Simon, Edward**
**Harlow Essex CM19 5AW (GB)**

(74) Representative: **Sewell, Richard Charles et al**
**GlaxoSmithKline**
**Corporate Intellectual Property**
**CN925.1**
**980 Great West Road**
**Brentford, Middlesex TW8 9GS (GB)**

(56) References cited:
**WO-A-2006/015828** **ES-A1- 2 159 479**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 086 643 B1

## Description

This invention relates to novel compounds which potentiate the glutamate receptor. The invention also relates to the use of the compounds in treating diseases and conditions mediated by potentiation of the glutamate receptor, compositions containing the derivatives and processes for their preparation.

Glutamate receptors, which mediate the majority of fast excitatory neurotransmission in the mammalian central nervous system (CNS), are activated by the excitatory amino acid, L-glutamate (for review see Watkins JC, Krogsgaard-Larsen P, Honore T (1990) Trends Pharmacol Sci 11: 25-33).

Glutamate receptors can be divided into two distinct families. The G-protein or second messenger-linked "metabotropic" glutamate receptor family which can be subdivided into three groups (Group I, mGlu1 and mGlu5: Group II, mGlu2 and mGlu3; Group III, mGlu4, mGlu6, mGlu7, mGlu8) based on sequence homology and intracellular transduction mechanisms (for review see Conn PJ and Pinn JP (1997) Ann Rev Pharmacol Toxicol 37: 205-237). The "ionotropic" glutamate receptor family, which directly couple to ligand-gated cation channels, can be subdivided into at least three subtypes based on depolarizing activation by selective agonists, N-methyl-D-aspartate (NMDA), α-amino-3-hydroxy-5-rnethylisoxazole-4-propionic acid (AMPA) and kainic acid (KA) (for review see Dingledine R, Borges K, Bowie, Traynelis S (1999) 51: 7-61).

Native AMPA receptors (AMPAR) exist as heterotetramers consisting of combinations of four different protein subunits (GluR1-4) (for review see Bettler B and Muller C (1995) 34: 123-139.). Receptor subunit diversity is increased further as each subunit can undergo alternative splicing of a 38 amino acid sequence in the extracellular region just before the fourth membrane spanning domain M4. Such editing results in so-called 'flip' and 'flop' receptor isoforms which differ in kinetic and pharmacological properties (Sommer B, Keinanen K, Verdoon TA, Wisden W, Burnashev N, Herb A, Kohler M, Takagi T, Sakmann B, Seeburg PH (1990) Science 249: 1580-1585).

Additionally, post-transcriptional editing of GluR2 mRNA changes a neutral glutamine to a positively charged arginine within M2. In normal humans >99% GluR2 is edited in this way. AMPAR containing such edited GluR2 subunit exhibit low calcium permeability (Burnachev N, Monyer H, Seeburg PH, Sakmann B (1992) Neuron 8: 189-198). There is a suggestion, however, that the number of AMPAR with high calcium permeability is elevated in certain disease-associated conditions (Weiss JH, and Sensi SL (2000) Trends in Neurosci 23: 365-371).

AMPAR depolarization removes voltage dependent $Mg^{2+}$ block of NMDA receptors which in turn leads to NMDA receptor activation, an integral stage in the induction of LTP (Bliss TVP, Collingridge GL (1993) Nature 361: 31-9). LTP is a physiological measure of increased synaptic strength following a repetitive stimulus or activity, such as occurs during learning.

Direct activation of glutamate receptors by agonists, in conditions where glutamate receptor function is reduced, increases the risk of excitotoxicity and additional neuronal damage. AMPAR positive allosteric modulators do not activate the receptor directly. However, when the ligand (L-glutamate or AMPA) is present AMPAR modulators increase receptor activity. Thus, AMPA receptor modulators enhance synaptic function when glutamate is released and is able to bind at post-synaptic receptor sites.

Compounds which act as AMPAR positive allosteric modulators have been shown to increase ligand affinity for the receptor (Arai A, Guidotti A, Costa E, Lynch G (1996) Neuroreport. 7: 2211-5.); reduce receptor desensitization and reduce receptor deactivation (Arai AC, Kessler M, Rogers G, Lynch G (2000) 58: 802-813) and facilitate the induction of LTP both in vitro (Arai A, Guidotti A, Costa E, Lynch G (1996) 7: 2211-5.) and in vivo (Staubli U, Perez Y, Xu F, Rogers G, Ingvar M, Stone-Elander S, Lynch G (1994) Proc Natl Acad Sci 91: 11158-11162). Such compounds also enhance the learning and performance of various cognitive tasks in rodent (Zivkovic I, Thompson DM, Bertolino M, Uzunov D, DiBella M, Costa E, Guidotti A (1995) JPET 272: 300-309, Lebrun C, Pilliere E, Lestage P (2000) Eu J Pharmacol 401: 205-212), sub-human primate (Thompson DM, Guidotti A, DiBella M, Costa E (1995) Proc Natl Acad Sci 92: 7667-7671) and man (Ingvar M, Ambros-Ingerson J, Davis M, Granger R, Kessler M, Rogers GA, Schehr RS, Lynch G (1997) Exp Neurol 146: 553-559).

WO2006/015828 discloses indenyl sulphonamides as potentiators of the glutamate receptor. ES2159479 discloses indazole sulphones as inhibitors of cycloogygenase II.

We have discovered a class of novel compounds that potentiate the AMPA receptor. According to a first aspect, the invention provides a compound of formula (I), or a salt or solvate thereof:

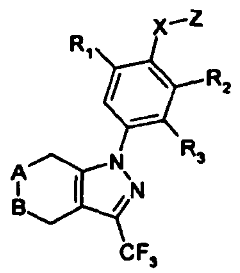

(I)

wherein

- one of A and B is $CH_2$ and the other is oxygen or $NR_4$, wherein $R_4$ is selected from hydrogen, $C_{1-2}$alkyl and $C_{1-2}$alkanoyl;
- $R_1$, $R_2$ and $R_3$ are independently selected from hydrogen and fluoro;
- X is selected from a bond, $CH_2$, C(O), $SO_2$ and $CH_2C(O)$ (wherein the carbonyl group is attached to Z);
- Z is selected from:

  (a) a C-linked pyrrolidinyl optionally substituted by $C_{1-2}$alkanoyl; and
  (b) a group $NR_5R_6$, wherein:

  - $R_5$ is hydrogen or $C_{1-4}$alkyl and $R_6$ is $C_{1-4}$alkyl, $C_{1-4}$alkylsulfonyl or Het-$C_{1-4}$alkyl wherein Het is a saturated 5 or 6 membered heterocyclic ring; or
  - $R_5$ and $R_6$ form an azetidinyl or a pyrrolidinyl group, wherein one of the carbon atoms in the azetidinyl or a pyrrolidinyl group is optionally replaced by $SO_2$, and the azetidinyl or pyrrolidinyl group is also optionally substituted by one or two groups selected from oxo, hydroxyl and halogen.

"$C_{1-4}$alkyl" refers to a straight chain or branched alkyl group containing 1, 2, 3 or 4 carbons. For example, a $C_{1-4}$alkyl group may be selected from methyl, ethyl, n-propyl, i-propyl, n-butyl, i-butyl or t-butyl. For example, $C_{1-4}$alkyl is methyl. $C_{1-2}$alkyl includes methyl and ethyl. "Me" refers to methyl. "Et" refers to ethyl.
"$C_{1-4}$alkylsulfonyl" refers to the group "$C_{1-4}$alkyl-$SO_2$-".
"$C_{1-2}$alkanoyl" refers to -C(O)H or -C(O)$CH_3$.
"Saturated 5 or 6 membered heterocyclic ring" as used in the definition of Het refers to a saturated 5 or 6 membered ring wherein one, two or three atoms in the ring are replaced by N, S or O. Examples include: pyrrolidinyl, imidazolidinyl, pyrazolidinyl, isothiazolidinyl, thiazolidinyl, tetrahydrofuranyl dioxolanyl, piperidyl, piperazinyl, morpholinyl, thiomorpholinyl, tetrahydrothienyl, dioxanyl and dithianyl.
"Oxo" refers to the group "=O". "C(O)" and "C(=O)" are interchangeable.
"Halogen" and its abbreviation "halo" refer to fluorine, chlorine, bromine or iodine.
In one embodiment, there is provided a compound of formula (I), or a salt or solvate thereof as defined above, provided that:

- when B is oxygen, $R_1$, $R_2$ and $R_3$ are all hydrogen and X is $SO_2$, then Z is not an unsubstituted N-linked pyrrolidinyl;
- when B is oxygen and $R_1$, $R_2$ and $R_3$ are all hydrogen, then Z is not an N-linked azetidinyl substituted by hydroxyl or pyrrolidinyl substituted by hydroxyl;
- when A is oxygen and $R_3$ is F, then Z is not an unsubstituted N-linked pyrrolidinyl;
- when A is NH and $R_1$ is F, then Z is not an unsubstituted N-linked pyrrolidinyl;
- when A is NMe and $R_1$, $R_2$ and $R_3$ are all hydrogen, then Z is not

  - an unsubstituted N-linked pyrrolidinyl or
  - an N-linked azetidinyl substituted with one or two halogens or
  - a group NH($C_{1-4}$alkylsulfonyl).

In one embodiment, there is provided a compound of formula (I), or a salt or solvate thereof as defined above, excluding:

1-[4-(1-pyrrolidinylsulfonyl)phenyl]-3-(trifluoromethyl)-1,4,6,7-tetrahydropyrano[4,3-c]pyrazole
1-({4-[3-(trifluoromethyl)-6,7-dihydropyrano[4,3-c]pyrazol-1(4H)-yl]phenyl}carbonyl)-3-azetidinol
1-({4-[3-(trifluoromethyl)-6,7-dihydropyrano[4,3-c]pyrazol-1(4H)-yl]phenyl}carbonyl)-3-pyrrolidinol
6-methyl-1-[4-(1-pyrrolidinylcarbonyl)phenyl]-3-(trifluoromethyl)-4,5,6,7-tetrahydro-1H-pyrazolo[3,4-c]pyridine
1-[3-fluoro-4-(1-pyrrolidinylcarbonyl)phenyl]-3-(trifluoromethyl)-4,5,6,7-tetrahydro-1H-pyrazolo[3,4-c]pyridine
1-{4-[(3,3-difluoro-1-azetidinyl)carbonyl]phenyl}-6-methyl-3-(trifluoromethyl)-4,5,6,7-tetrahydro-1H-pyrazolo[3,4-c]pyridine
N-({4-[6-methyl-3-(trifluoromethyl)-4,5,6,7-tetrahydro-1H-pyrazolo[3,4-c]pyridin-1-yl]phenyl}methyl)methanesulfonamide and
1-[2-fluoro-4-(1-pyrrolidinylcarbonyl)phenyl]-3-(trifluoromethyl)-1,4,5,7-tetrahydropyrano[3,4-c]pyrazole.

In one embodiment, A is $CH_2$ and B is oxygen or $NR_4$, wherein $R_4$ is selected from hydrogen, $C_{1-2}$alkyl and $C_{1-2}$alkanoyl.

In one embodiment, A is $CH_2$ and B is oxygen.

In one embodiment, A is $CH_2$ and B is $NR_4$, wherein $R_4$ is selected from hydrogen, $C_{1-2}$alkyl and $C_{1-2}$alkanoyl.

In one embodiment, B is $CH_2$ and A is oxygen or $NR_4$, wherein $R_4$ is selected from hydrogen, $C_{1-2}$alkyl and $C_{1-2}$alkanoyl.

In one embodiment, B is $CH_2$ and A is oxygen.

In one embodiment, B is $CH_2$ and A is $NR_4$, wherein $R_4$ is selected from hydrogen, $C_{1-2}$alkyl and $C_{1-2}$alkanoyl.

In one embodiment, $R_1$, $R_2$ and $R_3$ are all hydrogen.

In one embodiment, $R_1$ is F and $R_2$ and $R_3$ are both hydrogen.

In one embodiment, $R_1$ and $R_2$ are both hydrogen and $R_3$ is F.

In one embodiment, X is a bond.

In one embodiment, X is $CH_2$.

In one embodiment, X is C(O).

In one embodiment, X is $SO_2$.

In one embodiment, X is $CH_2C(O)$ (wherein the carbonyl group is attached to Z).

In one embodiment, Z is a C-linked pyrrolidinyl optionally substituted by $C_{1-2}$alkanoyl.

In one embodiment, Z is a C-linked pyrrolidinyl substituted by $C_{1-2}$alkanoyl.

In one embodiment, Z is a group $NR_5R_6$, wherein $R_5$ is hydrogen or $C_{1-4}$alkyl and $R_6$' is $C_{1-4}$alkyl, $C_{1-4}$alkylsulfonyl or Het-$C_{1-4}$alkyl wherein Het is a saturated 5 or 6 membered heterocyclic ring.

In one embodiment, Z is a group $NR_5R_6$, wherein $R_5$ is hydrogen or $C_{1-4}$alkyl and $R_6$ is $C_{1-4}$alkyl, $C_{1-4}$alkylsulfonyl or tetrahydrofuranyl-methyl.

In one embodiment, Z is a group $NR_5R_6$, wherein $R_5$ and $R_6$ form an azetidinyl or a pyrrolidinyl group, wherein one of the carbon atoms in the azetidinyl or a pyrrolidinyl group is optionally replaced by $SO_2$, and the azetidinyl or pyrrolidinyl group is also optionally substituted by one or two groups selected from oxo, hydroxyl and halogen.

In one embodiment, the invention provides a compound of formula (Ia), or a salt or solvate thereof:

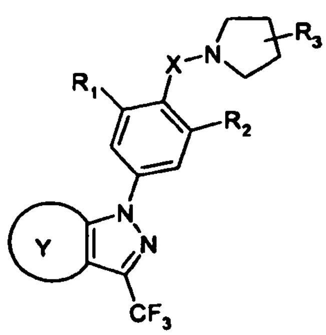

**(Ia)**

wherein

- Y is a 6-membered saturated carbocyclic group, wherein one of the carbon atoms is replaced by a heteroatom selected from oxygen and $NR_4$, wherein $R_4$ is selected from hydrogen and $C_{1-4}$alkyl;
- X is $CH_2$, $R_3$ is oxo, and $R_1$ and $R_2$ are independently selected from hydrogen and fluoro;
  or

- X is -C(=O)-, $R_3$ is hydrogen, and $R_1$ and $R_2$ are both hydrogen or one of $R_1$ and $R_2$ is hydrogen and the other is fluoro.

In one embodiment, there is provided a compound of formula (Ib) or a salt or solvate thereof:

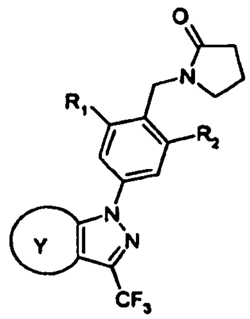

(Ib)

wherein

- Y is a 6-membered saturated carbocyclic group, wherein one of the carbon atoms is replaced by a heteroatom selected from oxygen and $NR_4$, wherein $R_4$ is selected from hydrogen and $C_{1-4}$alkyl; and
- $R_1$ and $R_2$ are independently selected from hydrogen and fluoro.

In one embodiment, the invention provides a compound of formula (Ic), or a salt or solvate thereof:

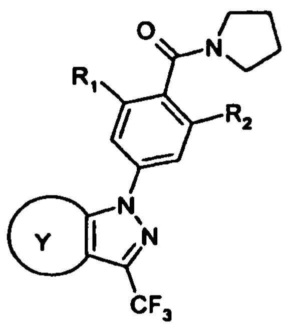

(Ic)

wherein

- Y is a 6-membered saturated carbocyclic group, wherein one of the carbon atoms is replaced by a heteroatom selected from oxygen and $NR_4$, wherein $R_4$ is selected from hydrogen and $C_{1-4}$alkyl; and
- $R_1$ and $R_2$ are both hydrogen or one of $R_1$ and $R_2$ is hydrogen and the other is fluoro.

In one embodiment, there is provided a compound of formula (Id), or a salt or solvate thereof:

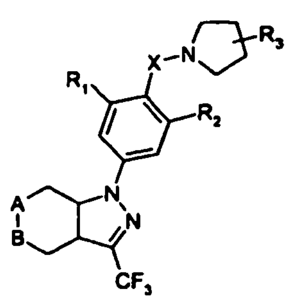

(Id)

wherein

- one of A and B is $CH_2$ and the other is selected from oxygen and $NR_4$, wherein $R_4$ is selected from hydrogen and $C_{1-4}$alkyl;
- X is $CH_2$, $R_3$ is oxo, and $R_1$ and $R_2$ are independently selected from hydrogen and fluoro;
  or
- X is -C(=O)-, $R_3$ is hydrogen, and $R_1$ and $R_2$ are both hydrogen or one of $R_1$ and $R_2$ is hydrogen and the other is fluoro.

In one embodiment, there is provided a compound of formula (Ie), or a salt or solvate thereof:

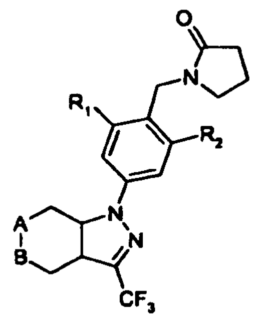

(Ie)

wherein

- one of A and B is $CH_2$ and the other is selected from oxygen and $NR_4$, wherein $R_4$ is selected from hydrogen and $C_{1-4}$alkyl; and
- $R_1$ and $R_2$ are independently selected from hydrogen and fluoro.

In one embodiment, there is provided a compound of formula (If), or a salt or solvate thereof:

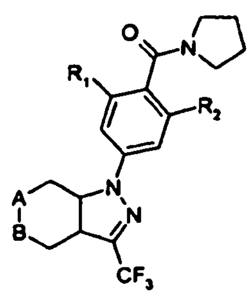

(If)

wherein

- one of A and B is $CH_2$ and the other is selected from oxygen and $NR_4$, wherein $R_4$ is selected from hydrogen and $C_{1-4}$alkyl; and
- $R_1$ and $R_2$ are both hydrogen or one of $R_1$ and $R_2$ is hydrogen and the other is fluoro.

It will be understood that, where appropriate, an embodiment described above for one part of the invention may be combined with an embodiment of another part of the invention. For the avoidance of doubt, the term independently means that where more than one substituent is selected from a number of possible substituents, those substituents may be the same or different.

All features and embodiments of formula (I) apply to formulae (Ia) to (If) mutatis mutandis. All references hereinafter to compounds of formula (I) and salts and solvates thereof are intended to include compounds of formulae (Ia) to (If) and salts and solvates thereof.

It is intended that the scope of the invention includes both enantiomers and all mixtures thereof, including but not limited to racemic mixtures, which demonstrate appropriate biological activity with reference to the procedures described herein. In one embodiment a compound of the invention in chiral form has at least 80% enantiomeric excess. In another embodiment, a compound of the invention in chiral form has at least 90% e.e., for example at least 95% e.e. In another embodiment the isomers correspond to at least 98% e.e, for example at least 99% e.e.

Examples of compounds of formula (I) include:

1-[4-(1-pyrrolidinylcarbonyl)phenyl]-3-(trifluoromethyl)-1,4,6,7-tetrahydropyrano[4,3-*c*]pyrazole
1-({4-[3-(trifluoromethyl)-6,7-dihydropyrano[4,3-*c*]pyrazol-1(4*H*)-yl]phenyl}methyl)-2-pyrrolidinone
1-[4-(1-pyrrolidinylcarbonyl)phenyl]-3-(trifluoromethyl)-1,4,5,7-tetrahydropyrano[3,4-*c*]pyrazole
1-({4-[3-(trifluoromethyl)-4,7-dihydropyrano[3,4-*c*]pyrazol-1(5*H*)-yl]phenyl}methyl)-2-pyrrolidinone
1-({4-[6-methyl-3-(trifluoromethyl)-4,5,6,7-tetrahydro-1*H*-pyrazolo[3,4-*c*]pyridin-1-yl]phenyl}methyl)-2-pyrrolidinone
1-({4-[3-(trifluoromethyl)-4,5,6,7-tetrahydro-1*H*-pyrazolo[3,4-*c*]pyridin-1-yl]phenyl}methyl)-2-pyrrolidinone
1-({2,6-difluoro-4-[3-(trifluoromethyl)-6,7-dihydropyrano[4,3-*c*]pyrazol-1(4*H*)-yl]phenyl}methyl)-2-pyrrolidinone
1-({2-fluoro-4-[3-(trifluoromethyl)-4,7-dihydropyrano[3,4-*c*]pyrazol-1(5*H*)-yl]phenyl}methyl)-2-pyrrolidinone
1-({2-fluoro-4-[6-methyl-3-(trifluoromethyl)-4,5,6,7-tetrahydro-1*H*-pyrazolo[3,4-*c*]pyridin-1-yl]phenyl}methyl)-2-pyrrolidinone
1-[3-fluoro-4-(1-pyrrolidinylcarbonyl)phenyl]-3-(trifluoromethyl)-1,4,5,7-tetrahydropyrano[3,4-*c*]pyrazole

and salts and solvates thereof.

Further examples include:

1-({4-[5-methyl-3-(trifluoromethyl)-4,5,6,7-tetrahydro-1H-pyrazolo[4,3-c]pyridin-1-yl]phenyl}methyl)-2-pyrrolidinone
1-{4-[(1,1-dioxido-2-isothiazolidinyl)methyl]phenyl}-3-(trifluoromethyl)-1,4,6,7-tetrahydropyrano[4,3-c]pyrazole
1-[4-(1-acetyl-2-pyrrolidinyl)phenyl]-3-(trifluoromethyl)-1,4,6,7-tetrahydropyrano[4,3-c]pyrazole
1-[4-(1-azetidinylcarbonyl)phenyl]-3-(trifluoromethyl)-1,4,5,7-tetrahydropyrano[3,4-c]pyrazole
1-({4-[3-(trifluoromethyl)-4,5,6,7-tetrahydro-1H-pyrazolo[4,3-c]pyridin-1-yl]phenyl}methyl)-2-pyrrolidinone
1-[4-(1-azetidinylcarbonyl)phenyl]-3-(trifluoromethyl)-1,4,6,7-tetrahydropyrano[4,3-c]pyrazole
N-ethyl-N-methyl-4-[3-(trifluoromethyl)-6,7-dihydropyrano[4,3-c]pyrazol-1(4H)-yl]benzamide
1-[4-(1-azetidinylcarbonyl)-3-fluorophenyl]-3-(trifluoromethyl)-1,4,5,7-tetrahydropyrano[3,4-c]pyrazole
1-{4-[(3,3-difluoro-1-azetidinyl)carbonyl]-3-fluorophenyl}-3-(trifluoromethyl)-1,4,5,7-tetrahydropyrano[3,4-c]pyrazole
1-{4-[(3,3-difluoro-1-azetidinyl)carbonyl]phenyl}-3-(trifluoromethyl)-1,4,6,7-tetrahydropyrano[4,3-c]pyrazole
1-{4-[(3,3-difluoro-1-azetidinyl)carbonyl]phenyl}-3-(trifluoromethyl)-1,4,5,7-tetrahydropyrano[3,4-c]pyrazole
N-({4-[3-(trifluoromethyl)-6,7-dihydropyrano[4,3-c]pyrazol-1(4H)-yl]phenyl}methyl)methanesulfonamide
1-{4-[(1,1-dioxido-2-isothiazolidinyl)methyl]phenyl}-3-(trifluoromethyl)-1,4,5,7-tetrahydropyrano[3,4-c]pyrazole
N-({4-[3-(trifluoromethyl)-4,7-dihydropyrano[3,4-c]pyrazol-1(5H)-yl]phenyl}methyl)methanesulfonamide
1-{4-[(1,1-dioxido-2-isothiazolidinyl)methyl]phenyl}-6-methyl-3-(trifluoromethyl)-4,5,6,7-tetrahydro-1H-pyrazolo[3,4-c]pyridine
1-[2-fluoro-4-(1-pyrrolidinylcarbonyl)phenyl]-3-(trifluoromethyl)-1,4,6,7-tetrahydropyrano[4,3-c]pyrazole
1-({3-fluoro-4-[3-(trifluoromethyl)-4,7-dihydropyrano[3,4-c]pyrazol-1(5H)-yl]phenyl}methyl)-2-pyrrolidinone
1-({3-fluoro-4-[3-(trifluoromethyl)-6,7-dihydropyrano[4,3-c]pyrazol-1(4H)-yl]phenyl}methyl)-2-pyrrolidinone
1-{4-[(2-oxo-1-pyrrolidinyl)methyl]phenyl}-3-(trifluoromethyl)-1,4,5,7-tetrahydro-6H-pyrazolo[3,4-c]pyridine-6-carbaldehyde
N-(tetrahydro-2-furanylmethyl)-4-[3-(trifluoromethyl)-6,7-dihydropyrano[4,3-c]pyrazol-1(4H)-yl]benzenesulfonamide
1-({2-fluoro-4-[3-(trifluoromethyl)-6,7-dihydropyrano[4,3-c]pyrazol-1(4H)-yl]phenyl}methyl)-2-pyrrolidinone
1-({2-fluoro-4-[3-(trifluoromethyl)-6,7-dihydropyrano[4,3-c]pyrazol-1(4H)-yl]phenyl}carbonyl)-3-azetidinol
1-({2-fluoro-4-[3-(trifluoromethyl)-4,5,6,7-tetrahydro-1H-pyrazolo[3,4-c]pyridin-1-yl]phenyl}methyl)-2-pyrrolidinone hdrochloride
1-{4-[(3,3-difluoro-1-azetidinyl)carbonyl]-3-fluorophenyl}-3-(trifluoromethyl)-1,4,6,7-tetrahydropyrano[4,3-c]pyrazole
1-{3-fluoro-4-[2-oxo-2-(1-pyrrolidinyl)ethyl]phenyl}-3-(trifluoromethyl)-1,4,6,7-tetrahydropyrano[4,3-c]pyrazole
1-[3-fluoro-4-(1-pyrrolidinylcarbonyl)phenyl]-3-(trifluoromethyl)-1,4,6,7-tetrahydropyrano[4,3-c]pyrazole
1-({4-[6-acetyl-3-(trifluoromethyl)-4,5,6,7-tetrahydro-1H-pyrazolo[3,4-c]pyridin-1-yl]-2-fluorophenyl}methyl)-2-pyrrolidinone

1-{3-fluoro-4-[2-oxo-2-(1-pyrrolidinyl)ethyl]phenyl}-3-(trifluoromethyl)-1,4,5,7-tetrahydropyrano[3,4-c]pyrazole
1-({2,6-difluoro-4-[3-(trifluoromethyl)-4,7-dihydropyrano[3,4-c]pyrazol-1(5H)-yl]phenyl}methyl)-2-pyrrolidinone
1-[4-(1-pyrrolidinylcarbonyl)phenyl]-3-(trifluoromethyl)-4,5,6,7-tetrahydro-1H-pyrazolo[3,4-c]pyridine hydrochloride
1-{4-[(3,3-difluoro-1-azetidinyl)carbonyl]phenyl}-3-(trifluoromethyl)-4,5,6,7-tetrahydro-1H-pyrazolo[3,4-c]pyridine
1-[4-(1-azetidinylcarbonyl)phenyl]-6-methyl-3-(trifluoromethyl)-4,5,6,7-tetrahydro-1H-pyrazolo[3,4-c]pyridine
1-({4-[6-acetyl-3-(trifluoromethyl)-4,5,6,7-tetrahydro-1H-pyrazolo[3,4-c]pyridin-1-yl]phenyl}methyl)-2-pyrrolidinone

and salts and solvates thereof.

In one embodiment, an appropriate compound of formula (I) may be in the form of a salt. For example, such a salt may be a pharmaceutically acceptable salt.

As used herein, the term "salt" refers to any salt of a compound according to the present invention prepared from an inorganic or organic acid or base, quaternary ammonium salts and internally formed salts. Pharmaceutically acceptable salts are particularly suitable for medical applications because of their greater aqueous solubility relative to the parent compounds. Such salts must clearly have a pharmaceutically acceptable anion or cation. Suitably pharmaceutically acceptable salts of the compounds of the present invention include acid addition salts formed with inorganic acids such as hydrochloric, hydrobromic, hydroiodic, phosphoric, metaphosphoric, nitric and sulfuric acids, and with organic acids, such as tartaric, acetic, trifluoroacetic, citric, malic, lactic, fumaric, benzoic, formic, propionic, glycolic, gluconic, maleic, succinic, (1S)-(-)-10-camphorsulphonic, (1S)-(+)-10-camphorsulphonic, isothionic, mucic, gentisic, isonicotinic, saccharic, glucuronic, furoic, glutamic, ascorbic, anthranilic, salicylic, phenylacetic, mandelic, embonic (pamoic), methanesulfonic, ethanesulfonic, pantothenic, stearic, sulfinilic, alginic, galacturonic and arylsulfonic, for example naphthalene-1,5-disulphonic, naphthalene-1,3-disulphonic, benzenesulfonic, and p-toluenesulfonic, acids; base addition salts formed with alkali metals and alkaline earth metals and organic bases such as N,N-benzylethylenediamine, chloroprocaine, choline, diethanolamine, ethylenediamine, meglumaine (N-methylglucamine), lysine and procaine; and internally formed salts. Salts having a non- pharmaceutically acceptable anion or cation are within the scope of the invention as useful intermediates for the preparation of pharmaceutically acceptable salts and/or for use in non-therapeutic, for example, *in vitro,* situations. The salts may have any suitable stoichiometry. For example, a salt may have 1:1 or 2:1 stoichiometry. Non-integral stoichiometry ratios are also possible.

Furthermore, some of the crystalline forms of the compounds of structure (I) may exist as polymorphs, which are included in the present invention.

Some of the compounds of this invention may be crystallised or recrystallised from solvents such as aqueous and organic solvents. In such cases solvates may be formed. This invention includes within its scope stoichiometric solvates including hydrates as well as compounds containing variable amounts of solvent (including water) where non-stoichiometric solvates (hydrates in the case of water) may be produced by processes such as lyophilisation).

Hereinafter, compounds of formula (I), their salts and their solvates defined in any aspect of the invention (except Intermediate compounds in chemical processes) are referred to as "compounds of the invention".

The compounds of the invention may exist in one or more tautomeric forms. All tautomers and mixtures thereof are included in the scope of the present invention.

Compounds of the invention may exist in the form of optical isomers, e.g. diastereoisomers and mixtures of isomers in all ratios, e.g. racemic mixtures. The invention includes all such forms, in particular the pure isomeric forms. The different isomeric forms may be separated or resolved one from the other by conventional methods (for example chiral HPLC), or any given isomer may be obtained by conventional synthetic methods or by stereospecific or asymmetric syntheses.

Compounds of the invention may be prepared in a variety of ways, for example as described below. It will be appreciated by those skilled in the art that it may be necessary to protect certain reactive substituents during some of the procedures below. Standard protection and deprotection techniques, such as those described in Greene T.W. Protective groups in organic synthesis, New York, Wiley (1981), can be used. For example, primary amines can be protected as phthalimide, benzyl, t-butyloxycarbonyl, benzyloxycarbonyl or trityl derivatives. Carboxylic acid groups can be protected as esters. Aldehyde or ketone groups can be protected as acetals, ketals, thioacetals or thioketals. Deprotection of such groups is achieved using conventional procedures well known in the art. For example, protecting groups such as t-butyloxycarbonyl may be removed using an acid such as hydrochloric or trifluroroacetic acid in a suitable solvent such as dichloromethane, diethylether, isopropanol or mixtures thereof.

A compound of formula (I) may be prepared by coupling a compound of formula (II) where L is a leaving group such as a halogen (for example bromine or iodine) with a heterocyclic derivative of formula (III) according to reaction scheme 1. As would be realised by a skilled person, when A or B in formula (I) is N-tBOC, the BOC group may dissociate during this reaction to leave NH. Alternatively the BOC group may be removed at a later stage using standard conditions (e.g. trifluoroacetic acid (TFA) in dichloromethane). Typical coupling conditions comprise heating a compound of formula (II), a compound of formula (III), a base (such as potassium carbonate or cesium carbonate), copper (I) iodide or copper (I) oxide with N,N-dimethylglycine in dimethylsulfoxide at 180-190˚C in a microwave reactor or with conventional heating

at 130°C. In one embodiment, L is bromine or iodine. This route is also suitable for preparation of compounds wherein X-Z is COOH.

Scheme 1

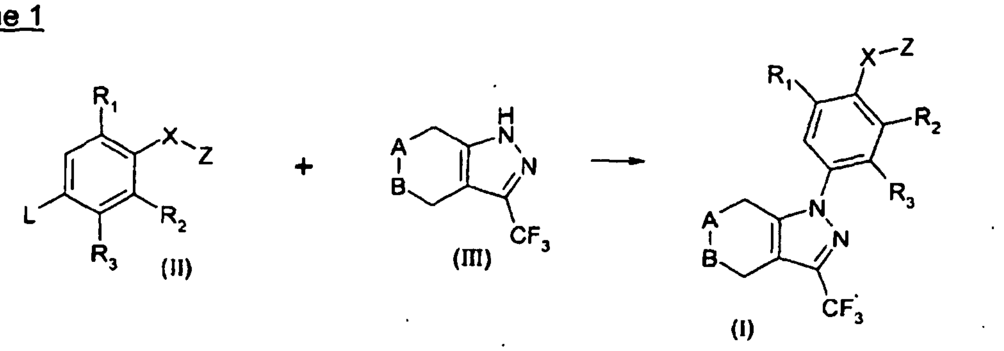

(II) (III) (I)

Thus, the present invention also provides a process for the manufacture of a compound of formula (I) or a salt or solvate thereof, the process comprising reacting a compound of formula (II):

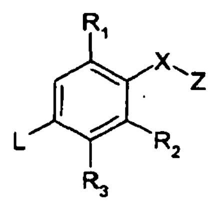

(II)

wherein L is a leaving group, and $R_1$, $R_2$, $R_3$, X and Z are as defined for formula (I), with a compound of formula (III):

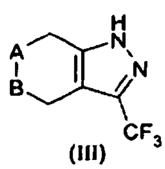

(III)

wherein A and B are as defined for formula (I), and thereafter optionally:

(i) converting a compound of formula (I) to another compound of formula (I) and/or
(ii) forming a salt or solvate thereof.

A compound of formula (V) [that is a compound of formula (I) in which X is carbonyl and Z is $NR_5R_6$ as defined for formula (I)] may be prepared by coupling a compound of formula (IV) with an amine according to reaction scheme 2. Typical coupling conditions comprise treatment of a compound of formula (IV) with 1,1'-carbonyldiimidazole in dichloromethane followed by the treatment with the amine ($HNR_5R_6$). Compounds of formula (IV) can be prepared in a manner similar to that described for compounds of formula (I) in scheme 1.

Scheme 2

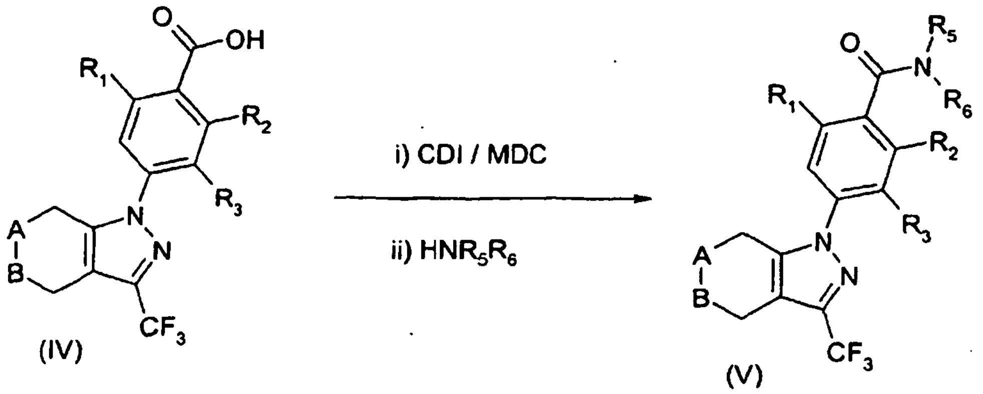


(IV)

(V)

A compound of formula (V) [that is a compound of formula (I) in which X is carbonyl and Z is $NR_5R_6$ as defined for formula (I)] may be prepared by coupling a compound of formula (IV) with an amine according to reaction scheme 3. Typical coupling conditions comprise treatment of a compound of formula (IV) with the amine hydrochloride salt ($HNR_5R_6$.HCl) in dimethylformamide in the presence of a base (such as diisopropylethylamine) followed by the treatment with HATU (2-(1H-7-Azabenzotriazol-1-yl)-1,1,3,3-tetramethyl uronium hexafluorophosphate methanaminium) . Compounds of formula (IV) can be prepared in a manner similar to that described for compounds of formula (I) in scheme 1.

Scheme 3

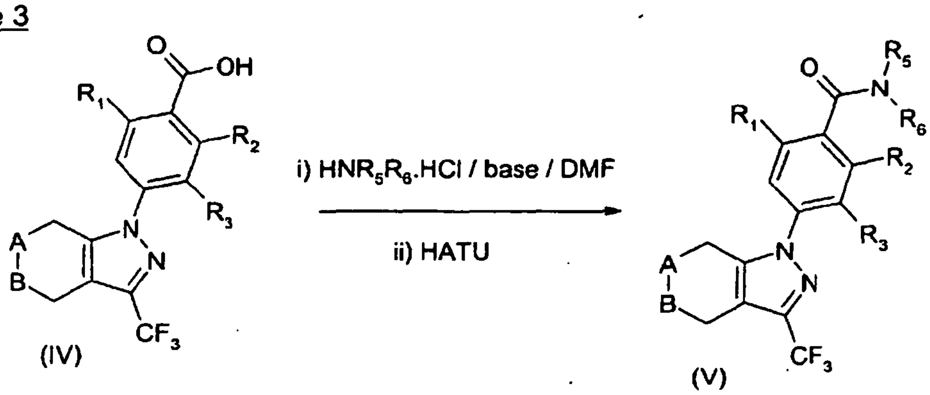


(IV)

(V)

A compound of formula (VII) [that is a compound of formula (I) where A is $NR_4$ and $R_4$ is $C_{1-2}$alkanoyl] may be prepared by an acylation reaction on a compound of formula (VI) according to reaction scheme 4. Typical reaction conditions comprise treatment of a compound of formula (VI) in dichloromethane with $R_4$-OH followed with microwave heating, or treatment of a compound of formula (VI) in dichloromethane with $R_4$-Cl in the presence of base (triethylamine or diisopropylethylamine). In one embodiment, $R_4$ is $C_{1-2}$alkanoyl. Compounds of formula (VI) can be prepared in a manner similar to that described for compounds of formula (I) in scheme 1. Scheme 4 also applies *mutatis mutandis* to the preparation of compounds of formula (I) wherein B is $NR_4$.

Scheme 4

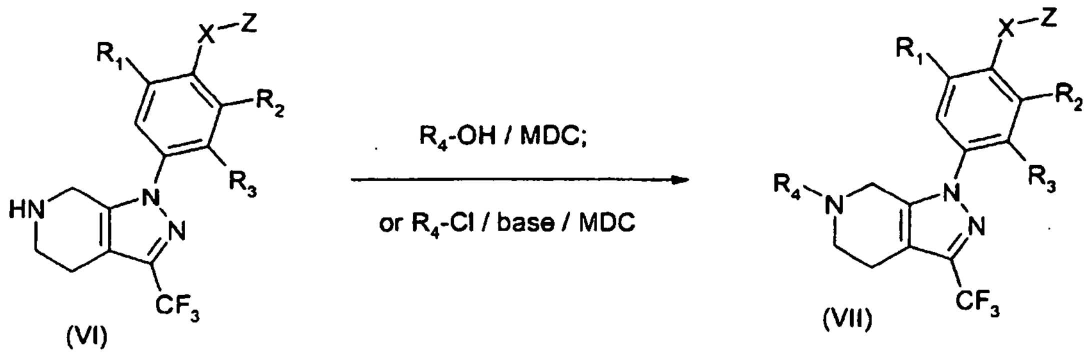


(VI) (VII)

An intermediate compound of formula (IX) [that is a compound of formula (II) in which X is carbonyl and Z is $NR_5R_6$ as defined for formula (I)] can be prepared by coupling a compound of formula (VIII) with a secondary amine according to reaction scheme 5. Typical coupling conditions comprise treatment of a compound of formula (VIII) with 1,1'-carbonyldiimidazole (CDI) in dichloromethane, followed by addition of the secondary amine ($HNR_5R_6$) (followed by the addition of a base such as triethylamine should the amine be available as a salt) after a 30 minute stirring period at ambient temperature. Compounds of formula (VIII) are commercially available.

<u>Scheme 5</u>

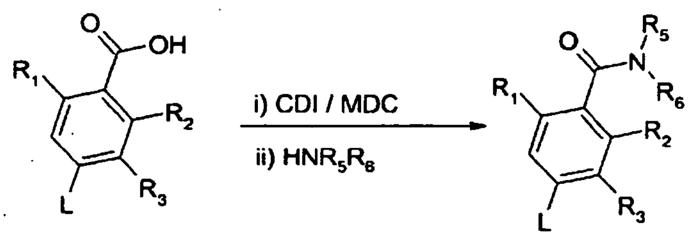


(VIII) (IX)

An intermediate compound of formula (XI) [that is a compound of formula (II) in which X is $CH_2$ and Z is $NR_5R_6$ as defined for formula (I)] can be prepared by alkylation of a secondary amide of formula ($HNR_5R_6$) with an alkylhalide compound of formula (X) according to reaction scheme 6. Typical alkylation conditions comprise treatment of a secondary amide ($HNR_5R_6$) with a suitable base such as sodium hydride (available as a 60% suspension in mineral oil) in dimethylformamide, followed by the addition of the alkylating agent (X). In one embodiment, $R_5$ and $R_6$ form a pyrrolidine ring substituted by an oxo group. Compounds of formula (X) are commercially available, or can be prepared as described below in schemes 11 and 15.

<u>Scheme 6</u>

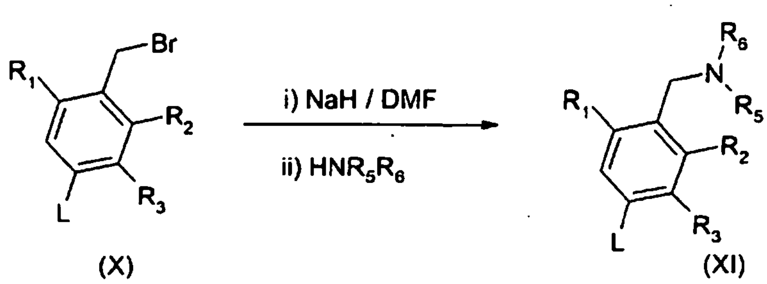


(X) (XI)

An intermediate compound of formula (XIII) can be prepared from a compound of formula (XII) by enolate addition to ethyl trifluoroacetate according to reaction scheme 7. Typical conditions comprise treatment of a compound of formula (XII) with a strong base (such as lithium diisopropylamide (LDA) or lithium hydride) in tetrahydrofuran or hexane followed by the addition of ethyl trifluoroacetate. Compounds of formula (XII) are commercially available.

Scheme 7

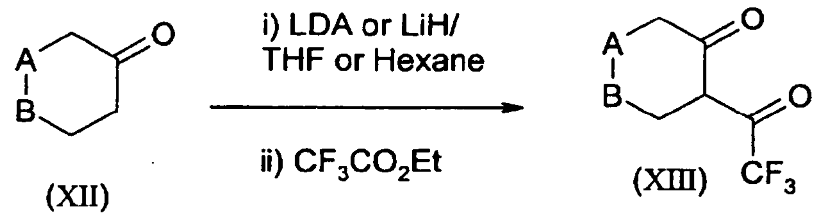


(XII) (XIII)

An intermediate compound of formula (III) can be prepared by condensation of a compound of formula (XIII) with hydrazine hydrate according to reaction scheme 8. Typical conditions comprise treatment of a compound of formula (XIII) in ethanol with hydrazine hydrate and heating (with or without acid catalysis). Compounds of formula (XIII) can be prepared in a similar manner as described in scheme 7.

Scheme 8

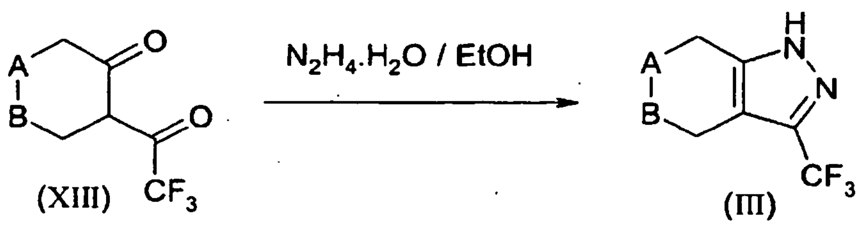


(XIII) (III)

An intermediate compound of formula (XV) can be prepared by the reduction of a compound of formula (XIV) using lithium aluminium hydride according to reaction scheme 9 (a skilled person would realise that the N-tBoc group may instead be at the position "B" in formula (I), to prepare compounds for formula (I) wherein B is $NR_4$). Typical reduction conditions comprise the addition of lithium aluminium hydride to a cooled solution of a compound of formula (XIV) in tetrahydrofuran (THF) followed by heating. Compounds of formula (XIV) can be prepared in a manner similar to that described for compounds of formula (XIII) in scheme 8.

Scheme 9

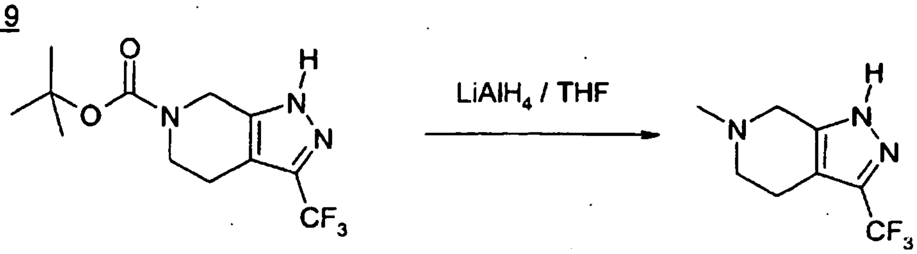


(XIV) (XV)

An intermediate compound of formula (XVII) can be prepared from a compound of formula (XVI) [prepared according to Liebigs Annalen Der Chemie 1984, 11, 1759-1882;] by enolate addition to ethyl trifluoroacetate followed by condensation with hydrazine hydrate according to reaction scheme 10. Typical conditions comprise treatment of a compound of formula (XVI) with a strong base (such as methyllithium) in tetrahydrofuran followed by the addition of ethyl trifluoroacetate at -78 degC. The isolated product is dissolved in ethanol and treated with hydrazine hydrate and heated at reflux for 6 hours.

Scheme 10

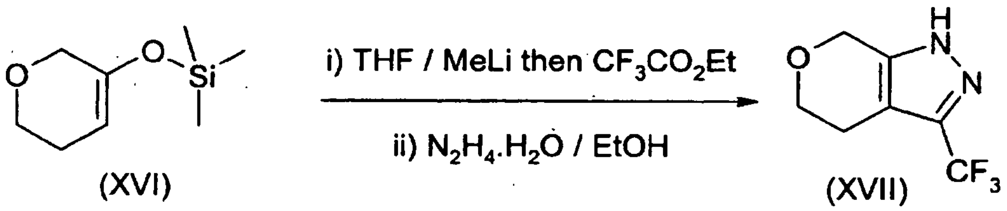


(XVI) (XVII)

An intermediate compound of formula (X) may be prepared by bromination of a compound of formula (XVIII) according to reaction scheme 11. Typical reaction conditions comprise addition of N-bromosuccinimide to a mixture of a compound of formula (XVIII) and triphenylphosphine in dichloromethane with cooling, followed with room temperature stirring. Compounds of formula (XVIII) are commercially available.

Scheme 11

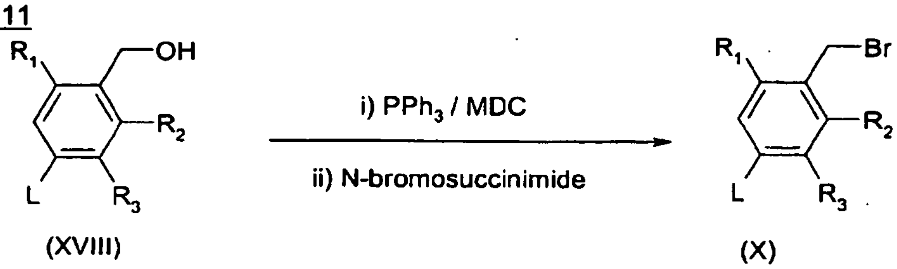


(XVIII) (X)

An intermediate compound of formula (IX) [that is a compound of formula (II) in which X is $CH_2$ and Z is $NR_5R_6$ as defined for formula (I)] may be prepared by coupling a compound of formula (VIII) with an amine according to reaction scheme 12. Typical coupling conditions comprise treatment of a compound of formula (VIII) with the amine ($HNR_5R_6$) in dimethylformamide (with or without a base such as diisopropylethylamine) followed by the treatment with HATU (2-(1H-7-Azabenzotriazol-1-yl)-1,1,3,3-tetramethyl uronium hexafluorophosphate methanaminium) . Compounds of formula (VIII) are commercially available.

Scheme 12

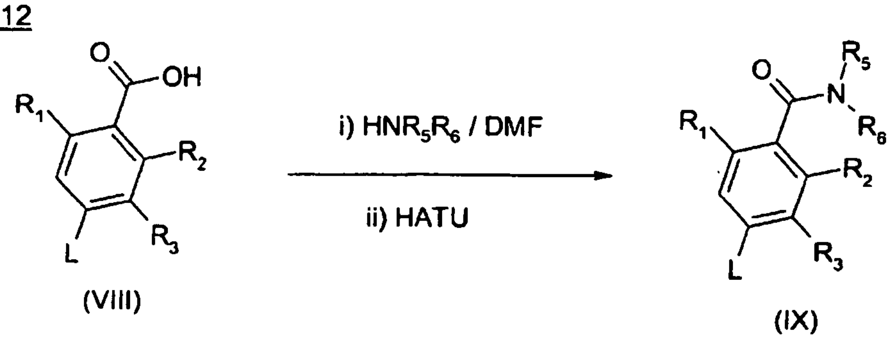


(VIII) (IX)

An intermediate compound of formula (XXIII) can be prepared by acylation then alkylation of a compound of formula (XXII) using a compound of formula (XXIV) according to reaction scheme 13. Typical conditions comprise treating a solution of a compound of formula (XXII) and a suitable base such as triethylamine in dimethylformamide with a compound of formula (XXIV), followed by the addition of excess sodium hydride (60% suspension in mineral oil) and stirring at room temperature until complete cyclisation. In one embodiment, W is a carbonyl or sulfonyl. Compounds of formula (XXII) are commercially available. Compounds of formula (XXIV) are commercially available.

Scheme 13

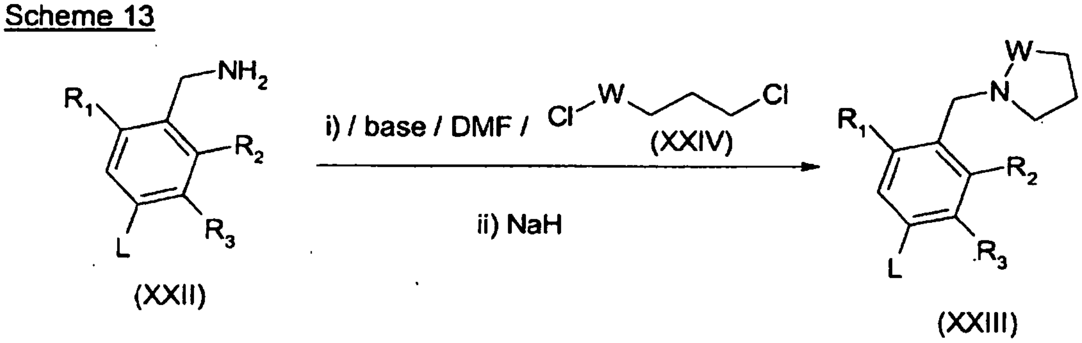


(XXII) (XXIII)

An intermediate compound of formula (XXVI) can be prepared by the acylation of a compound of formula (XXV) according to reaction scheme 14. Typical reaction conditions comprise treatment of a compound of formula (XXV) in dichloromethane with Y-Cl in the presence of base (triethylamine or diisopropylethylamine). In one embodiment, Y is $C_{1-2}$alkanoyl. Compounds of formula (XXV) wherein $R_1$, $R_2$ and $R_3$ are hydrogen are commercially available.

Scheme 14

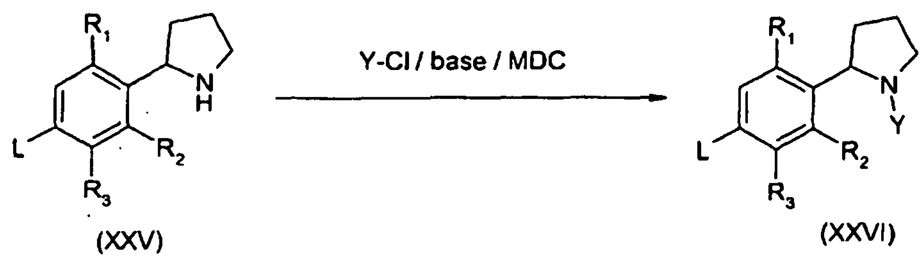


(XXV) (XXVI)

An intermediate compound of formula (X) may be prepared by bromination of a compound of formula (XXVII) according to reaction scheme 15. Typical reaction conditions comprise heating a mixture of a compound of formula (XXVII) with N-bromosuccinimide and benzoyl peroxide in carbon tetrachloride at reflux for approximately 20 hours. Compounds of formula (XXVII) are commercially available.

Scheme 15

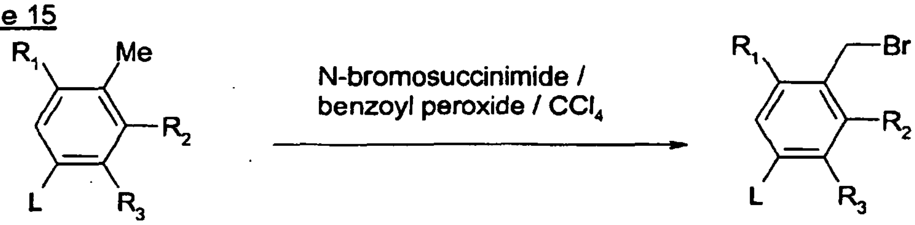


(XXVII) (X)

An intermediate compound of formula (XXIX) can be prepared from a compound of formula (X) by treatment with sodium cyanide according to reaction scheme 16. Typical reaction conditions comprise heating a mixture of a compound of formula (X) and sodium cyanide in ethanol and water at reflux for 3 hours. Compounds of formula (X) are commercially available or can be prepared in a similar manner as described in schemes 11 and 15.

Scheme 16

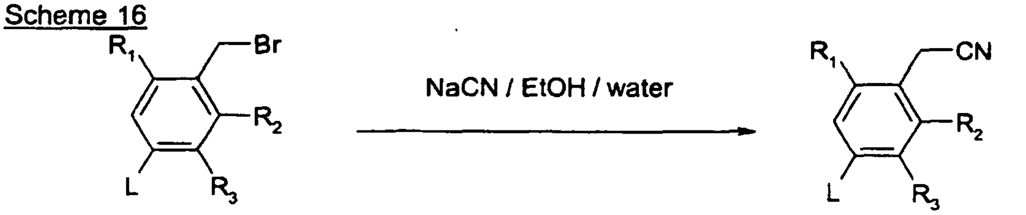


(X) (XXIX)

An Intermediate compound of formula (XXX) can be prepared from a compound of formula (XXIX) by base mediated hydrolysis according to reaction scheme 16. Typical reaction conditions comprise heating a mixture of a compound of formula (XXIX) and potassium hydroxide in ethanol and water at reflux for 5 hours. Compounds of formula (XXIX) are commercially available or can be prepared in a similar manner as described in scheme 16.

Scheme 17

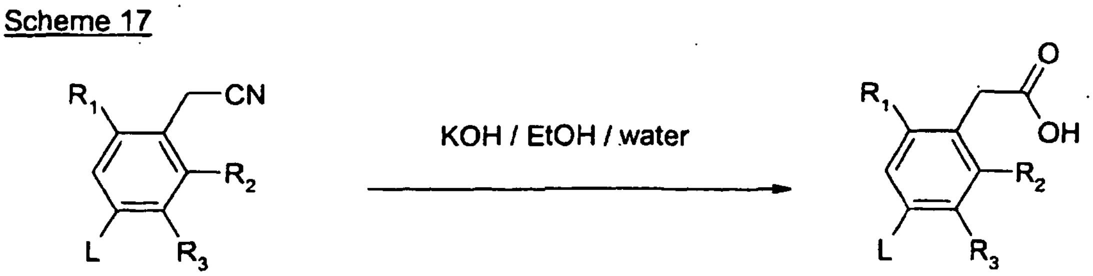


(XXIX) (XXX)

An intermediate compound of formula (XXXIII) can be prepared from a compound of formula (XXXI) by sulphonylation using a compound of formula (XXXII) according to reaction scheme 18. Typical reaction conditions comprise treatment of a compound of formula (XXXI) (or salt thereof) in dichloromethane and a base (such as triethylamine) with a sulphonyl chloride of formula (XXXII) with cooling, followed by room temperature stirring. Compounds of formula (XXXI) and (XXXII) are commercially available.

Scheme 18

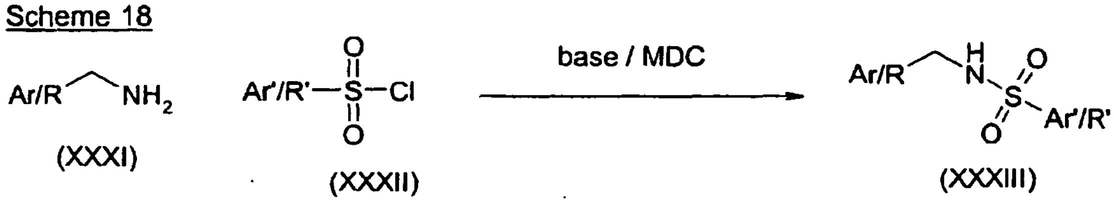


(XXXI) (XXXII) (XXXIII)

The compounds of the invention may be prepared singly or as compound libraries comprising at least 2, for example 5 to 1,400 compounds, for example 10 to 100 compounds. Libraries of compounds of the invention may be prepared by a combinatorial 'split and mix' approach or by multiple parallel synthesis using either solution phase or solid phase chemistry, by procedures known to those skilled in the art. Thus according to a further aspect there is provided a compound library comprising at least 2 compounds of the invention.

Compounds of the invention have been found to potentiate the AMPA receptor, and are thus expected to be useful in the treatment of disease states which require potentiation of such receptors, such as in the enhancement of cognition, for example in the treatment of cognition impairment brought about by various diseases. The compounds are also expected to be useful in the treatment of psychotic conditions, including schizophrenia. The compounds may be useful for these purposes on their own, or as part of a supplementary or adjunctive therapy. It will be appreciated that the invention includes the following further aspects:

i) a compound of formula (I) or a salt or solvate thereof for use as a medicament;
ii) the use of a compound of formula (I) or a salt or solvate thereof in the manufacture of a medicament for treating or preventing a disease or condition caused by a reduction or imbalance in glutamate receptor function in a mammal;
iii) a compound of formula (I) or a salt or solvate thereof for use in treating or preventing a disease or condition caused by a reduction or imbalance in glutamate receptor function in a mammal; and
iv) a pharmaceutical composition comprising a compound of formula (I) or a salt or solvate thereof and at least one pharmaceutically acceptable carrier or diluents.

Furthermore, the invention also provides a combination product of a compound of formula (I) with an antipsychotic. In addition, the invention provides:

i) a pharmaceutical composition comprising such a combination product and at least one pharmaceutically acceptable carrier or diluent;
ii) the use of such a combination in the manufacture of a medicament for treating or preventing a disease or condition caused by a reduction or imbalance in glutamate receptor function in a mammal;
iii) such a combination product for use in treating or preventing a disease or condition caused by a reduction or imbalance in glutamate receptor function in a mammal;
iv) such a combination product for use as a medicament.

The embodiments described in respect of the first aspect apply equally to each of these further aspects.

In one embodiment, the mammal to be treated is a human. It is to be understood that "treatment" as used herein includes prophylaxis as well as alleviation of established symptoms.

In the case of aspects ii), iii) and v), relevant diseases or conditions are: psychosis and psychotic disorders (including schizophrenia, schizo-affective disorder, schizophreniform diseases, brief reactive psychosis, child onset schizophrenia, "schizophrenia-spectrum" disorders such as schizoid or schizotypal personality disorders, acute psychosis, alcohol psychosis, drug-induced psychosis, autism, delerium, mania (including acute mania), manic depressive psychosis, hallucination, endogenous psychosis, organic psychosyndrome, paranoid and delusional disorders, puerperal psychosis, and psychosis associated with neurodegenerative diseases such as Alzheimer's disease); cognitive impairment (e.g. the treatment of impairment of cognitive functions including attention, orientation, memory (i.e. memory disorders, amnesia, amnesic disorders and age-associated memory impairment) and language function, and including cognitive impairment as a result of stroke, Alzheimer's disease, Aids-related dementia or other dementia states, as well as other acute or sub-acute conditions that may cause cognitive decline such as delirium or depression (pseudodementia states) trauma, aging, stroke, neurodegeneration, drug-induced states, neurotoxic agents), mild cognitive impairment, age related cognitive impairment, autism related cognitive impairment, Down's syndrome, cognitive deficit related to psychosis, post-electroconvulsive treatment related cognitive disorders; anxiety disorders (including generalised anxiety disorder, social anxiety disorder, agitation, tension, social or emotional withdrawal in psychotic patients, panic disorder, and obsessive compulsive disorder); neurodegenerative diseases (such as Alzheimer's disease, amyotrophic lateral sclerosis, motor neurone disease and other motor disorders such as Parkinson's disease (including relief from locomotor deficits and/or motor disability, including slowly increasing disability in purposeful movement, tremors, bradykinesia, hyperkinesia (moderate and severe), akinesia, rigidity, disturbance of balance and co-ordination, and a disturbance of posture), dementia in Parkinson's disease, dementia in Huntington's disease, neuroleptic-induced Parkinsonism and tardive dyskinesias, neurodegeneration following stroke, cardiac arrest, pulmonary bypass, traumatic brain injury, spinal cord injury or the like, and demyelinating diseases such as multiple sclerosis and amyotrophic lateral sclerosis); depression (which term includes bipolar (manic) depression (including type I and type II), unipolar depression, single or recurrent major depressive episodes with or without psychotic features, catatonic features, melancholic features, atypical features (e.g. lethargy, overeating/obesity, hypersomnia) or postpartum onset, seasonal affective disorder and dysthymia, depression-related anxiety, psychotic depression, and depressive disorders resulting from a general medical condition including, but not limited to, myocardial infarction, diabetes, miscarriage or abortion); post-traumatic stress syndrome; attention deficit disorder; attention deficit hyperactivity disorder, drug-induced (phencyclidine, ketamine and other dissociative anaesthetics, amphetamine and other psychostimulants and cocaine) disorders; Huntingdon's chorea; tardive dyskinesia; dystonia; myoclonus; spasticity; obesity; stroke; sexual dysfunction; sleep disorders and some forms of epilepsy.

Within the context of the present invention, the terms describing the indications used herein are classified in the Diagnostic and Statistical Manual of Mental Disorders, 4th Edition, published by the American Psychiatric Association (DSM-IV) and/or the International Classification of Diseases, 10th Edition (ICD-10). The various subtypes of the disorders mentioned herein are contemplated as part of the present invention. Numbers in brackets after the listed diseases below refer to the classification code in DSM-IV.

Within the context of the present invention, the term "psychotic disorder" includes :-Schizophrenia including

the subtypes Paranoid Type (295.30), Disorganised Type (295.10), Catatonic Type (295.20), Undifferentiated Type (295.90) and Residual Type (295.60); Schizophreniform Disorder (295.40); Schizoaffective Disorder (295.70) including the subtypes Bipolar Type and Depressive Type; Delusional Disorder (297.1) including the subtypes Erotomanic Type, Grandiose Type, Jealous Type, Persecutory Type, Somatic Type, Mixed Type and Unspecified Type; Brief Psychotic Disorder (298.8); Shared Psychotic Disorder (297.3); Psychotic Disorder Due to a General Medical Condition including the subtypes With Delusions and With Hallucinations; Substance-Induced Psychotic Disorder including the subtypes With Delusions (293.81) and With Hallucinations (293.82); and Psychotic Disorder Not Otherwise Specified (298.9).

Compounds of the invention may also be of use in the treatment of the following disorders:-Depression and mood disorders including Major Depressive Episode, Manic Episode, Mixed Episode and Hypomanic Episode; Depressive Disorders including Major Depressive Disorder, Dysthymic Disorder (300.4), Depressive Disorder Not Otherwise Specified (311); Bipolar Disorders including Bipolar I Disorder, Bipolar II Disorder (Recurrent Major Depressive Episodes with Hypomanic Episodes) (296.89), Cyclothymic Disorder (301.13) and Bipolar Disorder Not Otherwise Specified (296.80); Other Mood Disorders including Mood Disorder Due to a General Medical Condition (293.83) which includes the subtypes With Depressive Features, With Major Depressive-like Episode, With Manic Features and With Mixed Features), Substance-Induced Mood Disorder (including the subtypes With Depressive Features, With Manic Features and With Mixed Features) and Mood Disorder Not Otherwise Specified (296.90):

Anxiety disorders including Panic Attack; Panic Disorder including Panic Disorder without Agoraphobia (300.01) and Panic Disorder with Agoraphobia (300.21); Agoraphobia; Agoraphobia Without History of Panic Disorder (300.22). Specific Phobia (300.29, formerly Simple Phobia) including the subtypes Animal Type, Natural Environment Type, Blood-Injection-Injury Type, Situational Type and Other Type), Social Phobia (Social Anxiety Disorder, 300.23), Obsessive-Compulsive Disorder (300.3), Posttraumatic Stress Disorder (309.81), Acute Stress Disorder (308.3), Generalized Anxiety Disorder (300.02), Anxiety Disorder Due to a General Medical Condition (293.84), Substance-Induced Anxiety Disorder, Separation Anxiety Disorder (309.21). Adjustment Disorders with Anxiety (309.24) and Anxiety Disorder Not Otherwise Specified (300.00):

Substance-related disorders including Substance Use Disorders such as Substance Dependence, Substance Craving and Substance Abuse; Substance-Induced Disorders such as Substance Intoxication, Substance Withdrawal, Substance-Induced Delirium, Substance-Induced Persisting Dementia, Substance-Induced Persisting Amnestic Disorder, Substance-Induced Psychotic Disorder, Substance-Induced Mood Disorder, Substance-Induced Anxiety Disorder, Substance-Induced Sexual Dysfunction, Substance-Induced Sleep Disorder and Hallucinogen Persisting Perception Disorder (Flashbacks); Alcohol-Related Disorders such as Alcohol Dependence (303.90), Alcohol Abuse (305.00), Alcohol Intoxication (303.00), Alcohol Withdrawal (291.81), Alcohol Intoxication Delirium, Alcohol Withdrawal Delirium, Alcohol-Induced Persisting Dementia, Alcohol-Induced Persisting Amnestic Disorder, Alcohol-Induced Psychotic Disorder, Alcohol-Induced Mood Disorder, Alcohol-Induced Anxiety Disorder, Alcohol-Induced Sexual Dysfunction, Alcohol-Induced Sleep Disorder and Alcohol-Related Disorder Not Otherwise Specified (291.9); Amphetamine (or Amphetamine-Like)-Related Disorders such as Amphetamine Dependence (304.40), Amphetamine Abuse (305.70), Amphetamine Intoxication (292.89). Amphetamine Withdrawal (292.0), Amphetamine Intoxication Delirium, Amphetamine Induced Psychotic Disorder, Amphetamine-induced Mood Disorder, Amphetamine-Induced Anxiety Disorder, Amphetamine-Induced Sexual Dysfunction, Amphetamine-Induced Sleep Disorder and Amphetamine-Related Disorder Not Otherwise Specified (292.9); Caffeine Related Disorders such as Caffeine Intoxication (305.90), Caffeine-Induced Anxiety Disorder, Caffeine-Induced Sleep Disorder and Caffeine-Related Disorder Not Otherwise Specified (292.9); Cannabis-Related Disorders such as Cannabis Dependence (304.30), Cannabis Abuse (305.20), Cannabis Intoxication (292.89), Cannabis Intoxication Delirium, Cannabis-Induced Psychotic Disorder, Cannabis-Induced Anxiety Disorder and Cannabis-Related Disorder Not Otherwise Specified (292.9); Cocaine-Related Disorders such as Cocaine Dependence (304.20), Cocaine Abuse (305.60), Cocaine Intoxication (292.89), Cocaine Withdrawal (292.0), Cocaine Intoxication Delirium, Cocaine-Induced Psychotic Disorder, Cocaine-Induced Mood Disorder, Cocaine-Induced Anxiety Disorder, Cocaine-Induced Sexual Dysfunction, Cocaine-Induced Sleep Disorder and Cocaine-Related Disorder Not Otherwise Specified (292.9); Hallucinogen-Related Disorders such as Hallucinogen Dependence (304.50), Hallucinogen Abuse (305.30), Hallucinogen Intoxication (292.89), Hallucinogen Persisting Perception Disorder (Flashbacks) (292.89), Hallucinogen Intoxication Delirium, Hallucinogen-Induced Psychotic Disorder, Hallucinogen-Induced Mood Disorder, Hallucinogen-Induced Anxiety Disorder and Hallucinogen-Related Disorder Not Otherwise Specified (292.9); Inhalant-Related Disorders such as Inhalant Dependence (304.60), Inhalant Abuse (305.90), Inhalant Intoxication (292.89), Inhalant Intoxication Delirium, Inhalant-Induced Persisting Dementia, Inhalant-Induced Psychotic Disorder, Inhalant-Induced Mood Disorder, Inhalant-Induced Anxiety Disorder and Inhalant-Related Disorder Not Otherwise Specified (292.9); Nicotine-Related Disorders such as Nicotine Dependence (305.1), Nicotine Withdrawal (292.0) and Nicotine-Related Disorder Not Otherwise Specified (292.9); Opioid-Related Disorders such as Opioid Dependence (304.00), Opioid Abuse

(305.50), Opioid Intoxication (292.89), Opioid Withdrawal (292.0), Opioid Intoxication Delirium, Opioid-Induced Psychotic Disorder, Opioid-Induced Mood Disorder, Opioid-Induced Sexual Dysfunction, Opioid-Induced Sleep Disorder and Opioid-Related Disorder Not Otherwise Specified (292.9); Phencyclidine (or Phencyclidine-Like)-Related Disorders such as Phencyclidine Dependence (304.60), Phencyclidine Abuse (305.90), Phencyclidine Intoxication (292.89), Phencyclidine Intoxication Delirium, Phencyclidine-Induced Psychotic Disorder, Phencyclidine-Induced Mood Disorder, Phencyclidine-Induced Anxiety Disorder and Phencyclidine-Related Disorder Not Otherwise Specified (292.9); Sedative-, Hypnotic-, or Anxiolytic-Related Disorders such as Sedative, Hypnotic, or Anxiolytic Dependence (304.10), Sedative, Hypnotic, or Anxiolytic Abuse (305.40), Sedative, Hypnotic, or Anxiolytic Intoxication (292.89), Sedative, Hypnotic, or Anxiolytic Withdrawal (292.0), Sedative, Hypnotic, or Anxiolytic Intoxication Delirium, Sedative, Hypnotic, or Anxiolytic Withdrawal Delirium, Sedative-, Hypnotic-, or Anxiolytic-Persisting Dementia, Sedative-, Hypnotic-, or Anxiolytic- Persisting Amnestic Disorder, Sedative-, Hypnotic-, or Anxiolytic-Induced Psychotic Disorder, Sedative-, Hypnotic-, or Anxiolytic-Induced Mood Disorder, Sedative-, Hypnotic-, or Anxiolytic-Induced Anxiety Disorder Sedative-, Hypnotic-, or Anxiolytic-Induced Sexual Dysfunction, Sedative-, Hypnotic-, or Anxiolytic-Induced Sleep Disorder and Sedative-, Hypnotic-, or Anxiolytic-Related Disorder Not Otherwise Specified (292.9); Polysubstance-Related Disorder such as Polysubstance Dependence (304.80); and Other (or Unknown) Substance-Related Disorders such as Anabolic Steroids, Nitrate Inhalants and Nitrous Oxide:

Sleep disorders including primary sleep disorders such as Dyssomnias such as Primary Insomnia (307.42), Primary Hypersomnia (307.44), Narcolepsy (347), Breathing-Related Sleep Disorders (780.59), Circadian Rhythm Sleep Disorder (307.45) and Dyssomnia Not Otherwise Specified (307.47); primary sleep disorders such as Parasomnias such as Nightmare Disorder (307.47), Sleep Terror Disorder (307.46), Sleepwalking Disorder (307.46) and Parasomnia Not Otherwise Specified (307.47); Sleep Disorders Related to Another Mental Disorder such as Insomnia Related to Another Mental Disorder (307.42) and Hypersomnia Related to Another Mental Disorder (307.44); Sleep Disorder Due to a General Medical Condition, in particular sleep disturbances associated with such diseases as neurological disorders, neuropathic pain, restless leg syndrome, heart and lung diseases; and Substance-Induced Sleep Disorder including the subtypes Insomnia Type, Hypersomnia Type, Parasomnia Type and Mixed Type; sleep apnea and jet-lag syndrome:

Autism Spectrum Disorders including Autistic Disorder (299.00), Asperger's Disorder (299.80), Rett's Disorder (299.80), Childhood Disintegrative Disorder (299.10) and Pervasive Disorder Not Otherwise Specified (299.80, including Atypical Autism).

Attention-Deficit/Hyperactivity Disorder including the subtypes Attention-Deficit /Hyperactivity Disorder Combined Type (314.01), Attention-Deficit /Hyperactivity Disorder Predominantly Inattentive Type (314.00), Attention-Deficit /Hyperactivity Disorder Hyperactive-Impulse Type (314.01) and Attention-Deficit /Hyperactivity Disorder Not Otherwise Specified (314.9); Hyperkinetic Disorder; Disruptive Behaviour Disorders such as Conduct Disorder including the subtypes childhood-onset type (321.81), Adolescent-Onset Type (312.82) and Unspecified Onset (312.89), Oppositional Defiant Disorder (313.81) and Disruptive Behaviour Disorder Not Otherwise Specified; and Tic Disorders such as Tourette's Disorder (307.23):

Personality Disorders including the subtypes Paranoid Personality Disorder (301.0), Schizoid Personality Disorder (301.20), Schizotypal Personality Disorder (301,22), Antisocial Personality Disorder (301.7), Borderline Personality Disorder (301,83), Histrionic Personality Disorder (301.50), Narcissistic Personality Disorder (301,81), Avoidant Personality Disorder (301.82), Dependent Personality Disorder (301.6), Obsessive-Compulsive Personality Disorder (301.4) and Personality Disorder Not Otherwise Specified (301.9):

Enhancement of cognition including the treatment of cognition impairment in other diseases such as schizophrenia, bipolar disorder, depression, other psychiatric disorders and psychotic conditions associated with cognitive impairment, e.g. Alzheimer's disease: and

Sexual dysfunctions including Sexual Desire Disorders such as Hypoactive Sexual Desire Disorder (302.71), and Sexual Aversion Disorder (302.79); sexual arousal disorders such as Female Sexual Arousal Disorder (302.72) and Male Erectile Disorder (302.72); orgasmic disorders such as Female Orgasmic Disorder (302.73), Male Orgasmic Disorder (302.74) and Premature Ejaculation (302.75); sexual pain disorder such as Dyspareunia (302.76) and Vaginismus (306.51); Sexual Dysfunction Not Otherwise Specified (302.70); paraphilias such as Exhibitionism (302.4), Fetishism (302.81), Frotteurism (302.89), Pedophilia (302.2), Sexual Masochism (302.83), Sexual Sadism (302.84), Transvestic Fetishism (302.3), Voyeurism (302.82) and Paraphilia Not Otherwise Specified (302.9); gender identity disorders such as Gender Identity Disorder in Children (302.6) and Gender Identity Disorder in Adolescents

or Adults (302.85); and Sexual Disorder Not Otherwise Specified (302.9).

All of the various forms and sub-forms of the disorders mentioned herein are contemplated as part of the present invention.

Within the context of the present invention, the term "cognitive impairment" includes for example the treatment of impairment of cognitive functions including attention, orientation, learning disorders, memory (i.e. memory disorders, amnesia, amnesic disorders, transient global amnesia syndrome and age-associated memory impairment) and language function; cognitive impairment as a result of stroke, Alzheimer's disease, Huntington's disease, Pick disease, Aids-related dementia or other dementia states such as Multiinfarct dementia, alcoholic dementia, hypotiroidism-related dementia, and dementia associated to other degenerative disorders such as cerebellar atrophy and amyotropic lateral sclerosis; other acute or sub-acute conditions that may cause cognitive decline such as delirium or depression (pseudodementia states) trauma, head trauma, age related cognitive decline, stroke, neurodegeneration, drug-induced states, neurotoxic agents, mild cognitive impairment, age related cognitive impairment, autism related cognitive impairment, Down's syndrome, cognitive deficit related to psychosis, and post-electroconvulsive treatment related cognitive disorders; and dyskinetic disorders such as Parkinson's disease, neuroleptic-induced parkinsonism, and tardive dyskinesias.

The compounds of the invention may be used in combination with the following agents to treat or prevent psychotic disorders: i) antipsychotics (such as olanzapine, risperidone, clozapine, ziprazidone and talnetant); ii) drugs for extrapyramidal side effects, for example anticholinergics (such as benztropine, biperiden, procyclidine and trihexyphenidyl), antihistamines (such as diphenhydramine) and dopaminergics (such as amantadine); iii) antidepressants; iv) anxiolytics; and v) cognitive enhancers for example cholinesterase inhibitors (such as tacrine, donepezil, rivastigmine and galantamine).

The compounds of the invention may be used in combination with antidepressants to treat or prevent depression and mood disorders.

The compounds of the invention may be used in combination with the following agents to treat or prevent bipolar disease: i) mood stabilisers; ii) antipsychotics; and iii) antidepressants.

The compounds of the invention may be used in combination with the following agents to treat or prevent anxiety disorders: i) anxiolytics; and ii) antidepressants.

The compounds of the invention may be used in combination with the following agents to improve nicotine withdrawal and reduce nicotine craving: i) nicotine replacement therapy for example a sublingual formulation of nicotine beta-cyclodextrin and nicotine patches; and ii) bupropion.

The compounds of the invention may be used in combination with the following agents to improve alcohol withdrawal and reduce alcohol craving: i) NMDA receptor antagonists for example acamprosate; ii) GABA receptor agonists for example tetrabamate; and iii) Opioid receptor antagonists for example naltrexone.

The compounds of the invention may be used in combination with the following agents to improve opiate withdrawal and reduce opiate craving: i) opioid mu receptor agonist/opioid kappa receptor antagonist for example buprenorphine; ii) opioid receptor antagonists for example naltrexone; and iii) vasodilatory antihypertensives for example lofexidine.

The compounds of the invention may be used in combination with the following agents to treat or prevent sleeping disorders: i) benzodiazepines for example temazepam, lormetazepam, estazolam and triazolam; ii) non-benzodiazepine hypnotics for example zolpidem, zopiclone, zaleplon and indiplon; iii) barbiturates for example aprobarbital, butabarbital, pentobarbital, secobarbita and phenobarbital; iv) antidepressants; v) other sedative-hypnotics for example chloral hydrate and chlormethiazole.

The compounds of the invention may be used in combination with the following agents to treat anorexia: i) appetite stimulants for example cyproheptidine; ii) antidepressants; iii) antipsychotics; iv) zinc; and v) premenstral agents for example pyridoxine and progesterones.

The compounds of the invention may be used in combination with the following agents to treat or prevent bulimia: i) antidepressants; ii) opioid receptor antagonists; iii) antiemetics for example ondansetron; iv) testosterone receptor antagonists for example flutamide; v) mood stabilisers; vi) zinc; and vii) premenstral agents.

The compounds of the invention may be used in combination with the following agents to treat or prevent autism: i) antipsychotics; ii) antidepressants; iii) anxiolytics; and iv) stimulants for example methylphenidate, amphetamine formulations and pemoline.

The compounds of the invention may be used in combination with the following agents to treat or prevent Attention Deficit Hyperactivity Disorder: i) stimulants for example methylphenidate, amphetamine formulations and pemoline; and ii) non-stimulants for example norepinephrine reuptake inhibitors (such as atomoxetine), alpha 2 adrenoceptor agonists (such as clonidine), antidepressants, modafinil, and cholinesterase inhibitors (such as galantamine and donezepil).

The compounds of the invention may be used in combination with the following agents to treat personality disorders: i) antipsychotics; ii) antidepressants; iii) mood stabilisers; and iv) anxiolytics.

The compounds of the invention may be used in combination with the following agents to treat or prevent male sexual dysfunction: i) phosphodiesterase V inhibitors, for example vardenafil and sildenafil; ii) dopamine agonists/ dopamine transport inhibitors for example apomorphine and buproprion; iii) alpha adrenoceptor antagonists for example phentolamine; iv) prostaglandin agonists for example alprostadil; v) testosterone agonists such as testosterone; vi) serotonin transport inhibitors for example serotonin reuptake inhibitors; v) noradrenaline transport inhibitors for example reboxetine and vii) 5-HT1A agonists, for example flibanserine.

The compounds of the invention may be used in combination with the same agents specified for male sexual dysfunction to treat or prevent female sexual dysfunction, and in addition an estrogen agonist such as estradiol.

Antipsychotic drugs include Typical Antipsychotics (for example chlorpromazine, thioridazine, mesoridazine, fluphenazine, perphenazine, prochlorperazine, trifluoperazine, thiothixine, haloperidol, molindone and loxapine); and Atypical Antipsychotics (for example clozapine, olanzapine, risperidone, quetiapine, aripirazole, ziprasidone, amisulpride, ziprazidone and talnetant).

Antidepressant drugs include serotonin reuptake inhibitors (such as citalopram, escitalopram, fluoxetine, paroxetine and sertraline); dual serotonin/noradrenaline reuptake inhibitors (such as venlafaxine, duloxetine and milnacipran); Noradrenaline reuptake inhibitors (such as reboxetine); tricyclic antidepressants (such as amitriptyline, clomipramine, imipramine, maprotiline, nortriptyline and trimipramine); monoamine oxidase inhibitors (such as isocarboxazide, moclobemide, phenelzine and tranylcypromine); and others (such as bupropion, mianserin, mirtazapine, nefazodone and trazodone).

Mood stabiliser drugs include lithium, sodium valproate/valproic acid/divalproex, carbamazepine, lamotrigine, gabapentin, topiramate and tiagabine.

Anxiolytics include benzodiazepines such as alprazolam and lorazepam.

Since the compounds of the invention are intended for use in pharmaceutical compositions, it will readily be understood that they are each optionally provided in substantially pure form, for example at least 60% pure, for example at least 75% pure or at least 85%, or at least 98% pure (% are on a weight for weight basis). Impure preparations of the compounds may be used for preparing the more pure forms used in the pharmaceutical compositions; these less pure preparations of the compounds should contain at least 1%.,or at least 5% or from 10 to 59% of a compound of the invention.

The compounds of the invention may be administered in conventional dosage forms prepared by combining a compound of the invention with standard pharmaceutical carriers or diluents according to conventional procedures well known in the art. These procedures may involve mixing, granulating and compressing or dissolving the ingredients as appropriate to the desired preparation.

The pharmaceutical compositions of the invention may be formulated for administration by any route, and include those in a form adapted for oral, topical or parenteral administration to mammals including humans.

The compositions may be formulated for administration by any route. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilising the compound and a sterile vehicle, for example water. The compound, depending on the vehicle and concentration used, can be either suspended or dissolved in the vehicle. In preparing solutions the compound can be dissolved in water for injection and filter sterilised

before filling into a suitable vial or ampoule and sealing.

Advantageously, agents such as a local anaesthetic, preservative and buffering agents can be dissolved in the vehicle. To enhance the stability, the composition can be frozen after filling into the vial and the water removed under vacuum. The dry lyophilised powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilisation cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, for example from 10-60% by weight, of the active material, depending on the method of administration. Where the compositions comprise dosage units, each unit may, for example contain from 0.1 to 20 mg of the active ingredient. For example, such a unit may contain from 1 to 10 mg.

It will be recognised by one of skill in the art that the optimal quantity and spacing of individual dosages of a compound of the invention will be determined by the nature and extent of the condition being treated, the form, route and site of administration, and the particular mammal being treated, and that such optimums can be determined by conventional techniques. It will also be appreciated by one of skill in the art that the optimal course of treatment, i.e. the number of doses of a compound of the invention given per day for a defined number of days, can be ascertained by those skilled in the art using conventional course of treatment determination tests.

The invention is illustrated by the Examples described below.

**Abbreviations**

| | |
|---|---|
| TEA | Triethylamine |
| TMS-Cl | Trimethylsilyl chloride |
| DME | Dimethyl ether |
| ss | saturated solution |
| TFA | Trifluoroacetic acid |
| DAD | Diode Array Detector |
| CD | Circular dichroism |
| a/a% | percentage by area unde the curve |
| LC/MS | Liquid Chromatography / Mass Spectrometry |
| NMR | Nuclear Magnetic Resonance |
| SCX | Chromatography column supplied by Varian™ |
| THF | Tetrahydrofuran |
| DMSO | Dimethylsulfoxide |
| DMF | Dimethylformamide |
| DCM / MDC | Dichloromethane / Methylene dichloride |
| CDI | 1,1'-Carbonyldiimidazole |
| LDA | Lithium diisopropylamide |
| EDC | 1-ethyl-3-(dimethylaminopropyl)carbodiimide |
| MsCl | Methanesulfonyl chloride |
| AcOH | Acetic acid |
| HOAt | 1-hydroxy-7-azabenzotriazole |
| HOBt | 1-hydroxybenzotriazole |
| Pd on C | Palladium on Charcoal |
| MeCN | Acetonitrile |
| MDAP | Mass-directed auto-preparation |
| HATU . | 2-(1H-7-Azabenzotriazol-1-yl)-1,1,3,3-tetramethyl uronium hexafluorophosphate methanaminium |

Starting materials were obtained from commercial suppliers and used without further purification unless otherwise stated. Flash chromatography was carried out using pre-packed Isolute Flash™ or Biotage™ silica-gel columns as the stationary phase and analytical grade solvents as the eluent.

NMR spectra were obtained at 298K, at the frequency stated using either a Bruker™ DPX400 or an Oxford

Instruments™ 250 MHz machine and run as a dilute solution of $CDCl_3$ unless otherwise stated. All NMR spectra were reference to tetramethylsilane (TMS $\delta_H$ 0, $\delta_c$ 0). All coupling constants are reported in hertz (Hz), and multiplicities are labelled s (singlet), br s or bs, (broad singlet), d (doublet), t (triplet), q (quartet), dd (doublet of doublets), dt (doublet of triplets) and m (multiplet).

Total ion current traces were obtained for electrospray positive and negative ionisation (ES+ / ES-) and atmospheric pressure chemical positive and negative ionisation (AP+ / AP-).

**Analytical chromatographic conditions**

| | |
|---|---|
| Column: | Gemini C18, 50 x 4.6 mm, 5 □m |
| Mobile phase: | A: $NH_4HCO_3$ sol. 10 mM, (pH10; B: $CH_3CN$ |
| Gradient: | 35% (B) for 0.5 min, 35% (B) → 95% (B) in 4.5 min, 95% (B) for 1.5 min |
| Flow rate: | 2 ml/min |
| UV wavelength range: | 210-350 nm |
| Ionization: | ES+/ES- |
| Mass range: | 100-900 amu |

**Mass-directed preparative chromatography conditions**

Column

The columns used are Waters Atlantis, the dimensions of which are 19mm x 100mm with particle size is 5mm.

Solvents

A : Aqueous solvent = Water + 0.1% Trifluoroacetic Acid
B : Organic solvent = Acetonitrile + 0.1% Trifluoroacetic Acid
Make up solvent = Methanol : Water 80:20 + 0.1 % Formic Acid
Needle rinse solvent = Methanol

Methods

There are five methods used depending on the analytical retention time of the compound of interest. They have a 20 minute runtime, which comprises of a 15.5 minute gradient followed by a 3.5 minute column flush and re-equilibration step.

**Method 1.8-2.1 = 0-30% B**

**Method 2.1-2.6 = 10-45% B**

**Method 2.6-3.1 = 15-65% B**

**Method 3.1-4.1 = 30-75% B**

Method > 4.1 = 50-100% B (in 14 minutes followed by 5 minutes flush and re-equilibration)

The above methods have a flow rate of 20ml/mins. The injection volume for the method is a 500ul partial loop injection. The column temperature is ambient

**High pH mass-directed preparative chromatography conditions**

**Column**

The columns used are Waters XBridge, the dimensions of which are 19mm x 100mm (small scale) and 30mm x 100mm (large scale). The stationary phase particle size is 5m m.

**Solvents**

A : Aqueous solvent = 10mM Ammonium Carbonate adjusted to pH 10 with ammonia soln
B : Organic solvent = Acetonitrile
Make up solvent = Methanol : Water 80:20
Needle rinse solvent = Methanol

**Methods**

There are five methods used depending on the analytical retention time of the compound of interest. They have a 13.5-minute runtime, which comprises of a 10-minute gradient followed by a 3.5 minute column flush and re-equilibration step.

Large/Small Scale 1.0-1.5 = 5-30% B

Large/Small Scale 1.5-2.2 = 15-55% B

Large/Small Scale 2.2-2.9 = 30-85% B

Large/Small Scale 2.9-3.6 = 50-99% B

Large/Small Scale 3.6-5.0 = 80-99% B (in 6 minutes followed by 7.5 minutes flush and re-equilibration)

**Flow rate**

All of the above methods have a flow rate of either 20mls/min (Small Scale) or 40mls/min (Large Scale).

In the procedures that follow, reference to an Intermediate or Example by number is typically provided. This is provided merely for assistance to the skilled chemist to identify the starting material used. The starting material may not necessarily have been prepared from the batch referred to. Unless otherwise stated, all compounds with chiral centre (s) are racemic.

**Description 1: 1-[(4-iodophenyl)methyl]-2-pyrrolidinone (D1)**

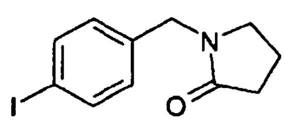

A solution of 2-pyrrolidinone (3.15g, 37.1mmol) in dimethylformamide (130ml) was cooled in an ice/methanol

bath with stirring under an atmosphere of argon. Then a solid suspension of sodium hydride (60% in mineral oil, 1.48g, 37.0mmol) was added portionwise over 10 minutes. The reaction mix was allowed to stir with cooling for 30 minutes, then 4-iodobenzyl bromide (10g, 33.7mmol) was added portionwise over 10 minutes. The whole mix was allowed to warm slowly up to room temperature then stirred for a further 3 hours. The reaction mixture was partitioned between dichloromethane (150ml) and water (100ml), the aqueous layer was washed a second time with dichloromethane (100ml), the combined organic layers were removed and washed with water (3 x 100ml) then brine (100ml). The organic layer was dried over sodium sulphate and evaporated under reduced pressure to give the title compound as a yellow solid (9.98g, 98%).
$^1$H-NMR (400MHz, $CDCl_3$) δ: 7.65 (2H, m), 7.00 (2H, m), 4.39 (2H, s), 3.25 (2H, m), 2.44 (2H, m), 2.00 (2H, m); LC/MS Retention time 2.57mins/(ES+) 302 (M+H, $C_{11}H_{12}INO$ requires 301).

**Description 2: 1-[(4-iodophenyl)carbonyl]pyrrolidine (D2)**

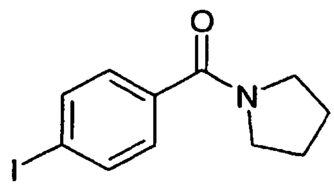

A suspension of 4-iodobenzoic acid (8.68g, 35mmol) in dichloromethane (120ml) was treated portionwise over 5 minutes with solid 1,1'-carbonyldiimidazole (5.67g, 35mmol) at room temperature with stirring under an atmosphere of argon. This mix was stirred at room temperature for a further 30 minutes. Pyrrolidine (2.49g, 35mmol) was then added to the reaction mixture slowly over 15 minutes. The whole mix was then stirred at room temperature under argon for 1 hour. The reaction mix was washed twice with saturated sodium bicarbonate solution (100ml each), the organic layer was dried over sodium sulphate and the solvent removed under reduced pressure to give the title compound as a brown solid (10.05g, 95%).
$^1$H-NMR (400MHz, $CDCl_3$) δ:7.75 (2H, m), 7.26 (2H, m), 3.63 (2H, t, J= 7Hz), 3.40 (2H, m, J=7Hz), 1.96 (2H, m), 1.88 (2H, m); LC/MS Retention time 2.51mins/(ES+) 302 (M+H, $C_{11}H_{12}INO$ requires 301).

**Description 3: 3-(trifluoroacetyl)tetrahydro-4*H*-pyran-4-one (D3)**

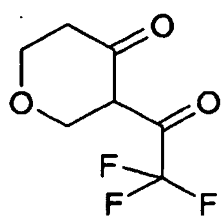

A solution of tetrahydro-4H-pyran-4-one (5.0g, 50mmol) in tetrahydrofuran (100ml) was cooled to -70˚C with stirring under argon. The solution was then treated with a 2M solution of lithium diisopropylamide in tetrahydrofuran (25ml) dropwise over 30 minutes. The mix was then stirred at -70˚C for 30 minutes, then treated dropwise with ethyl trifluoroacetate (7.1g, 5.9ml, 50mmol)with stirring under argon. The mix was then allowed to warm slowly up to 20˚C and stirred for 16 hours under argon. The reaction mix was then partitioned between ethyl acetate (50ml) and water (100ml). The organic layer was dried over sodium sulphate and the solvent removed by rotary evaporation to give the title compound as a foamy yellow solid (10.34g, quantitative).
$^1$H-NMR (400MHz, $MeOD_4$) δ: 4.50 (2H, m), 3.87 (2H, m), 3.22 (1H, m), 2.34 (2H, m); LC/MS Retention time 2.35mins/(ES-) 195 (M-H, $C_7H_7F_3O_3$ requires 196).

**Description 4: 3-(trifluoromethyl)-1,4,6,7-tetrahydropyrano[4,3-*c*]pyrazole (D4)**

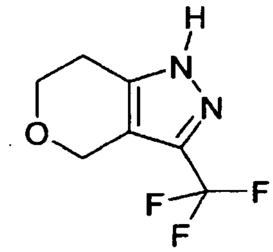

A mixture of 3-(trifluoroacetyl)tetrahydro-4*H*-pyran-4-one (D4, 5.42g, 27.7mmol), and hydrazine hydrate (1.38g, 1.4ml, 27.6 mmol) in ethanol (120ml) was stirred at 60˚C under argon for 6 hours. A further 0.7ml (14mmol) of hydrazine hydrate was added and the reaction stirred at 70˚C for 3 hours. The reaction mix was allowed to cool and the solvent removed by rotary evaporation. The residue was partitioned between dichloromethane and water. The organic layer was separated, dried over sodium sulphate, and solvent removed by rotary evaporation. The aqueous layer was neutralised with 2N HCl and re-extracted with dichloromethane. The organic layer was separated, dried over sodium sulphate, and the solvent removed by rotary evaporation. The 2 extracts were combined to give the title compound as a yellow solid (3.64g, 68%).
$^1$H-NMR (400MHz, $CDCl_3$) δ: 11.32 (1H, br s), 4.76 (2H, s), 3.95 (2H, m), 2.83 (2H, m); LC/MS Retention time 1.92mins/ (ES-) 191 (M-H, $C_7H_7F_3N_2O$ requires 192).

**Description 5: 3-(trifluoromethyl)-1,4,5,7-tetrahydropyrano[3,4-*c*]pyrazole (D5)**

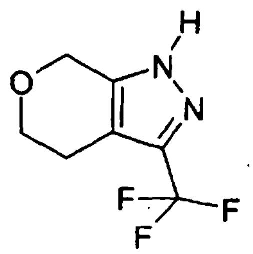

To a solution of (5,6-dihydro-2*H*-pyran-3-yloxy)(trimethyl)silane (prepared according to Liebigs Annalen Der Chemie 1984, 11, 1759-1882; - 28.7g, 167mmol) in dry THF (570ml), a solution of Methyllithium 1.6M in diethyl ether (104ml), 167mmol) was added dropwise under argon at room temperature. After 2.5 hours the mixture was cooled to -78˚C, and then treated dropwise at this temperature with a solution of ethyl trifluoroacetate (23.7g, 19.9ml, 167mmol) in dry THF (20 ml). The mixture was allowed to slowly warm up to room temperature, stirred for 2 hours then quenched with sat.$NH_4Cl$ solution (250ml), keeping the internal temperature below 10˚C. The two layers were separated and the aqueous extracted twice with ethyl acetate (each with 250ml). The resulting combined organic phase was finally dried over sodium sulphate and the solvent removed by rotary evaporation to give the intermediate 4-(trifluoroacetyl)dihydro-2*H*-pyran-3(4*H*)-one as a foamy yellow solid (32.7g, quantitative).

This intermediate (32.7g, 167mmol) was dissolved in ethanol (570ml) and to the solution hydrazine hydrate (16.7g, 16.6ml, 334mmol) was added at room temperature. The resulting mixture was stirred at reflux temperature for 6 hours, then it was allowed to cool down to room temperature and the solvent was removed by rotary evaporation. The residue was partitioned between dichloromethane (400ml) and water (200ml). The aqueous layer was extracted twice with dichloromethane (each with 150ml). The combined organic layers were washed with water (200ml), brine (150ml) and dried over sodium sulphate. The solvent was removed by rotary evaporation to obtain 20.47g of a yellow solid.

Another two batches of the same compound were prepared from the same procedure (the first of 5.6g was obtained starting from 8.2g of (5,6-dihydro-2*H*-pyran-3-yloxy)(trimethyl)silane and the second of 1g was obtained starting from 2g of (5,6-dihydro-2*H*-pyran-3-yloxy)(trimethyl)silane.

The three combined residues were purified by $SiO_2$ flash chromatography eluting with cyclohexane / ethyl acetate from 7/3 to 1/1. The desired product was isolated as a white solid (17.2g, 40%).
$^1$H-NMR (400MHz, $CDCl_3$) δ: 13.28 (1H, br s), 4.68 (2H, s), 3.80 (2H, t), 2.61 (2H, m);

**Description 6: 1-tert-butoxycarbonyl-4-(trifluoroacetyl)-3-piperidinone (D6)**

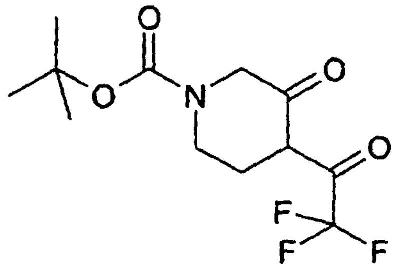

A solution of lithium diisopropylamide in THF (40.2ml, 2M) was cooled to -78˚C with stirring under argon. To this was added a solution of the 1-tert-butoxycarbonyl-3-piperidinone 16g, 80.4mmole) in THF (100ml) with stirring under argon. The mixture was stirred at -78˚C for 20mins before being treated dropwise with ethyl trifluoroacetate (9.56ml, 80.4mmol). The reaction mixture was allowed to warm up to room temperature and stirred for 2h. The reaction mixture was quenched with water and neutralized with dilute aqueous hydrochloric acid. The reaction mixture was extracted with ethyl acetate. The organic layer was dried over sodium sulphate and evaporated under reduced pressure to give the title compound as an orange foamy solid (23.6g, crude material).
LC/MS Retention time 3.31 mins/(ES-) 294 (M-H, $C_{12}H_{16}F_3NO_4$ requires 295).

**Description 7: 6-tert-butoxycarbonyl-3-(trifluoromethyl)-4,5,6,7-tetrahydro-1H-pyrazolo[3,4-c]pyridine (D7)**

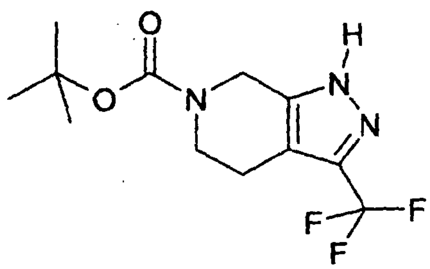

A solution of 1-tert-butoxycarbonyl-4-(trifluoroacetyl)-3-piperidinone (D6, 23.6g, 0.08mol) in ethanol (250ml) was treated with hydrazine monohydrate (4.16ml, 0.086mol). The mixture was allowed to stir at 50˚C for 24h. The reaction mixture was cooled to room temperature and then evaporated under reduced pressure. The resulting mixture was partitioned between ethyl acetate and brine. The organic layer was separated and dried over sodium sulphate and evaporated under reduced pressure. Residual material was recrystallised from ethyl acetate / n-pentane to give the title compound as a pale yellow solid (6.5g, 28%).
$^1$H-NMR (400MHz, $CDCl_3$) δ: 11.13 (1H, br s), 4.40 (2H, m), 3.66 (2H, m), 2.70 (2H, m), 1.48 (9H, s); LC/MS Retention time 2.90mins/(ES-) 290 (M-H, $C_{12}H_{16}F_3N_3O_2$ requires 291).

**Description 8: 6-methyl-3-(trifluoromethyl)-4,5,6,7-tetrahydro-1*H*-pyrazolo[3,4-*c*]pyridine (D8)**

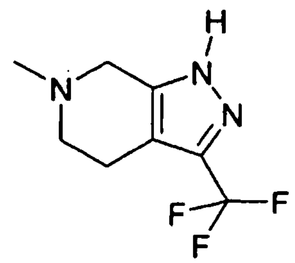

Under an inert atmosphere of argon, $LiAlH_4$ (2.3 M in. THF; 7.5 mL; 17.3 mmol) was added dropwise over 2 minutes to a cool (0°C) stirring solution of 6-tert-butoxycarbonyl-3-(trifluoromethyl)-4,5,6,7-tetrahydro-1H-pyrazolo[3,4-4] pyridine (D7, 2.00 g; 8.45 mmol) in anhydrous THF (42 mL). The resulting mixture was stirred in an oil bath at 58°C for 17 hours, cooled to 0°C, and quenched by the careful addition of an aqueous solution of sodium potassium tartrate (1 M; 50mL). After stirring at room temperature for 1 hour, the mixture was diluted with diethyl ether (50 mL) and more aqueous sodium potassium tartrate (1 M; 50mL). After stirring at this temperature for a further 1 hour, the mixture was partitioned between water (100 mL) and diethyl ether (200 mL). The separated aqueous phase was extracted with ethyl acetate (200 mL), and the combined organic phase was dried ($MgSO_4$) and concentrated in vacuo. The resulting off-white solid (1.39 g) was purified using an SCX column giving the title compound as a yellow solid (1.23 g; 6.01 mmol) $^1$H-NMR (400MHz, $CDCl_3$) δ: 5.01 (1H, br s), 3.58 (2H, app s), 2.74 (4H, app s), 2.48 (3H, app s); LC/MS Retention time 1.81 mins/(ES+) 206 (M+H, $C_8H_{10}F_3N_3$ requires 205).

**Description 9: 5-bromo-2-(bromomethyl)-1,3-difluorobenzene (D9)**

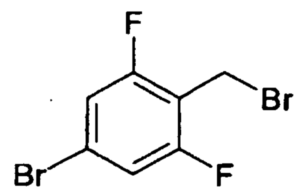

A solution of 4-Bromo-2,6-difluorobenzyl alcohol (3.517g, 15.77mmol) and triphenylphosphine (4.55g, 17.34mmol) in dichloromethane (70ml) was cooled to 0°C and N-bromosuccinimide (3.086g, 17.34mmol) was added in 5 portions over 20mins. The solution was warmed to 25°C and stirred for 16h. The reaction was quenched by the addition of dilute aqueous sodium bicarbonate. The resulting mixture was extracted with diethyl ether and the combined organic layers were washed with water, then brine and dried over sodium sulphate and concentrated under reduced pressure. The title compound was isolated by column chromatography on silica using 5 to 20% ethyl acetate in n-pentane to give the title compound as an oil (3.785g, 84%). $^1$H-NMR (400MHz. $CDCl_3$) δ: 7.10 (2H, m), 4.44 (2H, s); LC/MS Retention time 3.38mins/ M+H not observed.

**Description 10: 1-[(4-bromo-2,6-difluorophenyl)methyl]-2-pyrrolidinone (D10)**

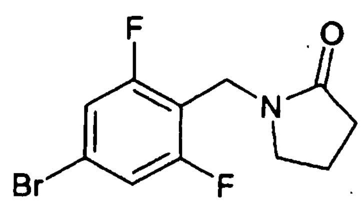

To a solution of 2-pyrrolidone (1.124g, 13.23mmol) in DMF (35ml) was added sodium hydride (60% in oil, 14.55mmol, 0.583g) portionwise under argon at room temperature and stirred for 15mins. Then 5-bromo-2-(bromomethyl)-1,3-difluorobenzene (D9, 3.785g, 13.23mmol) was added. The resulting mixture was allowed to stir at room temperature overnight. Then the reaction mixture was quenched by the addition of water (2ml). The DMF was evaporated off under reduced pressure and partitioned between ethyl acetate and water, organic layer was washed with brine and dried over sodium sulphate. The solvent was removed by rotary evaporation to give an oil which was purified by column chromatography on silica using 10 to 100% ethyl acetate in n-pentane to give the title compound as an oil which then solidified on standing (3.188g, 83%).
$^1$H-NMR (400MHz, $CDCl_3$) δ: 7.10 (2H, m), 4.53 (2H, s), 3.27 (2H, m), 2.37 (2H, m), 1.97 (2H, m); LC/MS Retention time 2.56mins/(ES+) 290, 292 (M+H, $C_{11}H_{10}$ $BrF_2NO$ requires 289 and 291).

**Description 11: 1-[(4-bromo-2-fluorophenyl)methyl]-2-pyrrolidinone (D11)**

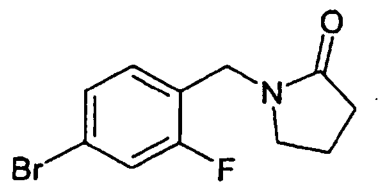

The title compound was prepared from 2-fluoro-4-bromo-benzylbromide (4.0g, 14.9mmol), 2-pyrrolidone (1.27g, 14.92mmol), sodium hydride (60% in oil, 0.656g, 16.41mmol) and DMF (40ml) according to the procedure described in Description 10 (3.946g, 97% yield).
$^1$H-NMR (400MHz, $CDCl_3$) δ: 7.13-7.28 (3H, m), 4.46 (2H, s), 3.31 (2H, m), 2.43 (2H, m), 2.0 (2H, m); LC/MS Retention time 2.56mins/(ES+) 272, 274 (M+H, $C_{11}H_{11}BrFNO$ requires 271 and 273).

**Decscription 12: 1-[(4-bromo-2-fluorophenyl)carbonyl]pyrrolidine (D12)**

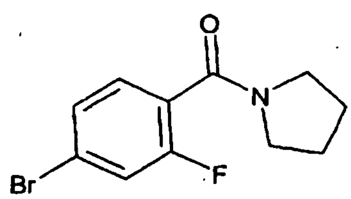

A mixture of 4-bromo-2-fluorobenzoic acid (2.0g, 9.13mmol), pyrrolidine (0.650g, 9.13mmol), diisopropylethylamine (3.17ml, 18.26mmol) in dimethylformamide (20ml) was stirred at room temperature under argon. Then HATU was added (3.818g, 10mmol). The reaction mixture was allowed to stir at room temperature for 16 h. Solvent was removed under reduce pressure. Residual material was partitioned between ethyl acetate and water, the organic layer was separated, dried over sodium sulphate, and solvent evaporated off under reduced pressure. The desired product was isolated by column chromatography on silica using 5 to 100% ethyl acetate in n-pentane to give the title compound as a colourless oil which solidified on standing (1.69g, 68%).
$^1$H-NMR (400MHz, $CDCl_3$) δ: 7.40-7.26 (3H, m), 3.64 (2H, m), 3.29 (2H,m), 1.98 (2H, m), 1.90 (2H,M); LC/MS Retention time 2.56mins/(ES+) 272, 274 (M+H, $C_{11}H_{11}NOFBr$ requires 271 and 273).

**Description 13: 1-methyl-3-(trifluoroacetyl)-4-piperidinone (D13)**

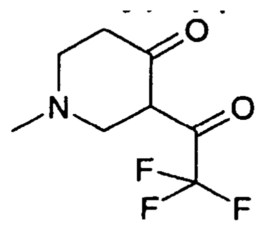

A solution of N-methyl-4-pyridone (1.13g, 10mmol) in hexane (20ml) was cooled in an ice/methanol bath with stirring under argon. Lithium hydride (95%, 84mg, 11mmol) was added portionwise and the resulting mix stirred under argon for 15 minutes. Ethyl trifluoroacetate (1.42g, 10mmol) was added dropwise and the mixture allowed to warm to room temperature and stirred for 18 hours. The hexane was removed under reduced pressure and the residue was partitioned between ethyl acetate (20ml) and water (20ml). The organic layer was dried over sodium sulphate and evaporated to give a yellow oil (250mg). The aqueous layer was evaporated under reduced pressure and the residue suspended in dichloromethane. This mix was dried over sodium sulphate and filtered. The solvent was removed under reduced pressure to give the title compound as a yellow oil (627mg in total, 30%).
$^1$H-NMR (400MHz, $CDCl_3$) δ: 2.72 (3H, m), 2.60-2.43 (3H, m), 2.43-2.26 (4H, m).

**Description 14: 5-methyl-3-(trifluoromethyl)-4,5,6,7-tetrahydro-1*H*-pyrazolo[4,3-*c*]pyridine (D14)**

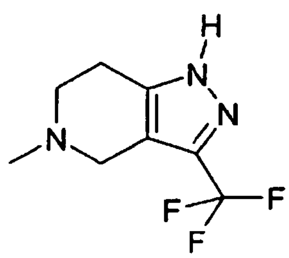

A solution of 1-methyl-3-(trifluoroacetyl)-4-piperidinone (D13, 627mg, 3mmol) in ethanol (15ml) was treated with hydrazine monohydrate (150mg, 3mmol) and the reaction mix stirred at 50°C for 4h. The reaction mix was cooled and the solvent removed by rotary evaporation. The residue was partitioned between dichloromethane and water. The organic layer was separated, dried over sodium sulphate, and evaporated under reduced pressure to give the title compound as a yellow oil (107mg crude).
LC/MS Retention time 0.76mins/(ES+) 206 (M+H, $C_8H_{10}F_3N_3$ requires 205).

**Description 15: 2-[(4-bromophenyl)methyl]isothiazolidine 1,1-dioxide (D15)**

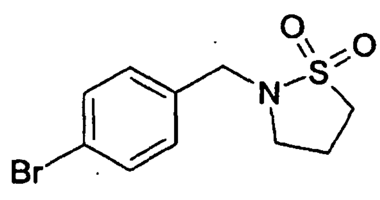

A solution of 4-bromobenzylamine (1.85g, 10mmol) and triethylamine (2g, 20mmol) in dimethylformamide (30ml) was treated with 3-chloropropanesulfonyl chloride (1.78g, 10mmol) by dropwise addition over 10 minutes with stirring under argon. This mix was stirred for 30 minutes before being treated with a 60% suspension of sodium hydride in mineral oil (1.2g, 30mmol of NaH) portionwise and the whole mix stirred at room temperature for 3 days. The reaction mixture was partitioned between water (50ml) and dichloromethane (30ml). The organic layer was dried over sodium sulphate and reduced to minimum volume by rotary evaporation. The residue was added to a 20g pre-packed silica column and eluted from 0-50% ethyl acetate in petroleum ether to give the title compound as a yellow oil (2.72g, 94%).

$^1$H-NMR (400MHz, $CDCl_3$) δ: 7.49 (2H, m), 7.24 (2H, m), 3.21 (2H, m), 3.13 (2H, s), 3.11 (2H, m), 2.32 (2H, m); LC/MS Retention time 2.68mins/(ES+) 290 (M+H, $C_{10}H_{12}{}^{79}BrNO_2S$ requires 289).

**Description 16: 1-acetyl-2-(4-bromophenyl)pyrrolidine (D16)**

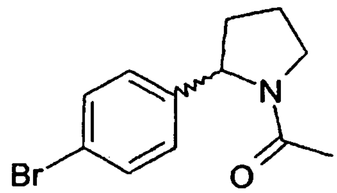

A solution of 2-(4-bromophenyl)pyrrolidine (504mg, 2.24mmol) was made up in a mixture of dichloromethane (8ml) and triethylamine (2.7mmol, 0.38ml) in a sealed 15ml plastic tube. Acetyl chloride (2.46mmol, 0.18ml), was added dropwise and the whole mixture shaken at room temperature for 1 hour. The reaction mixture was washed with water (5ml) and the organic layer was removed and dried over sodium sulphate. The solvent was removed under reduced pressure to give the title compound as a golden brown coloured oil (526mg, 88%).
LC/MS Retention time 2.59mins/(ES+) 268 (M+H, $C_{12}H_{14}{}^{79}BrNO$ requires 267).

**Description 17: 1-[(4-iodophenyl)carbonyl]azetidine (D17)**

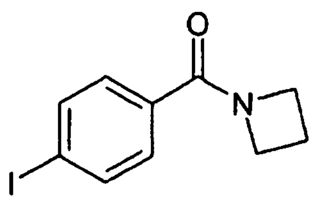

The title compound was prepared from 4-iodobenzoic acid and azetidine hydrochloride using a similar procedure to that described for Description 2.
$^1$H-NMR (400MHz, $CDCl_3$) δ: 7.76 (2H, m), 7.36 (2H, m), 4.29 (2H, m), 4.21 (2H, m), 2.35 (2H, m); LC/MS Retention time 2.40mins/(ES+) 288 (M+H, $C_{10}H_{10}INO$ requires 287).

**Description 18: 1-tert-butoxycarbonyl-3-(trifluoroacetyl)-4-piperidinone (D18)**

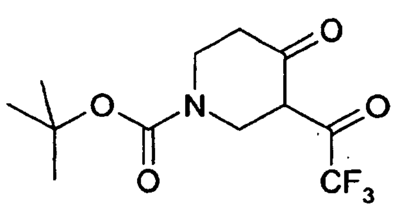

A solution of 1-tert-butoxycarbonyl-4-piperidinone (1.99g, 10mmol), in tetrahydrofuran (20ml) was cooled to -70˚C and stirred under argon. To the reaction mixture lithium diisopropylamide solution (2M in tetrahydrofuran, 5ml) was added dropwise over 20 minutes. The reaction was allowed to stir for 5 minutes, then ethyl trifluoroacetate (1.2ml), 10mmol) was added dropwise over 10 minutes, the reaction mixture was then allowed to return to room temperature and stirred under argon for 2 hours. The reaction mixture was then cooled to -70˚C, quenched with water and acidified to pH6 using 2M hydrochloric acid (aq). Reaction mixture was separated between ethyl acetate and water. The organic

layer was retained, the aqueous layer was washed with further ethyl acetate and the organic layers were combined and the solvent removed by rotary evaporation. Sample was then eluted through 5g pre-packed silica column using ethyl acetate to give the title compound (2.20g, 75%).
LC/MS Retention time 3.19mins/(ES-) 294 (M-H, $C_{12}H_{16}F_3NO_4$ requires 295).

**Description 19: 5-tert-butoxycarbonyl-3-(trifluoromethyl)-4,5,6,7-tetrahydro-1H-pyrazolo[4,3-c]pyridine (D19)**

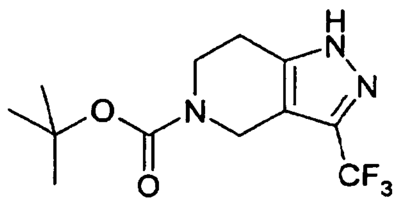

To a stirring solution of 1-tert-butoxycarbonyl-3-(trifluoroacetyl)-4-piperidinone (D18, 2.20g, 7.5mmol) in ethanol (25ml) hydrazine hydrate (375mg, 7.5mmol) was added in one portion and the reaction mixture heated at 50˚C under an atmosphere of argon for 18 hours. Reaction mixture was quenched with acetone, then the solvent was removed by rotary evaporation. Residue was separated between dichloromethane and water, organic layer retained and dried with sodium sulphate. The solvent was removed by rotary evaporation to give the title compound (1.41g, 65%).
$^1$H-NMR (400MHz, $CDCl_3$) δ: 4.36 (1H, s), 3.72 (1H, t, J=6Hz), 3.64 (1H, t, J=6Hz), 2.60 (1H, m), 2.44 (1H, t, J=6Hz), 1.57 (2H, m), 1.47 (9H, m).

**Description 20: 4-[3-(trifluoromethyl)-6,7-dihydropyrano[4,3-*c*]pyrazol-1(4*H*)-yl]benzoic acid (D20)**

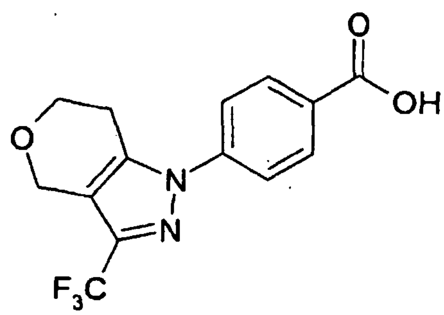

3-(trifluoromethyl)-1,4,6,7-tetrahydropyrano[4,3-*c*]pyrazole (D4, 1.08g, 5.6mmol), 4-iodobenzoic acid (1.39g, 5.6mmol), copper (I) iodide (10mol%, 107mg, 0.56mmol), N,N-dimethylglycine (20mol%, 116mg, 1.12mmol), potassium carbonate (1.62g, 11.7mmol) and dimethylsulfoxide (15ml) were added sequentially to a 20ml microwave vial. Reaction mixture was heated in an oil bath at 130˚C for 3.25 hours. The reaction mixture was then filtered under vacuum and the filtrate separated between ethyl acetate and water. The aqueous layer was retained and acidified to pH2 using 5M hydrochloric acid (aq). Aqueous fraction was then washed with ethyl acetate (x3). Organic layers were combined and dried over sodium sulphate and the solvent removed by rotary evaporation to give the title compound as a yellow solid (346mg, 20%).
$^1$H-NMR (400MHz, $CDCl_3$) δ: 8.24 (2H, m), 7.69 (2H, d, J=9Hz), 4.82 (2H, m), 3.96 (2H, t, J=6Hz), 2.97 (2H, m); LC/MS Retention time 2.75mins/(ES+) 313 (M+H, $C_{14}H_{11}F_3N_2O_3$ requires 312).

**Description 21: 3,3-difluoro-1-[(4-iodophenyl)carbonyl]azetidine (D21)**

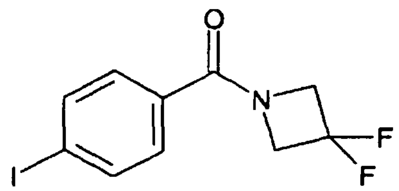

A suspension of 4-iodobenzoic acid (992mg, 4.0mmol) in dichloromethane (16ml) was treated portionwise with 1,1'-carbonyldiimidazole (648mg, 4.0mmol) over 5 minutes and then allowed to stir at room temperature for 30 minutes, then 3,3-difluoroazetidine hydrochloride (518mg, 4.0mmol) was added in one portion followed by triethylamine (444mg, 0.61 ml, 4.4mmol) and the whole mix stirred at room temperature for 1 hour under argon. The reaction mixture was then washed with saturated sodium bicarbonate. The organic layer was separated and dried over sodium sulphate. The solvent was removed under reduced pressure to give the title compound as a cream-coloured solid (745mg, 58%).
$^1$H-NMR (400MHz, $CDCl_3$) δ: 7.81 (2H, m), 7.37 (2H, m), 4.53 (4H, t, J=12Hz); LC/MS Retention time 2.73mins/(ES+) 324 (M+H, $C_{10}H_8F_2INO$ requires 323).

**Description 22: 1-[(4-bromo-3-fluorophenyl)carbonyl]pyrrolidine (D22)**

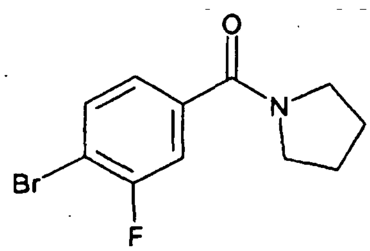

A suspension of 4-bromo-3-fluorobenzoic acid (1.09g, 5mmol) in dichloromethane (15ml) was treated with 1,1'-carbonyldiimidazole (810mg, 5mmol) in one portion and allowed to stir at room temperature for 15 minutes. Pyrrolidine (355mg, 5mmol) was then added and stirring continued for 1 hour. The reaction mixture was washed with saturated sodium bicarbonate solution. The organic layer was dried over sodium sulphate and concentrated by rotary evaporation to give the title compound as a yellow oil (1.17g, 86%).
$^1$H-NMR (400MHz, $CDCl_3$) δ: 7.51 (1H, m), 7.29 (1H, m), 7.16 (1H, m), 3.63 (2H, m), 3.42 (2H, m), 1.87-2.01 (4H, m); LC/MS Retention time 2.55mins/(ES+) 272 & 274 (M+H, $C_{11}H_{11}BrFNO$ requires 271 & 273).

**Description 23: 4-(bromomethyl)-2-fluoro-1-iodobenzene (D23)**

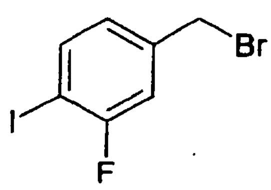

A mixture of 3-fluoro-4-iodotoluene (2.26g, 9.58mmol), N-bromosuccinimide (2.03g, 11.49mmol) and benzoyl peroxide (5mol%, 116mg, 0.48mmol) in carbon tetrachloride (5ml) was stirred at reflux for 20 hours, cooled, diluted with dichloromethane and filtered. The purple coloured filtrate was washed with saturated sodium thiosulphate solution (aq) (20ml). The organic layer was separated, dried over sodium sulphate and the solvent removed by rotary evaporation to give the title compound as a yellow oil (2.58g, 86%).
$^1$H-NMR (250MHz. $CDCl_3$): δ 7.72 (1H, m), 7.11 (1H, m), 6.94 (1H, m), 4.41 (2H, s),

**Description 24: 1-[(3-fluoro-4-iodophenyl)methyl]-2-pyrrolidinone (D24)**

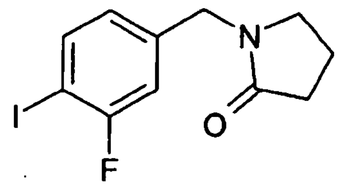

A solution of 2-pyrrolidinone (765mg, 9mmol) in anhydrous dimethylformamide (25ml) was cooled in an ice/methanol bath with stirring under argon, and then treated with sodium hydride (60% suspension in mineral oil, 360mg, 9mmol) over 10 minutes. The reaction mix was stirred with cooling for 0.5 hours before 4-(bromomethyl)-2-fluoro-1-iodobenzene (D23, 2.58g, 8.2mmol) in dimethylformamide (5ml) was added dropwise over 10 minutes. The whole mix was stirred at room temperature for 1 hour. The reaction mix was quenched with water (1ml), and then partitioned between water (30ml) and dichloromethane (20ml), the aqueous layer was washed again with dichloromethane (10ml). The combined organic fractions were washed twice with water (20ml) and then with brine (10ml), dried over sodium sulphate and the solvent removed via rotary evaporation to give a brown oil (2.45g) which was added to a 20g isolute pre-packed silica gel column and eluted with 0-75% ethyl acetate in petroleum ether. The relevant fractions were combined and the solvent removed by rotary evaporation to give the title compound as a yellow solid (1.27g, 49%).
$^1$H-NMR (400MHz, $CDCl_3$) δ: 7.69 (1H, m), 6.95 (1H, m), 6.81 (1H, m), 4.41 (2H, s), 3.28 (2H, m), 2.46 (2H, m), 2.05 (2H, m); LC/MS Retention time 2.66mins/(ES+) 320 (M+H, $C_{11}H_{11}FINO$ requires 319).

**Description 25: *N*-[(4-bromophenyl)methyl]methanesulfonamide (D25)**

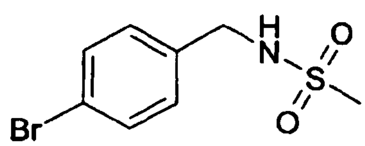

A solution of 4-bromobenzylamine (2.50g, 13.5mmol) and triethylamine (14.9mmol, 1.5g, 2.1ml) in dichloromethane (50ml) was cooled in an ice bath with stirring under argon. Methanesulfonyl chloride (1.55g, 13.7mmol, 1.05ml) was added dropwise and the resulting mix was allowed to warm up to room temperature and stirred for 1 hour. The reaction mix was washed 3 times with water (20ml) and the organic layer deied over sodium sulphate and reduced to minimum volume under reduced pressure to give the title compound as a colourless solid (3.45g, 97%).
$^1$H-NMR (400MHz, $CDCl_3$) δ: 7.51 (2H, m), 7.25 (2H, m), 4.67 (1H, m), 4.29 (2H, d, J=6Hz), 2.90 (3H, s); LC/MS Retention time 2.39mins/(ES-) 262 & 264 (M-H, $C_8H_{10}BrNO_2S$ requires 263 & 265).

**Description 26: *N*-[(4-iodophenyl)methyl]methanesulfonamide (D26)**

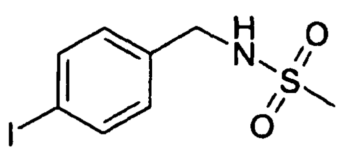

The title compound was prepared from 4-iodobenzylamine hydrochloride and methanesulfonyl chloride with triethylamine (2.1 equivalents) using a similar procedure to that described for Description 25.

$^1$H-NMR (400MHz, $CDCl_3$) δ: 7.70 (2H, m), 7.10 (2H, d, J=8Hz), 4.60 (1H, m), 4.27 (2H, m), 2.89 (3H, s)

**Description 27: 4-iodo-*N*-(tetrahydro-2-furanylmethyl)benzenesulfonamide (D27)**

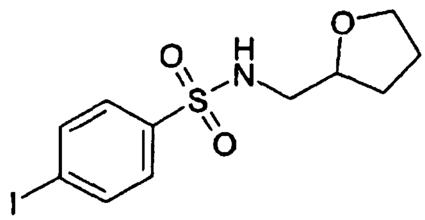

A solution of (tetrahydro-2-furanylmethyl)amine (0.167g, 1.65mmol) and triethylamine (0.35ml, 2.48mmol) in dichloromethane (10ml) was stirred in an ice/water bath with stirring under argon, and then 4-iodobenzenesulfonyl chloride (0.500g, 1.65mmol) was added dropwise with stirring. The resulting mixture was allowed to stir at room temperature for 16 hours. Then the solution was washed with water, organic layer separated, dried with sodium sulphate and the solvent was removed by rotary evaporation to give the title compound as a white solid (0.596g, 98%).
$^1$H-NMR (400MHz, $CDCl_3$) δ: 7.87 (2H, m), 7.57 (2H, m), 4.81 (1H, m), 3.91 (1H, m), 3.77 (1H, m), 3.70 (1H, m), 3.13 (1H, m), 2.88 (1H, m), 1.98 - 1.84 (3H, m), 1.58 (1H, m); LC/MS Retention time 2.70mins/(ES+) 368 (M+H, $C_{11}H_{14}INO_3S$ requires 367).

**Description 28: 2-fluoro-4-[3-(trifluoromethyl)-6,7-dihydropyrano[4,3-*c*]pyrazol-1(4*H*)-yl]benzoic acid (D28)**

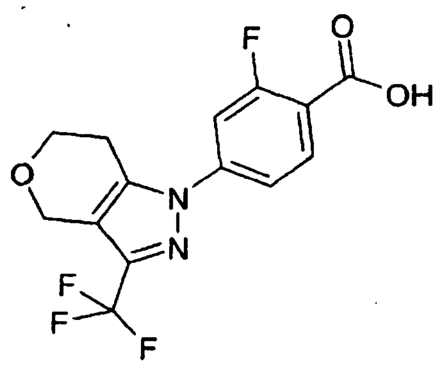

A mixture of 3-(trifluoromethyl)-1,4,6,7-tetrahydropyrano[4,3-*c*]pyrazole (D4, 500mg, 2.60mmol), copper (I) iodide (500mg, 2.60mmol), potassium carbonate (720mg, 5.20mmol) and dimethylsulfoxide (8ml) was stirred for 5 minutes, 4-bromo-2-fluorobenzoic acid (570mg, 2.60mmol) and N,N-dimethylglycine (267mg, 2.60mmol) were then successively added. The reaction mixture was heated with stirring at 130˚C under argon for 10 hours, then the reaction mixture was diluted with ethyl acetate and water, filtered through kieselguhr to remove the catalyst, aqueous layer was retained and acidified to pH -2 using 5 N aqueous hydrochloric acid. Aqueous fraction was then extracted with ethyl acetate (x3). Organic layers were combined and dried over sodium sulphate, filtered and the solvent was removed by rotary evaporation to give the title compound as a crude dark brown solid >75% pure (850mg, 74%).
$^1$H-NMR (400MHz, DMSO-$d_6$) δ: 8.04 (1H, m), 7.68 (2H, m), 4.73 (2H, s), 3.86 (2H, m), 3.03 (2H, m), 13.47 (1H, br s); LC/MS Retention time 2.73mins/(ES+) 331 (M+H, $C_{14}H_{10}F_4N_2O_3$ requires 330).

**Description 29: (4-bromo-2-fluorophenyl)acetonitrile (D29)**

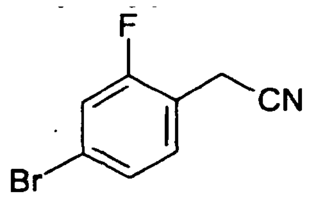

A solution of 4-bromo-1-(bromomethyl)-2-fluorobenzene (1.0g, 3.73mmol) in ethanol (5ml) and water (1ml) was treated with sodium cyanide (219mg, 4.48mmol) and refluxed for 3 hours. The volatiles were evaporated under reduced pressure and the residue was diluted with water and extracted with diethyl ether (x 2). The combined organic extracts were washed with water and brine, dried over anhydrous sodium sulphate, filtered and evaporated under reduced pressure to afford the desired product as a pale yellow solid (0.748g, 93%).
$^1$H-NMR (400MHz, $CDCl_3$) δ: 7.37 - 7.26 (3H, m), 3.72 (2H, s); LC/MS Retention time 2.76mins/ M+H not observed $C_8H_5{}^{79}BrFN$ requires 213.

**Description 30: (4-bromo-2-fluorophenyl)acetic acid (D30)**

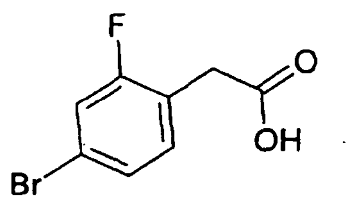

A solution of (4-bromo-2-fluorophenyl)acetonitrile (D29, 2.354g, 10.9mmol) in ethanol (60ml) and water (18ml) was treated with potassium hydroxide (5.2g, 93.09mmol) and refluxed for 5 hours. The volatiles were evaporated off under reduced pressure and the residue was diluted with water and poured into cold, dilute hydrochloric acid and the precipitated solid was filtered. The solid was dried under high vacuum at 37˚C for 48 hours (2.283g, 89.7%).
$^1$H-NMR (400MHz, DMSO-$d_6$) δ: 12.57 (1H, br s), 7.52 (1H, m), 7.38 (1H, m), 4.32 (1H, m), 3.62 (2H, s); LC/MS Retention time 2.34mins/ M+H not observed $C_8H_8{}^{79}BrFO_2$ requires 232.

**Description 31: 1-[(4-bromo-2-fluorophenyl)acetyl]pyrrolidine (D31)**

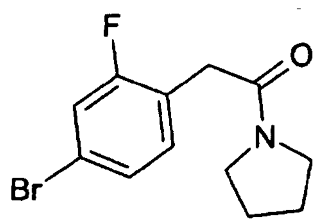

A mixture of (4-bromo-2-fluorophenyl)acetic acid (D30, 1.0g, 4.29mmol), pyrrolidine (305mg, 4.29mmol) and N,N-diisopropylethylamine (1.49ml, 8.58mmol) in dimethylformamide (15ml) was stirred at room temperature under argon and then HATU (1.79g, 4.72mmol) was added. The resulting mixture was allowed to stir at room temperature under argon overnight. The solvent was removed under reduced pressure and the residue was partitioned between ethyl acetate and water, organic layer separated, dried over sodium sulphate, filtered and evaporated in vacuo. The desired product was isolated by column chromatography on silica using 5 to 100% ethyl acetate in n-pentane to give the title compound as a white solid (0.978g, 79%).
$^1$H-NMR (400MHz, $CDCl_3$) δ: 7.24 (3H, m), 3.60 (2H, s), 3.48 (4H, m), 1.98 (2H, m), 1.86 (2H, m); LC/MS Retention time 2.61mins/(ES+) 286, 288 (M+H, $C_2H_{13}NBrFO$ requires 285, 287).

**Description 32: 1-[(4-bromo-2-fluorophenyl)carbonyl]azetidine (D32)**

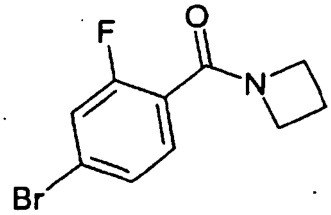

A mixture of 4-bromo-2-fluorobenzoic acid (2.0g, 9.13mmol), azetidine hydrochloride (939mg, 10.0mmol), N, N-diisopropylethylamine (3.18ml, 18.26mmol) and HATU (3.47g, 9.13mmol) in dimethylformamide (15ml) was stirred at room temperature under argon for 16 hours. Solvent was removed under reduced pressure and the resulting mixture was partitioned between ethyl acetate and water, organic layer separated, dried over sodium sulphate, filtered and evaporated under reduced pressure. The desired product was isolated by column chromatography on silica using 10 to 100% ethyl acetate in n-pentane to give the title compound as a white gum (2.1g, 89%).
$^1$H-NMR (400MHz, $CDCl_3$) δ: 7.43 (1H, m), 7.36 (1H, m), 7.27 (1H, m), 4.22 (2H, m), 4.10 (2H, m), 1.44 (2H, m); LC/MS Retention time 2.36mins/(ES+) 258 (M+H, $C_{10}H_9N^{79}BrFO$ requires 257).

**Description 33: 1-[(4-bromo-2-fluorophenyl)carbonyl]-3,3-difluoroazetidine (D33)**

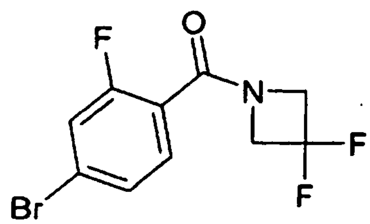

The title compound was prepared from 4-bromo-2-fluorobenzoic acid and 3,3-difluoroazetidine hydrochloride using a similar procedure to that described for Description 32.
$^1$H-NMR (400MHz, $CDCl_3$) δ: 7.51 (1H, m), 7.40 (1H, m), 7.32 (1H, m), 4.53 - 4.39 . (4H, m); LC/MS Retention time 2.67mins/(ES+) 294, 296 (M+H, $C_{10}H_7BrNF_3O$ requires 293, 295).

**Example 1: 1-[4-(1-pyrrolidinylcarbonyl)phenyl]-3-(trifluoromethyl)-1,4,6,7-tetrahydropyrano[4,3-*c*]pyrazole**

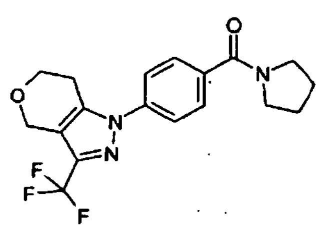

A mixture of 1-[(4-iodophenyl)carbonyl]pyrrolidine (D2, 843mg, 2.80mmol), 3-(trifluoromethyl)-1,4,6,7-tetrahydropyrano[4,3-*c*]pyrazole (D4, 563mg, 2.93mmol), copper (I) oxide (10mol%, 0.3mmol, 43mg), N,N-dimethylglycine (20mol%, 0.6mmol, 62mg) and cesium carbonate (5.8mmol, 1.89g) in dimethylsulfoxide (8ml) was stirred at 130˚C (oilbath temperature) for 16h. The reaction mix was cooled and then partitioned between water (30ml) and dichloromethane (2 x 20ml). The organic layers were dried over sodium sulphate and the solvent removed under reduced pressure.

The crude product was added to a 20g isolute pre-packed silica gel sep-pak column and eluted from 0-75% ethyl acetate in petroleum ether. The solvent was removed under reduced pressure to give the title compound as a yellow solid (616mg, 60%).
$^1$H-NMR (400MHz, $CDCl_3$) δ: 7.66 (2H, m), 7.58 (2H, m), 4.81 (2H, s), 3.94 (2H, t, J=6Hz), 3.67 (2H, t, J=7Hz), 3.44 (2H, t, J=7Hz), 2.90 (2H, t, J=6Hz), 2.03-1.88 (4H, m); LC/MS Retention time 2.85mins/(ES+) 366 (M+H, $C_{18}H_{18}F_3N_3O_2$ requires 365).

**Example 2: 1-({4-[3-(trifluoromethyl)-6,7-dihydropyrano[4,3-*c*]pyrazol-1(4*H*)-yl]phenyl}methyl)-2-pyrrolidinone**

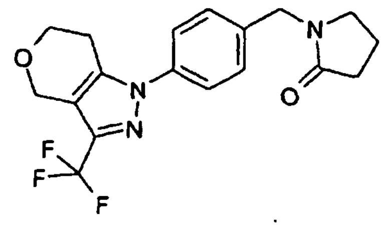

A mixture of 1-[(4-iodophenyl)methyl]-2-pyrrolidinone (D1, 150mg, 0.5mmol), 3-(trifluoromethyl)-1,4,6,7-tetrahydropyrano[4,3-*c*]pyrazole (D4, 96mg, 0.5mmol), copper (I) iodide (10mol%, 0.05mmol, 10mg), N,N-dimethylglycine (20mol%, 0.1mmol, 10mg) and potassium carbonate (1mmol, 138mg) in dimethylsulfoxide (2ml) was stirred at 190˚C in a microwave reactor for 30mins. The reaction mix was cooled and then partitioned between water (5ml) and dichloromethane (5ml). The organic layer was added to a 5g isolute pre-packed silica gel sep-pak column and washed through with ethyl acetate. The solvent was removed under reduced pressure and the residue purified via mass-directed auto-preparation to give the title compound as a yellow oil (122mg, 67%).
$^1$H-NMR (400MHz, $CDCl_3$) δ: 7.48 (2H, m), 7.37 (2H, d, J=8Hz), 4.80 (2H, s), 4.51 (2H, s), 3.93 (2H, t, J=6Hz), 3.29 (2H, m), 2.87 (2H, t, J=6Hz), 2.46 (2H, m), 2.03 (2H, m); LC/MS Retention time 2.72mins/(ES+) 366 (M+H, $C_{18}H_{18}F_3N_3O_2$ requires 365).

**Example 3: 1-[4-(1-pyrrolidinylcarbonyl)phenyl]-3-(trifluoromethyl)-1,4,5,7-tetrahydropyrano[3,4-*c*]pyrazole**

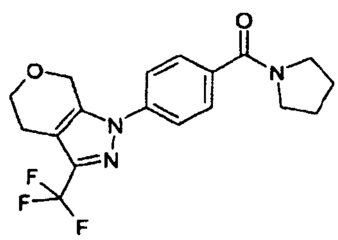

The title compound was prepared from 1-[(4-iodophenyl)carbonyl]pyrrolidine (D2) and 3-(trifluoromethyl)-1,4,5,7-tetrahydropyrano[3,4-*c*]pyrazole (D5) using a similar procedure to that described for Example 2.
$^1$H-NMR (400MHz, $CDCl_3$) δ: 7.66 (2H, m), 7.49 (2H, m), 4.84 (2H, s), 3.97 (2H, t, J=6Hz), 3.67 (2H, t, J=7Hz), 3.44 (2H, t, J=7Hz), 2.84 (2H, t, J=6Hz), 2.02-1.88 (4H, m); LC/MS Retention time 2.82mins/(ES+) 366 (M+H, $C_{18}H_{18}F_3N_3O_2$ requires 365).

**Example 4: 1-({4-[3-(trifluoromethyl)-4,7-dihydropyrano[3,4-*c*]pyrazol-1(5*H*)-yl]phenyl}methyl)-2-pyrrolidinone**

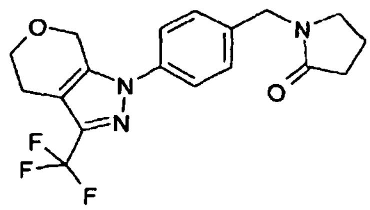

The title compound was prepared from 1-[(4-iodophenyl)methyl]-2-pyrrolidinone (D1) and 3-(trifluoromethyl)-1,4,5,7-tetrahydropyrano[3,4-*c*]pyrazole (D5) using a similar procedure to that described for Example 2.
$^1$H-NMR (400MHz, $CDCl_3$) δ: 7.38 (4H, m), 4.80 (2H, s), 4.50 (2H, s), 3.96 (2H, t, J=6Hz), 3.29 (2H, m), 2.83 (2H, m), 2.46 (2H, t, J=8Hz), 2.03 (2H, m); LC/MS Retention time 2.76mins/(ES+) 366 (M+H, $C_{18}H_{18}F_3N_3O_2$ requires 365).

**Example 5: 1-({4-[6-methyl-3-(trifluoromethyl)-4,5,6,-7-tetrahydro-1*H*-pyrazolo[3,4-*c*]pyridin-1-yl]phenyl}methyl)-2-pyrrolidinone**

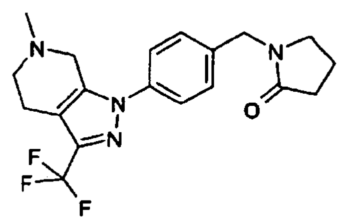

A mixture of 1-[(4-iodophenyl)methyl]-2-pyrrolidinone (D1, 90mg, 0.3mmol), 6-methyl-3-(trifluoromethyl)-4,5,6,7-tetrahydro-1*H*-pyrazolo[3,4-*c*]pyridine (D8, 62mg, 0.3mmol), copper (I) oxide (10mol%, 0.03mmol, 4mg), N,N-dimethylglycine (20mol%, 0.06mmol, 6mg) and cesium carbonate (0.6mmol, 196mg) in dimethylsulfoxide (1.5ml) was stirred at 190˚C in a microwave reactor for 30mins. The reaction mix was partitioned between water (5ml) and dichloromethane (5ml). The organic layer was added to a 5g isolute silica sep-pak column and eluted from 0-5% methanol in ethyl acetate to give a yellow oil which was purified by mass-directed auto-preparation to give an oil which was then partitioned between dichloromethane and saturated aqueous sodium bicarbonate solution. The organic layer was dried (sodium sulphate) and evaporated to give the title compound as a yellow solid (45mg, 40%).
$^1$H-NMR (400MHz, $CDCl_3$) δ: 7.41 (2H, m), 7.36 (2H, d, J=9Hz), 4.50 (2H, s), 3.58 (2H, s), 3.29 (2H, m), 2.81 (2H, m), 2.74 (2H, m), 2.50 (3H, s), 2.46 (2H, m), 2.03 (2H, m); LC/MS Retention time 1.72mins/(ES+) 379 (M+H, $C_{19}H_{21}F_3N_4O$ requires 378).

**Example 6: 1-({4-[3-(trifluoromethyl)-4,5,6,7-tetrahydro-1*H*-pyrazolo[3,4-*c*]pyridin-1-yl]phenyl}methyl)-2-pyrrolidinone**

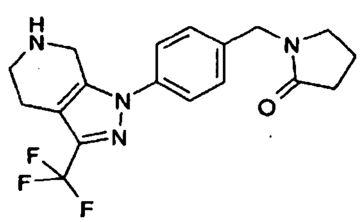

A mixture of 1-[(4-iodophenyl)methyl]-2-pyrrolidinone (D1, 188mg, 0.62mmol), 6-tert-butoxycarbonyl-3-(trifluoromethyl)-4,5,6,7-tetrahydro-1H-pyrazolo[3,4-c]pyridine (D7, 182mg, 0.62mmol), copper (I) iodide (10mol%, 0.06mmol, 12mg), N,N-dimethylglycine (20mol%, 0.013mmol, 14mg) and potassium carbonate (1.3mmol, 179mg) in dimethylsulfoxide (2.5ml) was stirred at 190˚C in a microwave reactor for 0.5h. The reaction mix was partitioned between water

(5ml) and dichloromethane (5ml). The organic layer was added to a 5g isolute silica sep-pak column and eluted from ethyl acetate and the solvent removed by rotary evaporation. The residue was dissolved in dichloromethane (3ml) and treated with a 1 M solution of HCl in diethyl ether (3ml) and allowed to stand for 4h. The reaction mix was evaporated to remove the solvent. The residue was added to a 5g SCX column and eluted from 0-10% 2M .880 ammonia in methanol / ethyl acetate to give the title compound as a brown oil (74mg, 33%)..
$^1$H-NMR (400MHz, $CDCl_3$) δ: 7.42 (2H, m), 7.34 (2H, m), 4.50 (2H, m), 4.02 (2H, s), 3.29 (2H, m), 3.12 (2H, t, J=6Hz), 2.74 (2H, m), 2.46 (2H, m), 2.03 (2H, m); LC/MS Retention time 1.66mins/(ES+) 365 (M+H, $C_{18}H_{19}F_3N_4O$ requires 364).

**Example 7: 1-({2,6-difluoro-4-[3-(trifluoromethyl)-6,7-dihydropyrano[4,3-*c*]pyrazol-1(4*H*)-yl]phenyl}methyl)-2-pyrrolidinone**

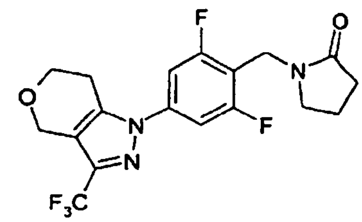

A mixture of 3-(trifluoromethyl)-1,4,6,7-tetrahydropyrano[4,3-*c*]pyrazole (D4, 0.993g, 5.17mmol), copper (I) oxide 0.739g, 5.17mmol), cesium carbonate (3.37g. 10.34mmol), 1-[(4-bromo-2,6-difluorophenyl)methyl]-2-pyrrolidinone (D10, 1.5g, 5.17mmol) and N,N-dimethylglycine (0.532g, 5.17mmol) in DMSO (15ml) was heated at 130˚C under argon for 4h. The reaction mixture was diluted with ethyl acetate. Catalyst was filtered off through kieselguhr. The reaction mixture was partitioned between ethyl acetate and water, separated organic layer, washed with brine, dried over sodium sulphate. The solvent was removed by rotary evaporation and the desired product was isolated by column chromatography on silica using 20 to 70% ethyl acetate in n-pentane. The residual material was recrystallised from ether to give the title compound as a white solid (0.62g, 30%).
$^1$H-NMR (400MHz, $CDCl_3$) δ: 7.18 (2H, m), 4.78 (2H, s), 4.60 (2H, s), 3.94 (2H, m), 3.30 (2H, m), 2.96 (2H, m), 2.40 (2H, m), 2.0 (2H, m); LC/MS Retention time 2.90mins/(ES+) 402 (M+H, $C_{18}H_{16}F_5N_3O_2$ requires 401).

**Example 8: 1-({2-fluoro-4-[3-(trifluoromethyl)-4,7-dihydropyrano[3,4-*c*]pyrazol-1(5*H*)-yl]phenyl}methyl)-2-pyrrolidinone**

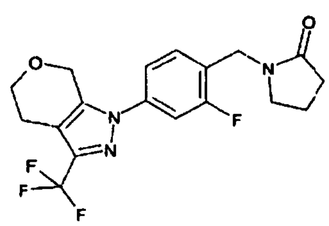

The title compound was prepared from 1-[(4-bromo-2-fluorophenyl)methyl]-2-pyrrolidinone (D11) and 3-(trifluoromethyl)-1,4,5,7-tetrahydropyrano[3,4-*c*]pyrazole (D5) using a similar procedure to that described for Example 2.
$^1$H-NMR (400MHz, $CDCl_3$) δ: 7.43 (1H, m), 7.27 (1H, m), 7.13 (1H, dd, J=8Hz and 2Hz), 4.83 (2H, s), 4.55 (2H, s), 3.96 (2H, t, J=6Hz), 3.35 (2H, t, J=7Hz), 2.82 (2H, m), 2.44 (2H, t, J=8Hz), 2.04 (2H, m); LC/MS Retention time 2.91mins/(ES+) 384 (M+H, $C_{18}H_{17}F_4N_3O_2$ requires 383).

**Example 9: 1-({2-fluoro-4-[6-methyl-3-(trifluoromethyl)-4,5,6,7-tetrahydro-1*H*-pyrazolo[3,4-*c*]pyridin-1-yl]phenyl}methyl)-2-pyrrolidinone hydrochloride**

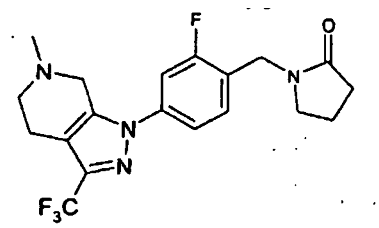

A mixture of 6-methyl-3-(trifluoromethyl)-4,5,6,7-tetrahydro-1*H*-pyrazolo[3,4-*c*]pyridine (D8, 0.3g, 1.46mmol), copper (I) oxide 0.209g, 1.46mmol), cesium carbonate (0.951g, 2.92mmol), 1-[(4-bromo-2-fluorophenyl)methyl]-2-pyrrolidinone (D11, 0.398g, 1.46mmol) and N,N-dimethylglycine (0.105g, 1.46mmol) in DMSO (2ml) was heated at 180˚C in a microwave for 0.5h. The reaction mixture was diluted with ethyl acetate and water. Catalyst was filtered off through kieselguhr. The reaction mixture was partitioned between ethyl acetate and water, separated organic layer, washed with brine, dried over sodium sulphate. The solvent was removed by rotary evaporation and the desired product was purified by SCX cartridge, washed with methanol and eluting with 1 M ammonia solution in MeOH. Solvent was removed and the oil was subjected to chromatography on silica gel column eluting with dichloromethane and further purified by MDAP (mass directed auto-prep). Treated with ethereal hydrochloride to give the title compound as a white solid (0.279g, 44%). $^1$H-NMR (400MHz, DMSO) δ: 11.52 (1H, br s), 7.56-7.46 (2H, m), 7.42 (1H, m), 4.76 (1H, m), 4.63 (1H, m), 4.50 (2H, s), 4.17 (1H, m), 3.65 (1H, m), 3.30 (2H, m), 3.0 (2H, m), 2.90 (3H, s), 2.30 (2H, m), 1.96 (2H, m); LC/MS Retention time 1.78mins/(ES+) 397 (M+H, $C_{19}H_{20}F_4N_4O$ requires 396).

**Example 10: 1-[3-fluoro-4-(1-pyrrolidinylcarbonyl)phenyl]-3-(trifluoromethyl)-1,4,5,7-tetrahydropyrano[3,4-*c*] pyrazole**

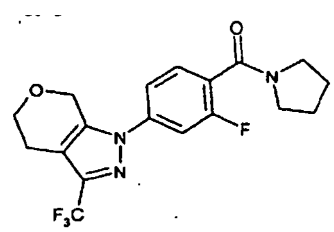

The title compound was prepared from 1-[(4-bromo-2-fluorophenyl)carbonyl]pyrrolidine (D12) and 3-(trifluoromethyl)-1,4,5,7-tetrahydropyrano[3,4-*c*]pyrazole (D5) using a similar procedure to that described for Example 2. $^1$H-NMR (400MHz, $CDCl_3$) δ: 7.55 (1H, m), 7.32 (1H, m), 7.22 (1H, dd, J=8Hz and 2Hz), 4.85 (2H, s), 3.96 (2H, t, J=6Hz), 3.67 (2H, t, J=7Hz), 3.33 (2H, t, J=7Hz), 2.83 (2H, m), 2.03-1.87 (4H, m); LC/MS Retention time mins 2.95mins/(ES+) 384 (M+H, $C_{18}H_{17}F_4N_3O_2$ requires 383).

**Example 11: 1-({4-[5-methyl-3-(trifluoromethyl)-4,5,6,7-tetrahydro-1*H*-pyrazolo[4,3-*c*]pyridin-1-yl]phenyl}methyl)-2-pyrrolidinone**

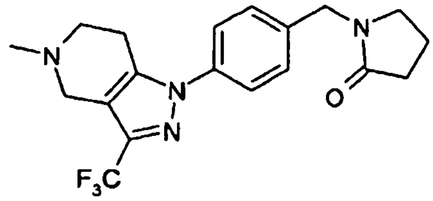

A mixture of 1-[(4-iodophenyl)methyl]-2-pyrrolidinone (D1, 150mg, 0.5mmol), 5-methyl-3-(trifluoromethyl)-4,5,6,7-tetrahydro-1*H*-pyrazolo[4,3-*c*]pyridine (D14, 107mg, 0.52mmol), copper (I) iodide (10mol%, 0.05mmol, 10mg), N,N-dimethylglycine (20mol%, 0.1mmol, 10mg) and potassium carbonate (1mmol, 138mg) in dimethylsulfoxide (2ml)

was stirred at 190˚C in a microwave reactor for 30mins. The reaction mix was partitioned between water (5ml) and dichloromethane (5ml). The organic layer was added to a 5g isolute silica sep-pak column and eluted from 0-100% ethyl acetate in petroleum ether to give a yellow oil which was purified by mass-directed auto-preparation. Once isolated the residue was partitioned between dichloromethane (3ml) and saturated aqueous sodium bicarbonate solution (3ml). The organic layer was dried over sodium sulphate and the solvent removed under reduced pressure to give the title compound as a yellow solid (21 mg, 11%).
$^1$H-NMR (400MHz, $CDCl_3$) δ: 7.47 (2H, m), 7.35 (2H, d, J=8Hz), 4.50 (2H, s), 3.58 (2H, s), 3.28 (2H, m), 2.88 (2H, m), 2.73 (2H, m), 2.54 (3H, s), 2.46 (2H, m), 2.02 (2H, m); LC/MS Retention time 1.58mins/(ES+) 379 (M+H, $C_{19}H_{21}F_3N_4O$ requires 378).

**Example 12: 1-{4-[(1,1-dioxido-2-isothiazolidinyl)methyl]phenyl}-3-(trifluoromethyl)-1,4,6,7-tetrahydropyrano [4,3-*c*]pyrazole**

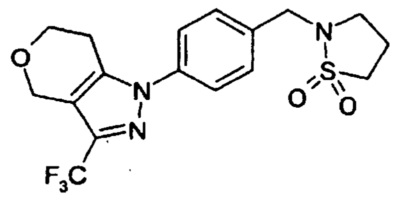

The title compound was prepared from 3-(trifluoromethyl)-1,4,6,7-tetrahydropyrano[4,3-*c*]pyrazole (D4) and 2-[(4-bromophenyl)methyl]isothiazolidine 1,1-dioxide (D15) using a similar procedure to that described for Example 2.
$^1$H-NMR (400MHz, $CDCl_3$) δ: 7.50 (4H, m), 4.81 (2H, s), 4.24 (2H, s), 3.93 (2H, m), 3.23 (2H, t, J=8Hz), 3.14 (2H, t, J=7Hz), 2.88 (2H, m), 2.35 (2H, m); LC/MS Retention time 2.78mins/(ES+) 402 (M+H, $C_{17}H_{18}F_3N_3O_3S$ requires 401).

**Example 13: 1-[4-(1-acetyl-2-pyrrolidinyl)phenyl]-3-(trifluoromethyl)-1,4,6,7-tetrahydropyrano[4,3-*c*]pyrazole**

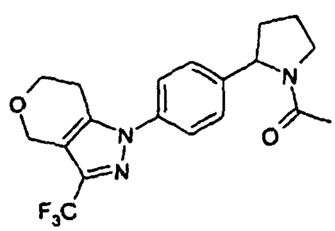

The title compound was prepared from 3-(trifluoromethyl)-1,4,6,7-tetrahydropyrano[4,3-*c*]pyrazole (D4) and 1-acetyl-2-(4-bromophenyl)pyrrolidine (D16) using a similar procedure to that described for Example 2.
$^1$H-NMR (400MHz, $CDCl_3$) δ: 7.52 (1H, m), 7.43 (1H, m), 7.28 (2H, m), 5.26 & 4.99 (1H, m, rotomers), 4.70 (2H, m), 3.93 (2H, m), 3.79-3.48 (2H, m), 2.86 (2H, m), 2.44 & 2.31 (1H, m, rotomers), 2.02-1.82 (6H, m); LC/MS Retention time 2.68 mins/(ES+) 380 (M+H, $C_{19}H_{20}F_3N_3O_2$ requires 379).

**Example 14: 1-[4-(1-azetidinylcarbonyl)phenyl]-3-(trifluoromethyl)-1,4,5,7-tetrahydropyrano[3,4-*c*]pyrazole**

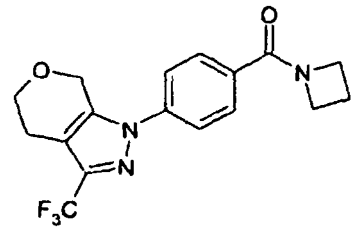

The title compound was prepared from 3-(trifluoromethyl)-1,4,5,7-tetrahydropyrano[3,4-c]pyrazole (D5) and 1-[(4-iodophenyl)carbonyl]azetidine (D17) using a similar procedure to that described for Example 2.
$^1$H-NMR (400MHz, $CDCl_3$) δ: 7.76 (2H, m), 7.48 (2H, m), 4.85 (2H, s), 4.33 (2H, m), 4.26 (2H, m), 3.97 (2H, m), 2.83 (2H, m), 2.38 (2H, m); LC/MS Retention time 2.69mins/(ES+) 352 (M+H, $C_{17}H_{16}F_3N_3O_2$ requires 351).

**Example 15: 1-({4-[3-(trifluoromethyl)-4,5,6,7-tetrahydro-1*H*-pyrazolo[4,3-*c*]pyridin-1-yl]phenyl}methyl)-2-pyrrolidinone**

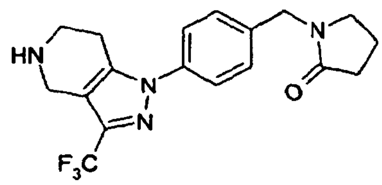

5-tert-butoxycarbonyl-3-(trifluoromethyl)-4,5,6,7-tetrahydro-1H-pyrazolo[4,3-c]pyridine (D19, 100mg, 0.34mmol), 1-[(4-iodophenyl)methyl]-2-pyrrolidinone (D1, 103mg, 0.34mmol), potassium carbonate (99mg, 0.72mmol), copper (I) iodide (10mol%, 6mg), N,N-dimethylglycine (20mol%, 7mg) and dimethylsulfoxide (4ml) were added sequentially to a microwave vial. Reaction mixture was heated in a microwave reactor for 1 hour at 190˚C. Reaction mixture was then separated between ethyl acetate and brine. The organic layer was retained and dried over sodium sulphate. The solvent was removed by rotary evaporation and the sample loaded onto a 2g SCX column using dichloromethane and methanol. Neutral phase was discarded and desired product was removed using 1M ammonia in methanol. The solvent was removed using rotary evaporation to give the title compound as a yellow oil (41 mg, 33%).
$^1$H-NMR (400MHz, $CDCl_3$) δ: 7.48 (2H, m), 7.36 (2H, m), 4.50 (2H, s), 4.01 (2H, s), 3.28 (2H, m), 3.12 (2H, m), 2.78 (2H, m), 2.47 (2H, m), 2.03 (2H, m); LC/MS Retention time 1.66mins/(ES+) 365 (M+H, $C_{18}H_{19}F_3N_4O$ requires 364).

**Example 16-17:**

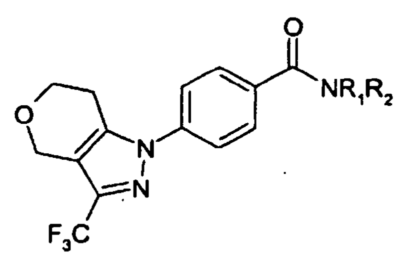

Typical Procedure: 1,1'-carbonyldiimidazole (55mg, 0.34mmol) and 4-[3-(trifluoromethyl)-6,7-dihydropyrano[4,3-*c*]pyrazol-1(4*H*)-yl]benzoic acid (D20, 70mg, 0.22mol) was added to a plastic sarstedt tube and dissolved in dichloromethane (2ml). The reaction was shaken for 20 minutes and then a solution of the appropriate secondary amine (0.05ml) (azetidine, (Ex 16) and N-methylethylamine (Ex 17)) in dichloromethane (1ml) was added in one portion. Reaction mixture was shaken for 3 hours. Reaction mixture was washed with brine. The organic layer was retained and dried with sodium sulphate. Products obtained were purified using mass directed auto-preparation to give the named compounds.

| Ex | NR1R2 | Name | LC/Mass Spec (ES) | 1H-NMR (400MHz, $CDCl_3$) |
|---|---|---|---|---|
| 16 | 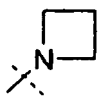 | 1-[4-(1-azetidinylcarbonyl) phe nyl]-3-(trifluoromethyl)-1,4,6,7-tetrahydropyrano [4,3-*c*]pyrazole | Retention time 2.72mins/ (ES+) 352 (M+H, $C_{17}H_{16}F_3N_3O_2$ requires 351). | δ: 7.77 (2H, m), 7.59 (2H, m), 4.81 (2H, s), 4.32 (2H, m), 4.26 (2H, m), 3.94 (2H, t, J=6Hz), 2.91 (2H, m), 2.38 (2H, m) |
| 17 | 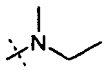 | *N*-ethyl-*N*-methyl-4-[3-(trifluoromethyl)-6,7-dihydropyrano[4,3-*c*] pyrazol-1(4*H*)-yl]benzamide | Retention time 2.80mins/ (ES+) 354 (M+H, $C_{17}H_{18}F_3N_3O_2$ requires 351). | δ: 7.56 (4H, m), 4.81 (2H, s), 3.94 (2H, t, J=6Hz), 3.62 & 3.30 (2H, m, rotomers), 3.09 & 2.96 (3H, m rotomers), 2.90 (2H, t, J=6Hz), 1.27 (3H, m). |

**Examples 18-19:**

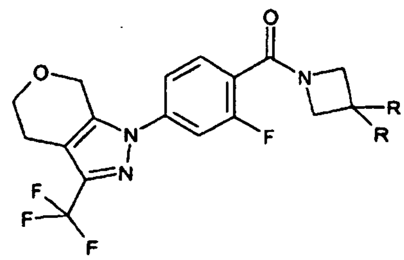

Typical Procedure: The named compounds were prepared from 3-(trifluoromethyl)-1,4,5,7-tetrahydropyrano [3,4-*c*]pyrazole (D5) and 1-[(4-bromo-2-fluorophenyl)carbonyl]azetidine (D32) (Example 18) and 1-[(4-bromo-2-fluorophenyl)carbonyl]-3,3-difluoroazetidine (D33) (Example 19) using a similar procedure to that described for Example 2.

| Ex | R | Name | LC/Mass Spec (ES) | 1H-NMR (400MHz, $CDCl_3$) |
|---|---|---|---|---|
| 18 | H | 1-[4-(1-azetidinylcarbonyl)-3-fluorophenyl]-3-(trifluoromethyl)-1,4,5,7-tetrahydropyrano[3,4-*c*] pyrazole | Retention time 2.82mins/(ES+) 370 (M+H, $C_{17}H_{15}F_4N_3O_2$ requires 369). | δ: 7.68 (1H, m), 7.32 (1H, m), 7.22 (1H, m), 4.86 (2H, s), 4.23 (2H, m), 4.13 (2H, t, J=8Hz), 3.96 (2H, t, J=6Hz), 2.83 (2H, m), 2.35 (2H, m). |
| 19 | F | 1-{4-[(3,3-difluoro-1-azetidinyl) carbonyl]-3-fluorophenyl}-3-(trifluoromethyl)-1,4,5,7-tetrahydropyrano[3,4-*c*] pyrazole | Retention time 3.07mins/(ES+) 406 (M+H, $C_{17}H_{13}F_5N_3O_2$ requires 405). | δ: 7.77 (1H, m), 7.39 (1H, m), 7.26 (1H, m), 4.88 (2H, s), 4.59-4.41 (4H, m), 3.96 (2H, m), 2.84 (2H, m) |

**Example 20: 1-{4-[(3,3-difluoro-1-azetidinyl)carbonyl]phenyl}-3-(trifluoromethyl)-1,4,6,7,tetrahydropyrano [4,3-*c*]pyrazole**

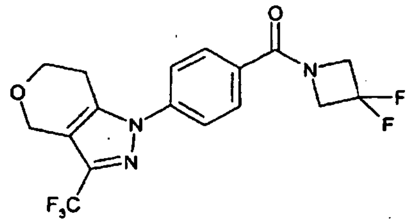

The title compound was prepared from 3-(trifluoromethyl)-1,4,6,7-tetrahydropyrano[4,3-*c*]pyrazole (D4) and 3,3-difluoro-1-[(4-iodophenyl)carbonyl]azetidine (D21) using a similar procedure to that described for Example 2.
$^1$H-NMR (400MHz, $CDCl_3$) δ: 7.79 (2H, m), 7.65 (2H, m), 4.81 (2H, m), 4.57 (4H, t, J=12Hz), 3.95 (2H, t, J=6Hz), 2.93 (2H, t, J=6Hz); LC/MS Retention time 2.97mins/(ES+) 388 (M+H, $C17H_{17}F_{14}N_3O_2$ requires 387).

**Example 21: 1-{4-[(3,3-difluoro-1-azetidinyl)carbonyl]phenyl}-3-(trifluoromethyl)-1,4,5,7-tetrahydropyrano [3,4-*c*]pyrazole**

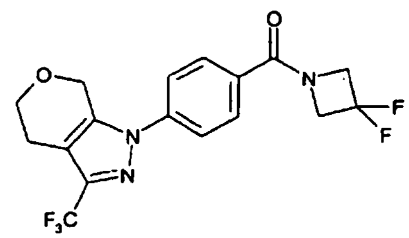

The title compound was prepared from 3-(trifluoromethyl)-1,4,5,7-tetrahydropyrano[3,4-*c*]pyrazole (D5) and 3,3-difluoro-1-[(4-iodophenyl)carbonyl]azetidine (D21) using a similar procedure to that described for Example 2.
$^1$H-NMR (400MHz, $CDCl_3$) δ: 7.78 (2H, m), 7.53 (2H, m), 4.87 (2H, s), 4.57 (4H, t, J=12Hz), 3.97 (2H, t, J=6Hz), 2.84 (2H, t, J=6Hz); LC/MS Retention time 2.99mins/(ES+) 388 (M+H, $C_{17}H_{14}F_5N_3O_2$ requires 387).

**Example 22: *N*-({4-[3-(trifluoromethyl)-6,7-dihydropyrano[4,3-*c*]pyrazol-1(4*H*)-yl]phenyl}methyl)methanesulfonamide**

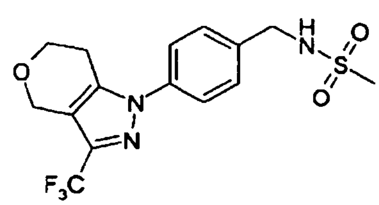

A mixture of *N*-[(4-bromophenyl)methyl]methanesulfonamide (D25, 132mg, 0.5mmol), 3-(trifluoromethyl)-1,4,6,7-tetrahydropyrano[4,3-*c*]pyrazole (D4, 96mg, 0.5mmol), copper (I) oxide (10mol%, 0.05mmol, 7mg), N,N-dimethylglycine (20mol%, 0.1mmol, 10mg) and cesium carbonate (1mmol, 326mg) in dimethylsulfoxide (2ml) was stirred at 190˚C in a microwave reactor for 0.5 hours. Then the reaction mix was cooled and partitioned between dichloromethane and water. The organic layer was added directly to a 5g isolute silica sep-pak column and eluted from ethyl acetate. The solvent was removed under reduced pressure and the residue further purified by mass directed auto-preparation to give the title compound as a pale yellow oil (10mg, 5%).
$^1$H-NMR (400MHz, $CDCl_3$) δ: 7.54 (2H, m), 7.49 (2H, d, J=8Hz), 4.81 (2H, s), 4.67 (1H, m), 4.40 (2H, d, J=6Hz), 3.93 (2H, t, J=6Hz), 2.94 (3H, s), 2.88 (2H, m); LC/MS Retention time 2.67mins/(ES+) 376 (M+H, $C_{15}H_{16}F_3N_3O_3S$ requires 375).

**Example 23: 1-{4-[(1,1-dioxido-2-isothiazolidinyl)methyl]phenyl}-3-(trifluoromethyl)-1,4,5,7-tetrahydropyrano [3,4-*c*]pyrazole**

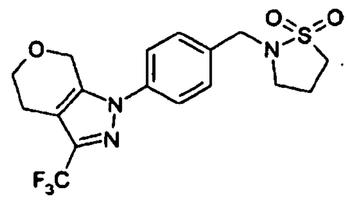

The title compound was prepared from 3-(trifluoromethyl)-1,4,5,7-tetrahydropyrano[3,4-*c*]pyrazole (D5) and 2-[(4-bromophenyl)methyl]isothiazolidine 1,1-dioxide (D15) using a similar procedure to that described for Example 22. $^1$H-NMR (400MHz, $CDCl_3$) δ: 7.49 (2H, d, J=8Hz), 7.42 (2H, d, J=8Hz), 4.81 (2H, s), 4.23 (2H, s), 3.96 (2H, t, J=6Hz), 3.23 (2H, m), 3.14 (2H, t, J=7Hz), 2.83 (2H, m), 2.35 (2H, m); LC/MS Retention time 2.92mins/(ES+) 402 (M+H, $C_{17}H_{18}F_3N_3O_3S$ requires 401).

**Example 24: *N*-({4-[3-(trifluoromethyl)-4,7-dihydropyrano[3,4-*c*]pyrazol-1(5*H*)-yl]phenyl}methyl)methanesulfonamide**

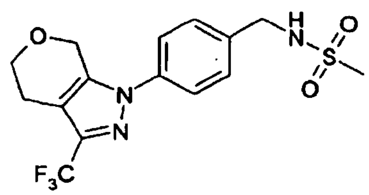

A mixture of *N*-[(4-iodophenyl)methyl]methanesulfonamide (D26, 622mg, 2.0mmol), 3-(trifluoromethyl)-1,4,5,7-tetrahydropyrano[3,4-*c*]pyrazole (D5, 384mg, 2.0mmol), copper (I) iodide (50mol%, 1.0mmol, 190mg), N,N-dimethylglycine (206mg, 2.0mmol,) and potassium carbonate (552mg, 4.0mmol,) in dimethylsulfoxide (6ml) was stirred at 190˚C in a microwave reactor for 0.5 hours, then cooled to room temperature. The reaction mix was partitioned between dichloromethane (8ml) and 0.5M hydrochloric acid (15ml). The organic layer was added directly to a 5g isolute prepacked silica sep-pak column and eluted from ethyl acetate. The solvent was removed by rotary evaporation and the residue further purified by mass directed auto-preparation to give the title compound as a colourless solid (429mg, 56%). $^1$H-NMR (400MHz, $CDCl_3$) δ: 7.49 (2H, m), 7.43 (2H, m), 4.83 (3H, m), 4.38 (2H, d, J=6Hz), 3.96 (2H, t, J=6Hz), 2.93 (3H, s), 2.83 (2H, m); LC/MS Retention time 2.75mins/(ES+) 376 (M+H, $C_{15}H_{16}F_3N_3O_3S$ requires 375).

**Example 25: 1-{4-[(1,1-dioxido-2-isothiazolidinyl)methyl]phenyl}-6-methyl-3-(trifluoromethyl)-4,5,6,7-tetrahydro-1*H*-pyrazolo[3,4-*c*]pyridine**

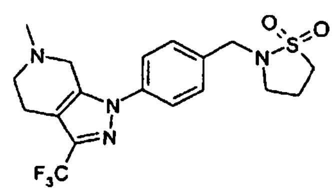

A mixture of 2-[(4-bromophenyl)methyl]isothiazolidine 1,1-dioxide (D15, 289mg, 1mmol), 6-methyl-3-(trifluoromethyl)-4,5,6,7-tetrahydro-1*H*-pyrazolo[3,4-*c*]pyridine (D8, 205mg, 1mmol), copper (I) iodide (20mol%, 0.2mmol,

38mg), N,N-dimethylglycine (40mol%, 0.4mmol, 41 mg) and cesium carbonate (2mmol, 652mg) in dimethylsulfoxide (3ml) was stirred at 190°C in a microwave reactor for 0.5 hours. Then the reaction mix was cooled and partitioned between dichloromethane and water. The organic layer was added directly to a 5g isolute silica sep-pak column and eluted from 5% methanol in ethyl acetate. The solvent was removed under reduced pressure and the residue further purified by mass directed auto-preparation. The residue was partitioned between dichloromethane and saturated sodium bicarbonate solution to give the title compound as a brown oil (24mg, 6%).

$^1$H-NMR (400MHz, $CDCl_3$) δ: 7.46 (4H, m), 4.23 (2H, s), 3.59 (2H, s), 3.23 (2H, t, J=8Hz), 3.14 (2H, t, J=7Hz), 2.81 (2H, m), 2.75 (2H, m), 2.49 (3H, s), 2.34 (2H, m); LC/MS Retention time 1.61mins/(ES+) 415 (M+H, $C_{18}H_{21}F_3N_4O_2S$ requires 414).

**Example 26: 1-[2-fluoro-4-(1-pyrrolidinylcarbonyl)phenyl]-3-(trifluoromethyl)-1,4,6,7-tetrahydropyrano[4,3-*c*] pyrazole**

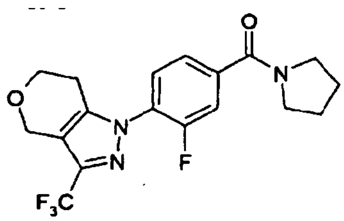

A mixture of 1-[(4-bromo-3-fluorophenyl)carbonyl]pyrrolidine (D22,190mg, 0.7mmol), 3-(trifluoromethyl)-1,4,6,7-tetrahydropyrano[4,3-*c*]pyrazole (D4, 134mg, 0.7mmol), copper (I) iodide (10mol%, 0.07mmol, 13mg), N,N-dimethylglycine (20mol%, 0.14mmol, 14mg) and potassium, carbonate (193mg, 1.4mmol) in dimethylsulfoxide (3ml) was stirred at 190°C in a microwave reactor for 0.5 hours. The reaction mix was treated with further copper (I) iodide (10mol%, 0.07mmol, 13mg) and N,N-dimethylglycine (20mol%, 0.14mmol, 14mg) and heating continued at 190°C in a microwave reactor for 0.5 hours. Then the reaction mix was partitioned between water (5ml) and dichloromethane (2 x 3ml). The organic fractions were added to a 5g isolute pre-packed silica column and eluted with ethyl acetate. The solvent was removed by rotary evaporation and the residue purified by mass directed auto-preparation to give the title compound as a brown oil (26mg, 10%).

$^1$H-NMR (400MHz, $CDCl_3$) δ: 7.57 (2H, m), 7.45 (1H. m), 4.81 (2H, s), 3.94 (2H, m), 3.66 (2H, t, J=6Hz), 3.45 (2H, m), 2.90 (1H, m), 2.71 (1H, m), 2.07-1.87 (4H, m); LC/MS Retention time 2.83mins/(ES+) 384 (M+H, $C_{18}H_{17}F_4N_3O_2$ requires 383).

**Example 27: 1-({3-fluoro-4-[3-(trifluoromethyl)-4,7-dihydropyrano[3,4-*c*]pyrazol-1(5*H*)-yl]phenyl}methyl)-2-pyrrolidinone**

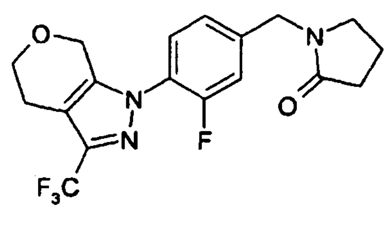

A mixture of 1-[(3-fluoro-4-iodophenyl)methyl]-2-pyrrolidinone (D24, 211mg, 0.66mmol), 3-(trifluoromethyl)-1,4,5,7-tetrahydropyrano[3,4-*c*]pyrazole (D5, 128mg, 0.66mmol), copper (I) iodide (10mol%, 0.07mmol, 13mg), N,N-dimethylglycine (20mol%, 0.13mmol, 14mg) and potassium carbonate (183mg, 1.32mmol) in dimethylsulfoxide (3ml) was stirred at 190°C in a microwave reactor for 0.5 hours. Then the reaction mix was cooled and partitioned between dichloromethane (5ml) and water (5ml). The organic layer was added directly to a 5g isolute pre-packed silica column and eluted with ethyl acetate. The solvent was removed by rotary evaporation and the residue further purified by mass

directed auto-preparation. Relevant fractions were combined and the solvent removed by rotary evaporation to give the title compound as a yellow oil (29mg, 11 %).
$^1$H-NMR (400MHz, $CDCl_3$) δ: 7.50 (1H, t, J=8Hz), 7.16 (2H, m), 4.63 (2H, m), 4.51 (2H, m), 3.96 (2H, t, J=6Hz), 3.32 (2H, m), 2.82 (2H, m), 2.48 (2H, m), 2.06 (2H, m); LC/MS Retention time 2.82mins/(ES+) 384 (M+H, $C_{18}H_{17}F_4N_3O_2$ requires 383).

**Example 28: 1-({3-fluoro-4-[3-(trifluoromethyl)-6,7-dihydropyrano[4,3-*c*]pyrazol-1(4*H*)-yl]phenyl}methyl)-2-pyrrolidinone**

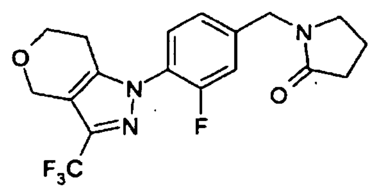

The title compound was prepared from 1-[(3-fluoro-4-iodophenyl)methyl]-2-pyrrolidinone (D24) and 3-(trifluoromethyl)-1,4,6,7-tetrahydropyrano[4,3-*c*]pyrazole (D4) using a similar procedure to that described for Example 27.
$^1$H-NMR (400MHz, $CDCl_3$) δ: 7.49 (1H, m), 7.16 (2H, m), 4.80 (2H, s), 4.52 (2H, m), 3.94 (2H, m), 3.32 (2H, m), 2.87 and 2.70 (2H, m), 2.48 (2H, m), 2.06 (2H, m); LC/MS . Retention time 2.75mins/(ES+) 384 (M+H, $C_{18}H_{17}F_4N_3O_2$ requires 383).

**Example 29: 1-{4-[(2-oxo-1-pyrrolidinyl)methyl]phenyl}-3-(trifluoromethyl)-1,4,5,7-tetrahydro-6H-pyrazolo[3,4-*c*]pyridine-6-carbaldehyde**

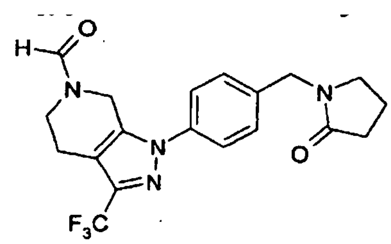

A solution of 1-({4-(3-(trifluoromethyl)-4,5,6,7-tetrahydro-1*H*-pyrazolo[3,4-*c*]pyridin-1-yl)phenyl}methyl)-2-pyrrolidinone (Example 6, 40mg, 0.11mmol) in dichloromethane (3ml) was treated with formic acid (0.1ml) in one portion and stirred for 18 hours. Reaction mix was then heated in a microwave for 1 hour at 100°C. Solvent was removed by rotary evaporation and the sample purified by mass directed auto-preparation to give the title compound as a pale yellow oil (13mg, 30%).
$^1$H-NMR (400MHz, $CDCl_3$) δ: 8.20 (1H, m), 7.42 (4H, m), 4.72 (2H, s), 4.51 (2H, m), 3.71 (2H, t, J=6Hz), 3.30 (2H, m), 2.88 (2H, t, J=6Hz), 2.47 (2H, m), 2.04 (2H, m); LC/MS Retention time 2.51mins/(ES+) 393 (M+H, $C_{19}H_{19}F_3N_4O_2$ requires 392).

**Example 30: *N*-(tetrahydro-2-furanylmethyl)-4-[3-(trifluoromethyl)-6,7-dihydropyrano[4,3-c]pyrazol-1(4*H*)-yl] benzenesulfonamide**

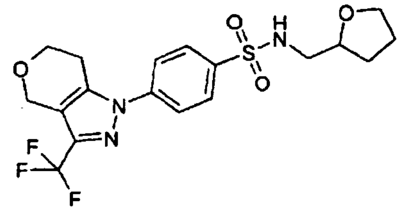

A mixture of copper (I) iodide (10mol%, 0.09mmol, 18mg), 3-(trifluoromethyl)-1,4,6,7-tetrahydropyrano[4,3-*c*] pyrazole .(D4, 100mg, 0.52mmol), potassium carbonate (144mg, 1.03mmol) and dimethylsulfoxide (3ml) was stirred for 1 minute, 4-iodo-*N*-(tetrahydro-2-furanylmethyl)benzenesulfonamide (D27, 191mg, 0.52mmol) and N,N-dimethylglycine (20mol%, 0.10mmol, 10mg) were then successively added. The reaction tube was quickly sealed and the contents were heated in a microwave reactor at 180˚C for 40 minutes. Then the reaction mixture was cooled and partitioned between ethyl acetate and water, organic layer separated, dried with sodium sulphate and evaporated. The residue was purified by mass directed auto-preparation (MDAP) to give the title compound as a pale yellow solid (59mg, 26%).
$^1$H-NMR (400MHz, $CDCl_3$) δ: 8.0 (2H, m), 7.72 (2H, m), 4.92 (1H, m), 4.81 (2H, s), 3.96 (3H, m), 3.79 (1H, m), 3.71 (1H, m), 3.18 (1H, m), 2.95 (3H, m), 1.90 (3H, m), 1.60 (1H,m); LC/MS Retention time 2.82mins/(ES+) 432 (M+H, $C_{18}H_{20}F_3N_3O_4S$ requires 431).

**Example 31: 1-({2-fluoro-4-[3-(trifluoromethyl)-6,7-dihydropyrano[4,3-*c*]pyrazol-1(4*H*)-yl]phenyl}methyl)-2-pyrrolidinone**

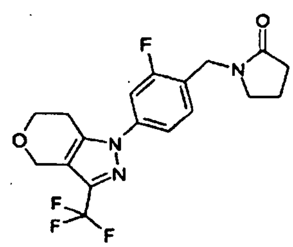

A mixture of copper (I) iodide (49.5mg, 0.26mmol), 3-(trifluoromethyl)-1,4,6,7-tetrahydropyrano[4,3-*c*]pyrazole (D4, 50mg, 0.26mmol), potassium carbonate (72mg, 0.52mmol) and dimethylsulfoxide (1.5ml) was stirred for 1 minute, 1-[(4-bromo-2-fluorophenyl)methyl]-2-pyrrolidinone (D11, 71mg, 0.26mmol) and N,N-dimethylglycine (20mol%, 0.05mmol, 5mg) were then successively added. The reaction tube was quickly sealed and the contents were heated in a microwave reactor at 180˚C for 40 minutes. Then the reaction mixture was cooled and partitioned between ethyl acetate and water, organic layer separated, dried with sodium sulphate and evaporated. The residue was purified by mass directed auto-preparation (MDAP) to give the title compound as a solid (30mg, 30%).
$^1$H-NMR (400MHz, $CDCl_3$) δ: 7.42 (1H, m), 7.31 (2H, m), 4.80 (2H, m), 4.56 (2H, s), 3.93 (2H, m), 3.35 (2H, m), 2.90 (2H, m), 2.44 (2H, m), 2.03 (2H, m); LC/MS Retention time 2.75mins/(ES+) 384 (M+H, $C_{18}H_{17}F_4N_3O_2$ requires 383).

**Example 32: 1-({2-fluoro-4-[3-(trifluoromethyl)-6,7-dihydropyrano[4,3-*c*]pyrazol-1(4*H*)-yl]phenyl}carbonyl)-3-azetidinol**

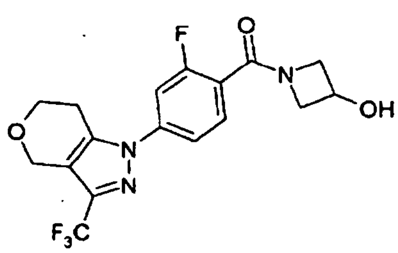

A mixture of 2-fluoro-4-[3-(trifluoromethyl)-6;7-dihydropyrano[4,3-*c*]pyrazol-1(4*H*)-yl]benzoic acid (D28, 85mg, 0.26mmol), 3-azetidinol hydrochloride (31 mg, 0.28mmol) and N,N-diisopropylethylamine (0.09ml, 0.52mmol) in dimethylformamide (5ml) was stirred at room temperature under argon and then HATU (97mg, 0.26mmol) was added. The resulting mixture was allowed to stir at room temperature overnight. The solvent was removed under reduced pressure and the residue was partitioned between ethyl acetate and water, organic layer was dried over sodium sulphate, filtered and evaporated in vacuo. The desired product was isolated by mass directed auto-preparation (MDAP) to give the title compound as a white solid (38mg, 38%).
$^1$H-NMR (400MHz, $CDCl_3$) δ: 7.69 (1H, m), 7.40 (2H, m), 4.80 (2H, s), 4.75 (1H, m), 4.48 (1H, m), 4.29 (1H, m), 4.06 (2H, m), 4.95 (2H, m), 2.93 (2H, m), 2.27 (1H, m); LC/MS Retention time 2.34mins/(ES+) 386 (M+H, $C_{17}H_{15}F_4N_3O_3$ requires 385).

**Example 33: 1-({2-fluoro-4-[3-(trifluoromethyl)-4,5,6,7-tetrahydro-1*H*-pyrazolo[3,4-*c*]pyridin-1-yl]phenyl}methyl)-2-pyrrolidinone hydrochloride**

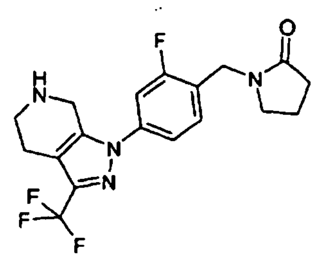

A mixture of copper (I) iodide (105mg, 0.55mmol), 6-tert-butoxycarbonyl-3-(trifluoromethyl)-4,5,6,7-tetrahydro-1H-pyrazolo[3,4-*c*]pyridine (D7, 160mg, 0.55mmol), potassium carbonate (152mg, 1.10mmol) and dimethylsulfoxide (2ml) was stirred for 1 minute, 1-[(4-bromo-2-fluorophenyl)methyl]-2-pyrrolidinone (D11, 160mg, 0.551mmol) and N,N-dimethylglycine (57mg, 0.55mmol) were then successively added. The reaction tube was quickly sealed and the contents were heated in a microwave reactor at 180°C for 40 minutes. Then the reaction mixture was cooled and partitioned between ethyl acetate and water, organic layer separated, dried with sodium sulphate and evaporated. The residue purified by mass directed auto-preparation (MDAP) and SCX cartridge, washed with methanol (20ml) and eluting with 1 M ammonia methanol solution. Solvent was removed, dried under high vacuum and treated with ethereal hydrochloric acid to give the title compound as a solid (15mg, 6.5%).
$^1$H-NMR (400MHz, Free Base, $CDCl_3$) δ: 7.42 (1H, m), 7.28 (1H, m), 7.18 (1H, m), 4.55 (2H, s), 4.08 (2H, s), 3.35 (2H, m), 3.15 (2H, m), 2.76 (2H, m), 2.43 (2H, m), 2.03 (2H, m), 1.85 (1H, br s); Retention time 1.67mins/(ES+) 383 (M+H, $C_{18}H_{18}F_4N_4O$ requires 382).

**Example 34: 1-{4-[(3,3-difluoro-1-azetidinyl)carbonyl]-3-fluorophenyl}-3-(trifluoromethyl)-1,4,6,7-tetrahydropyrano[4,3-*c*]pyrazole**

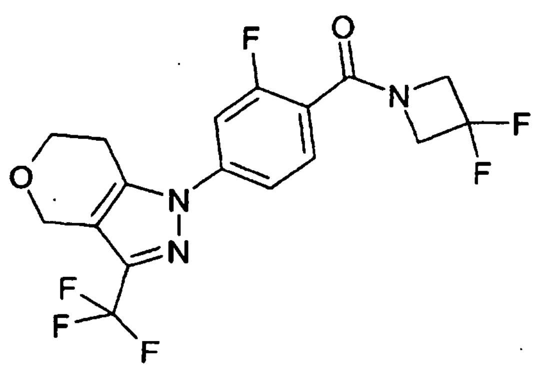

A mixture of 2-fluoro-4-[3-(trifluoromethyl)-6,7-dihydropyrano[4,3-*c*]pyrazol-1(4*H*)-yl]benzoic acid (D28, 150mg, 0.45mmol), 3,3-difluoroazetidine hydrochloride (65mg, 0.49mmol), N,N-diisopropylethylamine (0.24ml, 1.35mmol) in dimethylforamide (5ml) was stirred at room temperature under argon and then HATU (172mg, 0.45mmol) was added. The resulting mixture was allowed to stir at room temperature overnight. The solvent was removed under reduced pressure and the residue was partitioned between ethyl acetate and water, organic layer dried over sodium sulphate, filtered and evaporated under reduced pressure. The desired product was isolated by mass directed auto-preparation (MDAP) to give the title compound as a white solid (53mg, 28%).
$^1$H-NMR (400MHz, $CDCl_{3)}$ δ: 7.80 (1H, m), 7.69 (1H, m), 7.62 (1H, m), 4.73 (2H, s), 4.56 (4H, m), 3.87 (2H, m), 3.01 (2H, m); LC/MS Retention time 3.05mins/(ES+) 406 (M+H, $C_{17}H_{13}N_3F_6O_2$ requires 405).

**Example 35: 1-{3-fluoro-4-[2-oxo-2-(1-pyrrolidinyl)ethyl]phenyl}-3-(trifluoromethyl)-1,4,6,7-tetrahydropyrano [4,3-*c*]pyrazole**

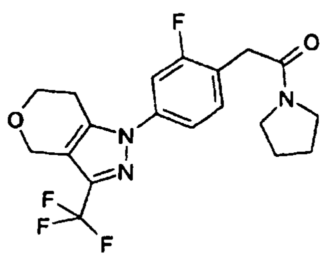

A mixture of copper (I) iodide (49.5mg, 0.26mmol), 3-(trifluoromethyl)-1,4,6,7-tetrahydropyrano[4,3-*c*]pyrazole (D4, 50mg, 0.26mmol), potassium carbonate (72mg, 0.52mmol) and dimethylsulfoxide (1.5ml) was stirred for 1 minute, 1-[(4-bromo-2-fluorophenyl)acetyl]pyrrolidine (D31, 74.5mg, 0.26mmol) and N,N-dimethylglycine (20mol%, 0.05mmol, 5mg) were then successively added. The reaction tube was quickly sealed and the contents were heated in a microwave reactor at 180°C for 40 minutes. Then the reaction mixture was cooled and partitioned between ethyl acetate and water, organic layer separated, dried with sodium sulphate and evaporated. The residue purified by mass directed auto-preparation (MDAP) to give the title compound as a brown gum (23mg, 22%).
$^1$H-NMR (400MHz, $CDCl_3$) δ: 7.46 (1H, m), 7.28 (2H, m), 4.79 (2H, s), 3.93 (2H, m), 3.69 (2H, s), 3.51 (4H, m), 2.90 (2H, m), 2.0 (2H, m), 1.88 (2H, m); LC/MS Retention time 2.83mins/(ES+) 398 (M+H, $C_{19}H_{19}F_4N_3O_2$ requires 397).

**Example 36: 1-[3-fluoro-4-(1-pyrrolidinylcarbonyl)phenyl]-3-(trifluoromethyl)-1,4,6,7-tetrahydropyrano[4,3-*c*] pyrazole**

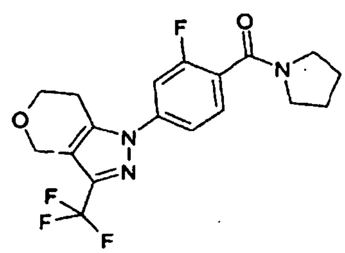

The title compound was prepared from -(trifluoromethyl)-1,4,6,tetrahydropyrano[4,3-*c*]pyrazole (D4) and 1-[(4-bromo-2-fluorophenyl)carbonyl]pyrrolidine (D12) using a similar procedure to that described for Example 31.
$^1$H-NMR (400MHz, $CDCl_3$) δ: 7.57 (1H, m), 7.38 (2H, m), 4.80 (2H, s), 3.94 (2H, m), 3.67 (2H, s), 3.33 (2H, m), 2.91 (2H, m), 2.03-1.88 (4H, m); LC/MS Retention time 2.77mins/(ES+) 384 (M+H, $C_{18}H_{17}F_4N_3O_2$ requires 383).

**Example 37: 1-({4-[6-acetyl-3-(trifluoromethyl)-4,5,6,7-tetrahydro-1*H*-pyrazolo[3,4-*c*]pyridin-1-yl]-2-fluorophenyl}methyl)-2-pyrrolidinone**

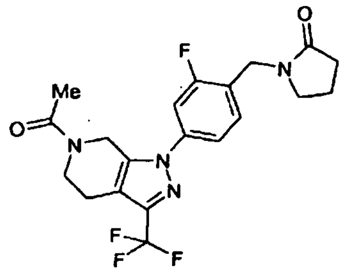

A mixture of 1-({2-fluoro-4-[3-(trifluoromethyl)-4,5,6,7-tetrahydro-1*H*-pyrazolo[3,4-*c*]pyridin-1-yl]phenyl}methyl)-2-pyrrolidinone (Example 33, 150mg, 0.36mmol) and triethylamine (0.2ml) in dichloromethane (10ml) was stirred in an ice bath. Acetyl chloride (0.05ml, 0.72mmol) was added. The resulting mixture was allowed to stir at room temperature under argon for 5 hours. Then the solution was washed with water, organic layer separated, dried over sodium sulphate and evaporated. The desired product was isolated by mass directed auto-preparation (MDAP) and triturated with n-pentane to give the title compound as a white solid (108mg, 71%).
$^1$H-NMR (400MHz, $CDCl_3$) δ: 7.46 (1H, m), 7.28-7.21 (2H, m), 4.81 (2H, s), 4.56 (2H, s), 3.75 (2H, m), 3.38 (2H, m), 2.85 (2H, m), 2.45 (2H, m), 2.20 (3H, s), 2.07 (2H, m);. LC/MS Retention time 2.59mins/(ES+) 425 (M+H, $C_{20}H_{20}F_4N_4O_2$ requires 424).

**Example 38: 1-(3-fluoro-4-[2-oxo-2-(1-pyrrolidinyl)ethyl]phenyl)-3-(trifluoromethyl)-1,4,5,7-tetrahydropyrano [3,4-*c*]pyrazole**

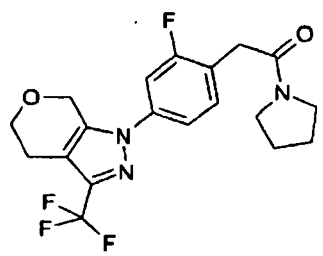

A mixture of copper (I) iodide (41 mg, 30mol%, 0.21 mmol), 3-(trifluoromethyl)-1,4,5,7-tetrahydropyrano[3,4-*c*] pyrazole (D5, 134mg, 0.7mmol), cesium carbonate (456mg, 1.4mmol) and dimethylsulfoxide (3ml) was stirred for 3 minutes, 1-[(4-bromo-2-fluorophenyl)acetyl]pyrrolidine (D31, 200mg, 0.7mmol) and N,N-dimethylglycine (72mg, 0.7mmol) were then successively added. The reaction tube was quickly sealed and the contents were heated in a microwave reactor at 180˚C for 20 minutes. Then the reaction mixture was diluted with ethyl acetate and filtered off the catalyst through kieselguhr. The organic solution was washed with water, organic layer separated, dried with sodium sulphate and evaporated. The residue purified by mass directed auto-preparation (MDAP) to give the title compound as a creamy yellow coloured solid (95mg, 34%).
$^1$H-NMR (400MHz, $CDCl_3$) δ: 7.46 (1H, m), 7.25 (1H, m), 7.12 (1H, m), 4.82 (2H, s), 3.95 (2H, m), 3.68 (2H, s), 3.51 (4H, m), 2.81 (2H, m), 1.99 (2H, m), 1.88 (2H, m); LC/MS Retention time 2.95mins/(ES+) 398 (M+H, $C_{19}H_{19}F_4N_3O_2$ requires 397).

**Example 39: 1-({2,6-difluoro-4-[3-(trifluoromethyl)-4,7-dihydropyrano[3,4-*c*]pyrazol-1(5*H*)-yl]phenyl}methyl)-2-pyrrolidinone**

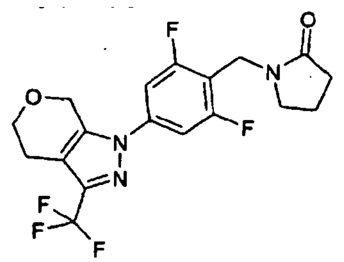

The title compound was prepared from 3-(trifluoromethyl)-1,4,5,7-tetrahydropyrano[3,4-*c*]pyrazole (D5) and 1-[(4-bromo-2,6-difluorophenyl)methyl]-2-pyrrolidinone (D10) using a similar procedure to that described for Example 38. $^1$H-NMR (400MHz, $CDCl_3$) δ: 7.06 (2H, m), 4.83 (2H, s), 4.57 (2H, s), 3.96 (2H, m), 3.30 (2H, m), 2.82 (2H, m), 2.40 (2H, m), 2.01 (2H, m); LC/MS Retention time 2.98mins/(ES+) 402 (M+H, $C_{18}H_{16}F_5N_3O_2$ requires 401).

**Example 40: 1-[4-(1-pyrrolidinylcarbonyl)phenyl]-3-(trifluoromethyl)-4,5,6,7-tetrahydro-1*H*-pyrazolo[3,4-*c*]pyridine hydrochloride**

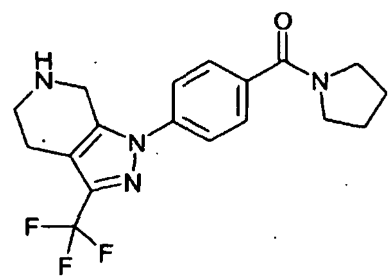

4-lodobenzoic acid (701mg, 2.83mmol) and $K_2CO_3$ (810mg, 5.86mmol) were succesively added each in one portion to a solution of 6-tert-butoxycarbonyl-3-(trifluoromethyl)-4,5,6,7-tetrahydro-1H-pyrazolo[3,4-*c*]pyridine (D7, 657mg, 2.77mmol) copper (I) iodide (44.0mg, 0.23mmol), and *N,N*-dimethylglycine (52mg, 0.50mmol) in anhydrous DMSO (5ml). This was diluted with more anhydrous DMSO (6ml) and stirred at 130°C. for 4 hours. Upon cooling to room temperature, the mixture was partitioned between DCM (150ml), and water (130ml), and acidified (pH 3) with aqueous HCl (2N, 5.5ml). The separated aqueous phase was further acidified (pH 0) with more aqueous HCl (2N, 5ml) and extracted twice with DCM (125ml then 50ml).

The combined organic phase was concentrated *in vacuo* using high vacuum, and partitioned between DCM (20ml) and water (10ml) to remove the residual DMSO. The organic phase was concentrated *in vacuo* giving a brown solid (1.09g). An aliquot (100mg) was dissolved in anhydrous DCM, pyrrolidine (100μl, 1.21mmol) and HATU (2-(1H-7-Azabenzotriazol-1-yl)-1,1,3,3-tetramethyl uronium hexafluorophosphate methanaminium) (118mg, 0.31mmol) were added, and the mixture stirred at room temperature for 5 hours. The resulting solution was concentrated *in vacuo,* dissolved in anhydrous DCM (5ml), and TFA (200μl, 2.70mmol) was added in one portion. After stirring at room temperature for 2 hours 10 minutes, more TFA (500μl, 6.75mmol) was added in one portion and the mixture stirred at room temperature for a further 17 hours. The mixture was then concentrated *in vacuo,* purified by successive SCX column chromatography and high pH MDAP, dissolved in DCM, and ethereal HCl (1M) added. Further concentration *in vacuo* gave the title compound (22.9mg, 62.8μmol).
LC/MS Retention time 1.74mins/(ES+) 365 (M+H, $C_{18}H_{19}F_3N_4O$ requires 364).

**Example 41: 1-{4-[(3,3-difluoro-1-azetidinyl)carbonyl]phenyl}-3-(trifluoromethyl)-4,5,6,7-tetrahydro-1*H*-pyrazolo[3,4-*c*]pyridine**

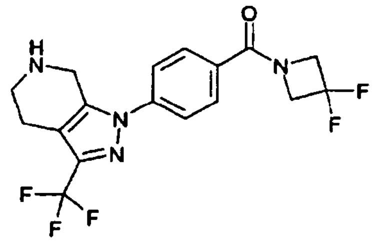

The title compound was made in a similar manner to 1-[4-(1-pyrrolidinylcarbonyl)phenyl]-3-(trifluoromethyl)-4,5,6,7-tetrahydro-1*H*-pyrazolo[3,4-*c*]pyridine hydrochloride (Example 40) but using 3,3-difluoroazetidine hydrochloride instead of pyrrolidine. Ethereal HCl was not added at the end of the synthesis.
LC/MS Retention time 1.78mins/(ES+) 387 (M+H, $C_{17}H_{15}F_5N_4O$ requires 386).

**Example 42: 1-[4-(1-azetidinylcarbonyl)phenyl]-6-methyl-3-(trifluoromethyl)-4,5,6,7-tetrahydro-1*H*-pyrazolo [3,4-*c*]pyridine**

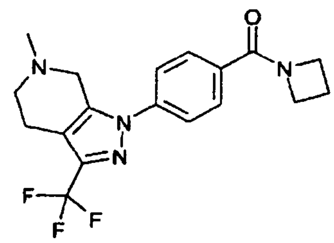

Copper (I) oxide (79.9mg, 0.56mmol) was added in one portion to a stirring mixture of *N,N*-dimethylglycine (64.2mg, 0.62mmol), 6-methyl-3-(trifluoromethyl)-4,5,6,7-tetrahydro-1*H*-pyrazolo[3,4-*c*]pyridine (D8, 100mg, 0.45mmol), 1-[(4-iodophenyl)carbonyl]azetidine (D17, 142mg, 0.49mmol), and $Cs_2CO_3$ (311mg, 0.95mmol) in anhydrous DMSO (1.6ml). The mixture was stirred at 190°C under microwave heating for 30 minutes, filtered through silica, and purified by SCX chromatography. Further purification by high pH MDAP gave the title compound as a sand coloured solid (35.7mg; 0.10mmol).
$^1$H-NMR (400MHz, $CDCl_3$) δ: 7.78-7.73 (2H, m), 7.55-7.49 (2H, m), 4.37-4.21 (4H, m), 3.62 (2H, s), 2.85-2.79 (2H, m), 2.78-2.72 (2H, m), 2.51 (3H, s), 2.43-2.33 (2H, m); LC/MS Retention time 1.65mins/(ES+) 365 (M+H, $C_{18}H_{19}F_3N_4O$ requires 364).

**Example 43: 1-({4-[6-acetyl-3-(trifluoromethyl)-4,5,6,7-tetrahydro-1*H*-pyrazolo[3,4-*c*]pyridin-1-yl]phenyl}methyl)-2-pyrrolidinone**

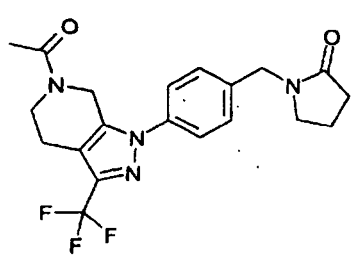

Acetyl chloride (20μl, 0.28mmol) was added in one portion to a stirring solution of 1-({4-[3-(trifluoromethyl)-4,5,6,7-tetrahydro-1*H*-pyrazolo[3,4-*c*]pyridin-1-yl]phenyl}methyl)-2-pyrrolidinone (Example 6, 57mg, 0.16mmol) and Hunig's base (50μl, 0.29mmol) in anhydrous DCM (2ml). After stirring at room temperature for 24 hours, the solvent was removed *in vacuo,* and the product purified by MDAP, giving the title compound as a colourless oil (43.6mg; 0.11 mmol).
LC/MS Retention time 2.62mins/(ES+) 407 (M+H, $C_{20}H_{21}F_3N_4O_2$ requires 406).

Biological Assay

The ability of the compounds of the invention to potentiate glutamate receptor-mediated response were determined a) by using fluorescent calcium-indicator dyes such as FLUO4; and additionally for some example compounds b) by measuring glutamate-evoked current recorded from human GluR2 flip unedited HEK293 cells.

a) Calcium Influx Fluorescence Assay

384 well plates were prepared containing confluent monolayer of HEK 293 cells either stably expressing or transiently transfected with human GluR2 flip (unedited) AMPA receptor subunit. These cells form functional homotetrameric AMPA receptors. The tissue culture medium in the wells was discarded and the wells were each washed three times with standard buffer (80 μL) for the stable cell line (145 mM NaCl, 5 mM KCl, 1 mM $MgCl_2$, 2 mM $CaCl_2$, 20 mM N-[2-hydroxyethyl]-piperazine-N-[2-ethanesulfonic acid (HEPES), 5.5 mM glucose, pH 7.3) or with a Na-free buffer for the transient transfected cells (145 mM N-methyl-glucamine instead of NaCl). The plates were then incubated for 60 minutes in the dark with 2 μM FLUO4-AM dye (Molecular Probes, Netherlands) at room temperature to allow cell uptake of the FLUO-4AM, which is then converted to FLUO-4 by intracellular esterases which is unable to leave the cell. After incubation each well was washed three times with buffer (80 μL) (30 μL of buffer remained in each well after washing).

Compounds of the invention (or reference compounds such as cyclothiazide) were dissolved in dimethylsulfoxide (DMSO) at a stock concentration of 10 mM. These solutions were further diluted with DMSO using a Biomek FX (Beckman Coulter) in a 384 compound plate. Each dilution (1 μL) was transferred to another compound plate and buffer (50 μL) was added. An agonist stimulus (glutamate) plate was prepared by dissolving sodium glutamate in water to give a concentration of 100 mM. This solution was diluted with buffer to give a final concentration of 500 μM and dispensed into another 384-well plate (50μL/well) using a Multidrop (Thermolabsystems).

The cell plate was then transferred into a fluorescence imaging plate based reader [such as the FLIPR384 (Molecular Devices)]. A baseline fluorescence reading was taken over a 10 to 240 second period, and then 10 μL from each plate containing a compound of the invention made up in standard buffer solution (in a concentration range from 100 μM to 10 pM) was added (to give a final concentration in the range 30 μM to 3 pM). The fluorescence was read over 5 minute period. 500 μM glutamate solution (10μL) was added (to give a final concentration of 100 μM). The fluoresecence was then read over a 4 minute period. The activities of the compounds of the invention and reference compounds were determined by measuring peak fluorescence after the last addition. The activity was also expressed relative to the fluorescence increase induced by cyclothiazide at their maximum response (i.e. greater than 30 μM).

All the Example compounds were screened using the assay as described above and the average of the measurable pEC50s were taken. All compounds gave an average $pEC_{50}$ equal to or greater than 3.5 and demonstrated an activity of, on average, at least 20% that of cyclothiazide (at its maximal response).

b) Whole cell voltage-clamp electrophysiology Assay

The ability of the compounds of the invention to potentiate AMPA-subtype glutamate receptor-mediated response are determined by measuring AMPA-evoked current recorded from rat cultured hippocampal neurons.

This assay involves the electrophysiological characterisation of AMPA receptor positive modulators using rat cultured hippocampal neurons. The extracellular recording solution contains: 145 mM NaCl, 2.5 mM KCl, 1.2 mM $MgCl_2$, 1.5 mM $CaCl_2$, 10 mM N-[2-hydroxyethyl]-piperazine-N-[2-ethanesulfonic acid (HEPES), 10 mM D-glucose, pH 7.3 with NaOH. The intracellular solution contains : 80 mM CsCl, 80 mM CsF, 10 mM N-[2-hydroxyethyl]-piperazine-N-[2-ethanesulfonic acid (HEPES), 10 mM ethylene glycol-bis(g-aminoethylether)-N,N,N',N,-tetra-acetic acid (EGTA), 14 mM MgATP, 14 mM DiTris Creatine Phosphate, 50 U/ml Creatine Phosphokinase pH 7.3 with CsOH. Recording electrodes are prepared from glass capillary tubes (Clark Electromedical GC120-F10) pulled into two equal lengths using a Zeitz Instruments DMZ Universal Puller, program 09, resulting in electrodes with a resistance of approximately 3-6 MOhms when measured in extracellular solution. Electrodes are back filled with internal recording solution. Positive pressure is applied to the electrode to prevent mixture of internal and external solutions and assist in formation of high resistance seal when the electrode makes contact with the cell membrane. Glass coverslip fragment, bearing rat cultured hippocampal neurons, is placed in the recording chamber positioned on the stage of an inverted microscope. A tube at the edge of the chamber is used to apply extracellular solution to the bath. Rapid solution exchange uses a fast step perfusion system (Biologic RSC160). Two outlet tubes attached together along their length are positioned close to a chosen cell so that the outflow from only one tube can pass directly over the cell surface. A motorized stepper could reposition the tubes such that the outflow from the second outlet tube flows over the cell allowing solution exchange at the cell membrane surface to occur within 10-20 ms. Excess bath solution is removed via a tube positioned at the edge of the chamber connected to a vacuum line.

A prospective cell is positioned in the centre of the microscope field of view. Recording electrode is positioned

directly above the cell membrane surface. Using fine manipulator control (Luigs and Neumann, SM-6) the electrode is lowered, while monitoring the change in electrode resistance during delivery of a 5 mV depolarizing pulse, until a high resistance seal (gigaseal) is achieved. Whole cell configuration is achieved by removing by suction a small fragment of cell membrane immediately beneath the recording electrode tip. The cell membrane potential is held at -70 mV (voltage-clamped) via the electrode (Axopatch 200B Integrating patch clamp amplifier, pClamp software, Axon Instruments). Test solutions are applied using the fast application system using the following protocol and changes in inward current are recorded and stored for off-line analysis.

1) Control current - exchange from extracellular solution to extracellular solution + 30 μM AMPA (2 s application time, 30 s interval between applications) repeated until measurements are stable.
2) Test current - exchange from extracellular solution + 10 nM of compound of invention to extracellular solution + 10 nM of compound of invention + 30 μM AMPA (2 s application time, 30 s interval between applications) repeated until measurements are stable.

All experiments are performed at ambient temperature (20 to 22°C).
The activity of a compound of the invention is determined by measuring the area under the curve (during 2 s period of application) for the 30 μM AMPA response in the presence of the compound of the invention and expressing it as % of potentiation of the 30 μM AMPA alone response (30 μM AMPA in the absence of the compound of the invention).
Some of the example compounds above were tested in this assay. The range of mean responses at 10nM increased 30 μM AMPA response by 8 to 50% and at 10μM by 42 to 679%.

## Claims

**1.** A compound of formula (I), or a salt, or solvate thereof:

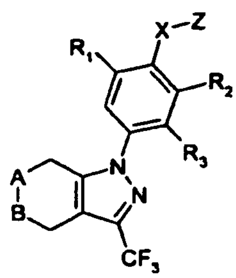

(I)

wherein

- one of A and B is $CH_2$ and the other is oxygen or $NR_4$, wherein $R_4$ is selected from hydrogen, $C_{1-2}$alkyl and $C_{1-2}$alkanoyl;
- $R_1$, $R_2$ and $R_3$ are independently selected from hydrogen and fluoro;
- X is selected from a bond, $CH_2$, C(O), $SO_2$ and $CH_2C(O)$ (wherein the carbonyl group is attached to Z);
- Z is selected from:

  (a) a C-linked pyrrolidinyl optionally substituted by $C_{1-2}$alkanoyl; and
  (b) a group $NR_5R_6$, wherein:

    - $R_5$ is hydrogen or $C_{1-4}$alkyl and $R_6$ is $C_{1-4}$alkyl, $C_{1-4}$alkylsulfonyl or Het-$C_{1-4}$alkyl wherein Het is a saturated 5 or 6 membered heterocyclic ring; or
    - $R_5$ and $R_6$ form an azetidinyl or a pyrrolidinyl group, wherein one of the carbon atoms in the azetidinyl or a pyrrolidinyl group is optionally replaced by $SO_2$, and the azetidinyl or pyrrolidinyl group is also optionally substituted by one or two groups selected from oxo, hydroxyl and halogen.

**2.** A compound as claimed in claim 1, wherein A is $CH_2$ and B is oxygen or $NR_4$, wherein $R_4$ is selected from hydrogen, $C_{1-2}$alkyl and $C_{1-2}$alkanoyl.

3. A compound as claimed in claim 1, wherein B is $CH_2$ and A is oxygen or $NR_4$, wherein $R_4$ is selected from hydrogen, $C_{1-2}$alkyl and $C_{1-2}$alkanoyl.

4. A compound as claimed in any of claims 1-3 wherein: $R_1$, $R_2$ and $R_3$ are all hydrogen; or $R_1$ is F and $R_2$ and $R_3$ are both hydrogen; or $R_1$ and $R_2$ are both hydrogen and $R_3$ is F.

5. A compound as claimed in any of claims 1-4 wherein Z is a C-linked pyrrolidinyl optionally substituted by $C_{1-2}$alkanoyl.

6. A compound as claimed in any of claims 1-4 wherein Z is a group $NR_5R_6$, wherein $R_5$ is hydrogen or $C_{1-4}$alkyl and $R_6$ is $C_{1-4}$alkyl, $C_{1-4}$alkylsulfonyl or Het-$C_{1-4}$alkyl wherein Het is a saturated 5 or 6 membered heterocyclic ring.

7. A compound as claimed in any of claims 1-4 wherein Z is a group $NR_5R_6$, wherein $R_5$ and $R_6$ form an azetidinyl or a pyrrolidinyl group, wherein one of the carbon atoms in the azetidinyl or a pyrrolidinyl group is optionally replaced by $SO_2$, and the azetidinyl or pyrrolidinyl group is also optionally substituted by one or two groups selected from oxo, hydroxyl and halogen.

8. A compound as claimed in claim 1 which is:

1-[4-(1-pyrrolidinylcarbonyl)phenyl]-3-(trifluoromethyl)-1,4,6,7-tetrahydropyrano[4,3-*c*]pyrazole
1-({4-[3-(trifluoromethyl)-6,7-dihydropyrano[4,3-*c*]pyrazol-1(4*H*)-yl]phenyl}methyl)-2-pyrrolidinone
1-[4-(1-pyrrolidinylcarbonyl)phenyl]-3-(trifluoromethyl)-1,4,5,7-tetrahydropyrano[3,4-*c*]pyrazole
1-({4-[3-(trifluoromethyl)-4,7-dihydropyrano[3,4-*c*]pyrazol-1(5*H*)-yl]phenyl}methyl)-2-pyrrolidinone
1-({4-[6-methyl-3-(trifluoromethyl)-4,5,6,7-tetrahydro-1*H*-pyrazolo[3,4-*c*]pyridin-1-yl]phenyl}methyl)-2-pyrrolidinone
1-({4-[3-(trifluoromethyl)-4,5,6,7-tetrahydro-1*H*-pyrazolo[3,4-*c*]pyridin-1-yl]phenyl}methyl)-2-pyrrolidinone
1-({2,6-difluoro-4-[3-(trifluoromethyl)-6,7-dihydropyrano[4,3-*c*]pyrazol-1(4*H*)-yl]phenyl}methyl)-2-pyrrolidinone
1-({2-fluoro-4-[3-(trifluoromethyl)-4,7-dihydropyrano[3,4-*c*]pyrazol-1(5*H*)-yl]phenyl}methyl)-2-pyrrolidinone
1-({2-fluoro-4-[6-methyl-3-(trifluoromethyl)-4,5,6,7-tetrahydro-1*H*-pyrazolo[3,4-*c*]pyridin-1-yl]phenyl}methyl)-2-pyrrolidinone
1-[3-fluoro-4-(1-pyrrolidinylcarbonyl)phenyl]-3-(trifluoromethyl)-1,4,5,7-tetrahydropyrano[3,4-*c*]pyrazole
1-({4-[5-methyl-3-(trifluoromethyl)-4,5,6,7-tetrahydro-1H-pyrazolo[4,3-c]pyridin-1-yl]phenyl}methyl)-2-pyrrolidinone
1-{4-[(1,1-dioxido-2-isothiazolidinyl)methyl]phenyl}-3-(trifluoromethyl)-1,4,6,7-tetrahydropyrano[4,3-c]pyrazole
1-[4-(1-acetyl-2-pyrrolidinyl)phenyl]-3-(trifluoromethyl)-1,4,6,7-tetrahydropyrano[4,3-*c*]pyrazole
1-[4-(1-azetidinylcarbonyl)phenyl]-3-(trifluoromethyl)-1,4,5,7-tetrahydropyrano[3,4-c]pyrazole
1-({4-[3-(trifluoromethyl)-4,5,6,7-tetrahydro-1H-pyrazolo[4,3-c]pyridin-1-yl]phenyl}methyl)-2-pyrrolidinone
1-[4-(1-azetidinylcarbonyl)phenyl]-3-(trifluoromethyl)-1,4,6,7-tetrahydropyrano[4,3-c]pyrazole
N-ethyl-N-methyl-4-[3-(trifluoromethyl)-6,7-dihydropyrano[4,3-c]pyrazol-1(4H)-yl]benzamide
1-[4-(1-azetidinylcarbonyl)-3-fluorophenyl]-3-(trifluoromethyl)-1,4,5,7-tetrahydropyrano[3,4-c]pyrazole
1-{4-[(3,3-difluoro-1-azetidinyl)carbonyl]-3-fluorophenyl}-3-(trifluoromethyl)-1,4,5,7-tetrahydropyrano[3,4-c]pyrazole
1-{4-[(3,3-difluoro-1-azetidinyl)carbonyl]phenyl}-3-(trifluoromethyl)-1,4,6,7-tetrahydropyrano[4,3-c]pyrazole 1-{4-[(3,3-difluoro-1-azetidinyl)carbonyl]phenyl}-3-(trifluoromethyl)-1,4,5,7-tetrahydropyrano[3,4-c]pyrazole
N-({4-[3-(trifluoromethyl)-6,7-dihydropyrano[4,3-c]pyrazol-1(4H)-yl]phenyl}methyl)methanesulfonamide
1-{4-[(1,1-dioxido-2-sothiazolidinyl)methyl]phenyl}-3-(trifluoromethyl)-1,4,5,7-tetrahydropyrano[3,4-c]pyrazole
N-({4-[3-(trifluoromethyl)-4,7-dihydropyrano[3,4-c]pyrazol-1(5H)-yl]phenyl}methyl)methanesulfonamide
1-{4-[(1,1-dioxido-2-isothiazolidinyl)methyl]phenyl}-6-methyl-3-(trifluoromethyl)-4,5,6,7-tetrahydro-1H-pyrazolo[3,4-c]pyridine
1-[2-fluoro-4-(1-pyrrolidinylcarbonyl)phenyl]-3-(trifluoromethyl)-1,4,6,7-tetrahydropyrano[4,3-c]pyrazole
1-({3-fluoro-4-[3-(trifluoromethyl)-4,7-dihydropyrano[3,4-c]pyrazol-1(5H)-yl]phenyl}methyl)-2-pyrrolidinone
1-({3-fluoro-4-[3-(trifluoromethyl)-6,7-dihydropyrano[4,3-c]pyrazol-1(4H)-yl]phenyl}methyl)-2-pyrrolidinone
1-{4-[(2-oxo-1-pyrrolidinyl)methyl]phenyl}-3-(trifluoromethyl)-1,4,5,7-tetrahydro-6H-pyrazolo[3,4-c]pyridine-6-carbaldehyde
N-(tetrahydro-2-furanylmethyl)-4-[3-(trifluoromethyl)-6,7-dihydropyrano[4,3-c]pyrazol-1 (4H)-yl]benzenesulfonamide
1-({2-fluoro-4-[3-(trifluoromethyl)-6,7-dihydropyrano[4,3-c]pyrazol-1(4H)-yl]phenyl}methyl)-2-pyrrolidinone
1-({2-fluoro-4-[3-(trifluoromethyl)-6,7-dihydropyrano[4,3-c]pyrazol-1(4H)-yl]phenyl}carbonyl)-3-azetidinol
1-({2-fluoro-4-[3-(trifluoromethyl)-4,5,6,7-tetrahydro-1H-pyrazolo[3,4-c]pyridin-1-yl]phenyl}methyl)-2-pyrrolidinone hdrochloride
1-{4-[(3,3-difluoro-1-azetidinyl)carbonyl]-3-fluorophenyl}-3-(trifluoromethyl)-1,4,6,7-tetrahydropyrano[4,3-c]pyrazole
1-{3-fluoro-4-[2-oxo-2-(1-pyrrolidinyl)ethyl]phenyl}-3-(trifluoromethyl)-1,4,6,7-tetrahydropyrano[4,3-c]pyrazole
1-[3-fluoro-4-(1-pyrrolidinylcarbonyl)phenyl]-3-(trifluoromethyl)-1,4,6,7-tetrahydropyrano[4,3-c]pyrazole
1-({4-[6-acetyl-3-(trifluoromethyl)-4,5,6,7-tetrahydro-1H-pyrazolo[3,4-c]pyridin-1-yl]-2-fluorophenyl}methyl)-2-pyrrolidinone
1-{3-fluoro-4-[2-oxo-2-(1-pyrrolidinyl)ethyl]phenyl}-3-(trifluoromethyl)-1,4,5,7-tetrahydropyrano[3,4-c]pyrazole
1-({2,6-difluoro-4-[3-(trifluoromethyl)-4,7-dihydropyrano[3,4-c]pyrazol-1(5H)-yl]phenyl}methyl)-2-pyrrolidinone
1-[4-(1-pyrrolidinylcarbonyl)phenyl]-3-(trifluoromethyl)-4,5,6,7-tetrahydro-1H-pyrazolo[3,4-c]pyridine hydrochloride
1-{4-[(3,3-difluoro-1-azetidinyl)carbonyl]phenyl}-3-(trifluoromethyl)-4,5,6,7-tetrahydro-1H-pyrazolo[3,4-c]pyridine
1-[4-(1-azetidinylcarbonyl)phenyl]-6-methyl-3-(trifluoromethyl)-4,5,6,7-tetrahydro-1H-pyrazolo[3,4-c]pyridine
1-({4-[6-acetyl-3-(trifluoromethyl)-4,5,6,7-tetrahydro-1H-pyrazolo[3,4-c]pyridin-1-yl]phenyl}methyl)-2-pyrrolidinone

or a salt or solvate thereof.

**9.** A compound as claimed in any of claims 1-8 for use as a medicament.

**10.** A compound as claimed in any of claims 1-8 for use in the treatment of schizophrenia.

**11.** A pharmaceutical composition comprising a compound as defined in any of claims 1-8 and at least one pharmaceutically acceptable carrier or diluent.

**12.** A combination product comprising a compound as defined in any of claims 1-8 and an antipsychotic.

**13.** Use of a compound as defined in any of claims 1-8, or a composition as defined in claim 11, or a product as defined in claim 12, in the manufacture of a medicament for treating or preventing schizophrenia.

**Patentansprüche**

**1.** Eine Verbindung der Formel (I) oder ein Salz oder Solvat davon:

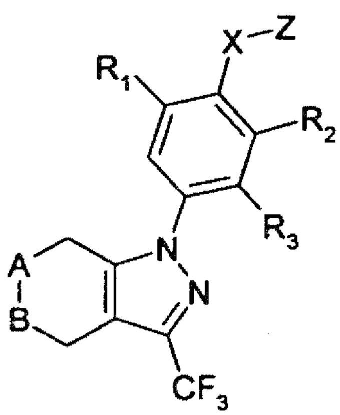

(I)

wobei:

• eines von A und B gleich $CH_2$ ist und das andere Sauerstoff oder $NR_4$ ist, wobei $R_4$ ausgewählt ist aus Wasserstoff, $C_{1-2}$-Alkyl und $C_{1-2}$-Alkanoyl,

- $R_1$, $R_2$ und $R_3$ unabhängig ausgewählt sind aus Wasserstoff und Fluor;
- X ausgewählt ist aus einer Bindung, $CH_2$, C(O), $SO_2$ und $CH_2C(O)$ (wobei der Carbonylrest an Z gebunden ist);
- Z ausgewählt ist aus:

(a) einem C-verknüpften Pyrrolidinyl, gegebenenfalls substituiert mit $C_{1-2}$-Alkanoyl; und
(b) einem Rest $NR_5R_6$, wobei:

- $R_5$ gleich Wasserstoff oder $C_{1-4}$-Alkyl ist und $R_6$ gleich $C_{1-4}$-Alkyl, $C_{1-4}$-Alkylsulfonyl oder Het-$C_{1-4}$-alkyl ist, wobei Het ein gesättigter 5- oder 6-gliedriger heterocyclischer Ring ist; oder
- $R_5$ und $R_6$ einen Azetidinyl- oder einen Pyrrolidinylrest bilden, wobei eines der Kohlenstoffatome im Azetidinyl- oder einem Pyrrolidinylrest gegebenenfalls durch $SO_2$ ersetzt ist, und der Azetidinyl- oder Pyrrolidinylrest ebenfalls gegebenenfalls mit einem oder zwei Resten, ausgewählt aus Oxo, Hydroxyl und Halogen, substituiert ist.

**2.** Eine Verbindung wie in Anspruch 1 beansprucht, wobei A gleich $CH_2$ ist und B gleich Sauerstoff oder $NR_4$ ist, wobei $R_4$ ausgewählt ist aus Wasserstoff, $C_{1-2}$-Alkyl und $C_{1-2}$-Alkanoyl.

**3.** Eine Verbindung wie in Anspruch 1 beansprucht, wobei B gleich $CH_2$ ist und A gleich Sauerstoff oder $NR_4$ ist, wobei $R_4$ ausgewählt ist aus Wasserstoff, $C_{1-2}$-Alkyl und $C_{1-2}$-Alkanoyl.

**4.** Eine Verbindung wie in einem der Ansprüche 1 bis 3 beansprucht, wobei: $R_1$, $R_2$ und $R_3$ alle Wasserstoff sind oder $R_1$ gleich F ist und $R_2$ und $R_3$ jeweils Wasserstoff sind oder $R_1$ und $R_2$ jeweils Wasserstoff sind und $R_3$ gleich F ist.

**5.** Eine Verbindung wie in einem der Ansprüche 1 bis 4 beansprucht, wobei Z ein C-verknüpftes Pyrrolidinyl, gegebenenfalls substituiert mit $C_{1-2}$-Alkanoyl, ist.

**6.** Eine Verbindung wie in einem der Ansprüche 1 bis 4 beansprucht, wobei Z ein Rest $NR_5R_6$ ist, wobei $R_5$ gleich Wasserstoff oder $C_{1-4}$-Alkyl ist und $R_6$ gleich $C_{1-4}$-Alkyl, $C_{1-4}$-Alkylsulfonyl oder Het-$C_{1-4}$-alkyl ist, wobei Het ein gesättigter 5- oder 6-gliedriger heterocyclischer Ring ist.

**7.** Eine Verbindung wie in einem der Ansprüche 1 bis 4 beansprucht, wobei Z ein Rest $NR_5R_6$ ist, wobei $R_5$ und $R_6$ einen Azetidinyl- oder einen Pyrrolidinylrest bilden, wobei eines der Kohlenstoffatome im Azetidinyl- oder einem Pyrrolidinylrest gegebenenfalls durch $SO_2$ ersetzt ist, und der Azetidinyl- oder Pyrrolidinylrest ebenfalls gegebenenfalls mit einem oder zwei Resten, ausgewählt aus Oxo, Hydroxyl und Halogen, substituiert ist.

**8.** Eine Verbindung wie in Anspruch 1 beansprucht., nämlich:

1-[4-(1-Pyrrolidinylcarbonyl)phenyl]-3-(trifluormethyl)-1,4,6,7-tetrahydropyrano[4,3-c]pyrazol
1-({4-[3-(Trifluormethyl)-6,7-dihydropyrano[4,3-c]pyrazol-1-(4H)-yl]phenyl}methyl)-2-pyrrolidinon
1-[4-(1-Pyrrolidinylcarbonyl)phenyl]-3-(trifluormethyl)-1,4,5,7-tetrahydropyrano[3,4-c]pyrazol
1-({4-[3-(Trifluormethyl)-4,7-dihydropyrano[3,4-c]pyrazol-1-(5H)-yl]phenyl}methyl)-2-pyrrolidinon
1-({4-[6-Methyl-3-(trifluormethyl)-4,5,6,7-tetrahydro-1H-pyrazolo[3,4-c]pyridin-1-yl]phenyl}methyl)-2-pyrrolidinon
1-({4-[3-(Trifluormethyl)-4,5,6,7-tetrahydro-1H-pyrazolo-[3,4-c]pyridin-1-yl]phenyl}-methyl)-2-pyrrolidinon
1-({2,6-Difluor-4-[3-(trifluormethyl)-6,7-dihydropyrano[4,3-c]pyrazol-1-(4H)-yl]-phenyl}methyl)-2-pyrrolidinon
1-({2-Fluor-4-[3-(trifluormethyl)-4,7-dihydropyrano[3,4-c]pyrazol-1-(5H)-yl]phenyl}-methyl)-2-pyrrolidinon
1-({2-Fluor-4-[6-methyl-3-(trifluormethyl)-4,5,6,7-tetrahydro-1H-pyrazolo[3,4-c]-pyridin-1-yl]phenyl}methyl)-2-pyrrolidinon
1-[3-Fluor-4-(1-pyrrolidinylcarbonyl)phenyl]-3-(trifluormethyl)-1,4,5,7-tetrahydro-pyrano[3,4-c]pyrazol
1-({4-[5-Methyl-3-(trifluormethyl)-4,5,6,7-tetrahydro-1H-pyrazolo[4,3-c]pyridin-1-yl]phenyl}methyl)-2-pyrrolidinon
1-{4-[(1,1-Dioxido-2-isothiazolidinyl)methyl]phenyl}-3-(trifluormethyl)-1,4,6,7-tetrahydropyrano[4,3-c]pyrazol
1-[4-(1-Acetyl-2-pyrrolidinyl)phenyl]-3-(trifluormethyl)-1,4,6,7-tetrahydropyrano[4,3-c]pyrazol
1-[4-(1-Azetidinylcarbonyl)phenyl]-3-(trifluormethyl)-1,4,5,7-tetrahydropyrano[3,4-c]pyrazol
1-({4-[3-(Trifluormethyl)-4,5,6,7-tetrahydro-1H-pyrazolo[4,3-c]pyridin-1-yl]phenyl}-methyl)-2-pyrrolidinon
1-[4-(1-Azetidinylcarbonyl)phenyl]-3-(trifluormethyl)-1,4,6,7-tetrahydropyrano[4,3-c]pyrazol
N-Ethyl-N-methyl-4-[3-(trifluormethyl)-6,7-dihydropyrano[4,3-c]pyrazol-1-(4H)-yl]benzamid
1-[4-(1-Azetidinylcarbonyl)-3-fluorphenyl]-3-(trifluormethyl)-1,4,5,7-tetrahydropyrano-[3,4-c]pyrazol

1-{4-[(3,3-Difluor-1-azetidinyl)carbonyl]-3-fluorphenyl}-3-(trifluormethyl)-1,4,5,7-tetra-hydropyrano [3,4-c]pyrazol
1-{4-[(3,3-Difluor-1-azetidinyl)carbonyl]phenyl}-3-(trifluormethyl)-1,4,6,7-tetrahydro-pyrano[4,3-c]pyrazol
1- {4-[(3,3-Difluor-1-azetidinyl)carbonyl]phenyl}-3-(trifluormethyl)-1,4,5,7-tetrahydro-pyrano[3,4-c]pyrazol
N-({4-[3-(Trifluormethyl)-6,7-dihydropyrano[4,3-c]pyrazol-1-(4H)-yl]phenyl}methyl)-methansulfonamid
1-{4-[(1,1-Dioxido-2-isothiazolidinyl)methyl]phenyl}-3-(trifluormethyl)-1,4,5,7-tetra-hydropyrano [3,4-c] pyrazol
N-({4-[3-(Trifluormethyl)-4,7-dihydropyrano[3,4-c]pyrazol-1-(5H)-yl]phenyl}methyl)-methansulfonamid
1-{4-[(1,1-Dioxido-2-isothiazolidinyl)methyl]phenyl}-6-methyl-3-(trifluormethyl)-4,5,6,7-tetrahydro-1H-pyrazolo [3,4-c]pyridin
1-[2-Fluor-4-(1-pyrrolidinylcarbonyl)phenyl]-3-(trifluormethyl)-1,4,6,7-tetrahydro-pyrano[4,3-c]pyrazol
1-({3-Fluor-4-[3-(trifluormethyl)-4,7-dihydropyrano[3,4-c]pyrazol-1-(5H)-yl]phenyl}-methyl)-2-pyrrolidinon
1-({3-Fluor-4-[3-(trifluormethyl)-6,7-dihydropyrano[4,3-c]pyrazol-1-(4H)-yl]phenyl}-methyl)-2-pyrrolidinon
1-{4-[(2-Oxo-1-pyrrolidinyl)methyl]phenyl}-3-(trifluormethyl)-1,4,5,7-tetrahydro-6H-pyrazolo[3,4-c]pyridin-6-carbaldehyd
N-(Tetrahydro-2-furanylmethyl)-4-[3-(trifluormethyl)-6,7-dihydropyrano[4,3-c]pyrazol-1-(4H)-yl]benzolsulfonamid
1-({2-Fluor-4-[3-(trifluormethyl)-6,7-dihydropyrano[4,3-c]pyrazol-1-(4H)-yl]phenyl}-methyl)-2-pyrrolidinon
1-({2-Fluor-4-[3-(trifluormethyl)-6,7-dihydropyrano[4,3-c]pyrazol-1-(4H)-yl]phenyl}carbonyl)-3-azetidinol
1-({2-Fluor-4-[3-(trifluormethyl)-4,5,6,7-tetrahydro-1H-pyrazolo[3,4-c]pyridin-1-yl]phenyl}methyl)-2-pyrrolidinonhydrochlorid
1- {4-[(3,3-Difluor-1-azetidinyl)carbonyl]-3-fluorphenyl}-3-(trifluormethyl)-1,4;6,7-tetrahydropyrano[4,3-c]pyrazol
1-{3-Fluor-4-[2-oxo-2-(1-pyrrolidinyl)ethyl]phenyl}-3-(tritluormethyl)-1,4,6,7-tetra-hydropyrano[4,3-c]pyrazol
1-[3-Fluor-4-(1-pyrrolidinylcarbonyl)phenyl]-3-(trifluormethyl)-1,4,6,7-tetrahydro-pyrano[4,3-c]pyrazol
1-({4-[6-Acetyl-3-(trifluormethyl)-4,5,6,7-tetrahydro-1H-pyrazolo[3,4-c]pyridin-1-yl]-2-fluorphenyl}methyl)-2-pyrrolidinon
1-{3-Fluor-4-[2-oxo-2-(1-pyrrolidinyl)ethyl]phenyl}-3-(trifluormethyl)-1,4,5,7-tetra-hydropyrano [3,4-c]pyrazol
1-({2,6-Difluor-4-[3-(trifluomnethyl)-4,7-dihydropyrano[3,4-c]pyrazol-1-(5H)-yl]-phenyl}methyl)-2-pyrrolidinon
1-[4-(1-Pyrrolidinylcarbonyl)phenyl]-3-(trifluormethyl)-4,5,6,7-tetrahydro-1H-pyrazolo-[3,4-c]pyridinhydrochlorid
1-{4-[(3,3-Difluor-1-azetidinyl)carbonyl]phenyl} -3-(trifluormethyl)-4,5,6,7-tetrahydro-1H-pyrazolo [3,4-c]pyridin
1-[4-(1-Azetidinylcarbonyl)phenyl]-6-methyl-3-(trifluormethyl)-4,5,6,7-tetrahydro-1H-pyrazolo-[3,4-c]pyridin
1-({4-[6-Acetyl-3-(trifluonnethyl)-4,5,6,7-tetrallydro-1H-pyrazolo[3,4-c]pyridin-1-yl]phenyl}methyl)-2-pyrrolidinon

oder ein Salz oder Solvat davon.

**9.** Eine Verbindung wie in einem der Ansprüche 1 bis 8 beansprucht zur Verwendung als ein Medikament.

**10.** Eine Verbindung wie in einem der Ansprüche 1 bis 8 beansprucht zur Verwendung in der Behandlung von Schizophrenie.

**11.** Eine pharmazeutische Zusammensetzung, umfassend eine Verbindung wie in einem der Ansprüche 1 bis 8 definiert und mindestens ein(en) pharmazeutisch verträgliches(n) Träger oder Verdünnungsmittel.

**12.** Ein Kombinationsprodukt, umfassend eine Verbindung wie in einem der Ansprüche 1 bis 8 definiert und ein Antipsychotikum.

**13.** Verwendung einer Verbindung wie in einem der Ansprüche 1 bis 8 definiert oder eine Zusammensetzung wie in Anspruch 11 definiert oder ein Produkt wie in Anspruch 12 definiert zur Herstellung eines Medikaments zur Behandlung oder Prävention von Schizophrenie.

## Revendications

**1.** Composé de formule (I), ou un de ses sels ou un de ses produits de solvatation :

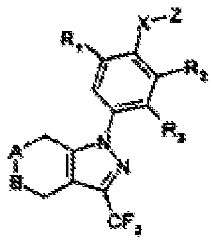

(I)

formule dans laquelle

- l'un de A et B représente un groupe $CH_2$ et l'autre représente un atome d'oxygène ou un groupe $NR_4$, dans lequel $R_4$ est choisi entre un atome d'hydrogène, des groupes alkyle en $C_1$ ou $C_2$ et alcanoyle en $C_1$ ou $C_2$ ;
- $R_1$, $R_2$ et $R_3$ sont choisis indépendamment entre un atome d'hydrogène et un groupe fluoro ;
- X est choisi entre une liaison, des groupes $CH_2$, C(O), $SO_2$ et $CH_2C(O)$ (où le groupe carbonyle est fixé à Z) ;
- Z est choisi entre :

  (a) un groupe pyrrolidinyle lié par C, facultativement substitué avec un substituant alcanoyle en $C_1$ ou $C_2$ ; et
  (b) un groupe $NR_5R_6$, dans lequel :

    - $R_5$ représente un atome d'hydrogène ou un groupe alkyle en $C_1$ à $C_4$ et $R_6$ représente un groupe alkyle en $C_1$ à $C_4$, alkylsulfonyle en $C_1$ à $C_4$ ou Het-(alkyle en $C_1$ à $C_4$) dans lequel Het représente un noyau hétérocyclique penta-ou hexagonal saturé ; ou
    - $R_5$ et $R_6$ forment un groupe azétidinyle ou pyrrolidinyle, dans lequel un des atomes de carbone dans le groupe azétidinyle ou pyrrolidinyle est facultativement remplacé par un groupe $SO_2$, et le groupe azétidinyle ou pyrrolidinyle est également facultativement substitué avec un ou deux groupes choisis entre des groupes oxo, hydroxyle et halogéno.

**2.** Composé suivant la revendication 1, dans lequel A représente un groupe $CH_2$ et B représente un atome d'oxygène ou un groupe $NR_4$, dans lequel $R_4$ est choisi entre un atome d'hydrogène, des groupes alkyle en $C_1$ ou $C_2$ et alcanoyle en $C_1$ ou $C_2$.

**3.** Composé suivant la revendication 1, dans lequel B représente un groupe $CH_2$ et A représente un atome d'oxygène ou un groupe $NR_4$, dans lequel $R_4$ est choisi entre un atome d'hydrogène, des groupes alkyle en $C_1$ ou $C_2$ et alcanoyle en $C_{1\ ou}$ $C_2$.

**4.** Composé suivant l'une quelconque des revendications 1 à 3, dans lequel $R_1$, $R_2$ et $R_3$ représentent tous des atomes d'hydrogène ; ou bien $R_1$ représente F et $R_2$, $R_3$ représentent l'un et l'autre un atome d'hydrogène ; ou bien $R_1$ et $R_2$ représentent l'un et l'autre un atome d'hydrogène et $R_3$ représente F.

**5.** Composé suivant l'une quelconque des revendications 1 à 4, dans lequel Z représente un groupe pyrrolidinyle lié par C, facultativement substitué avec un substituant alcanoyle en $C_1$ ou $C_2$.

**6.** Composé suivant l'une quelconque des revendications 1 à 4, dans lequel Z représente un groupe $NR_5R_6$, dans lequel $R_5$ représente un atome d'hydrogène ou un groupe alkyle en $C_1$ à $C_4$ et $R_6$ représente un groupe alkyle en $C_1$ à $C_4$, alkylsulfonyle en $C_1$ à $C_4$ ou Het-(alkyle en $C_1$ à $C_4$) dans lequel Het représente un noyau hétérocyclique penta-ou hexagonal saturé.

**7.** Composé suivant l'une quelconque des revendications 1 à 4, dans lequel Z représente un groupe $NR_5R_6$, dans lequel $R_5$ et $R_6$ forment un groupe azétidinyle ou pyrrolidinyle, dans lequel un des atomes de carbone dans le groupe azétidinyle ou pyrrolidinyle est facultativement remplacé par un groupe $SO_2$, et le groupe azétidinyle ou pyrrolidinyle est également facultativement substitué avec un ou deux groupes choisis entre des groupes oxo, hydroxyle et halogéno.

**8.** Composé suivant la revendication 1, qui est :

le 1-[4-(1-pyrrolidinylcarbonyl)phényl]-3-(trifluoro-méthyl)-1,4,6,7-tétrahydropyranno[4,3-c]pyrazole
la 1-({4-[3-(trifluorométhyl)-6,7-dihydropyranno[4,3-c]pyrazole-1(4*H*)-yl]phényl}méthyl)-2-pyrrolidinone
le 1-[4-(1-pyrrolidinylcarbonyl)phényl]-3-(trifluoro-méthyl)-1,4,5,7-tétrahydropyranno[3,4-c]pyrazole
la 1-({4-[3-(trifluorométhyl)-4,7-dihydropyranno[3,4-c]pyrazole-1(5*H*)-yl]phényl}méthyl)-2-pyrrolidinone
la 1-({4-[6-méthyl-3-(trifluorométhyl-4,5,6,7-tétra-hydro-1*H*-pyrazolo[3,4-c]pyridine-1-yl]phényl}méthyl)-2-pyrrolidinone
la 1-({4-[3-(trifluorométhyl)-4,5,6,7-tétrahydro-1H-pyrazolo[3,4-c]pyridine-1-yl]phényl}méthyl)-2-pyrrolidinone
la 1-({2,6-difluoro-4-[3-(trifluorométhyl)-6,7-dihydro-pyranno[4,3-c]pyrazole-1(4H)-yl]phényl}méthyl)-2-pyrrolidinone
la 1-({2-fluoro-4-[3-(trifuorométhyl)-4,7-dihydro-pyranno[3,4-c]pyrazole-1(5H)-yl]phényl}méthyl)-2-pyrrolidinone
la 1-({2-fluoro-4-[6-méthyl-3-(trifluorométhyl)-4,5,6,7-tétrahydro-1H-pyrazolo[3,4-c]pyridine-1-yl]phényl}-méthyl)-2-pyrrolidinone
le 1-[3-fluoro-4-(1-pyrrolidinylcarbonyl)phényl]-3-(trifluorométhyl)-1,4,5,7-tétrahydropyranno[3,4-c]pyrazole
la 1-({4-[5-méthyl-3-(trifluorométhyl)-4,5,6,7-tétra-hydro-1H-pyrazolo[4,3-c]pyridine-1-yl]phényl}méthyl)-2-pyrrolidinone
le 1-{4-[(1,1-dioxido-2-isothiazolidinyl)méthyl]-phényl}-3-(trifluorométhyl)-1,4,6,7-tétrahydropyranno[4,3-c]pyrazole
le 1-[4-(1-acétyl-2-pyrrolidinyl)phényl]-3-(trifluoro-méthyl)-1,4,6,7-tétrahydropyranno[4,3-c]pyrazole
la 1-[4-(1-azétidinylcarbonyl)phényl]-3-(trifluoro-méthyl)-1,4,5,7-tétrahydropyranno[3,4-c]pyrazole
la 1-({4-[3-(trifluorométhyl)-4,5,6,7-tétrahydro-1H-pyrazolo[4,3-c]pyridine-1-yl]phényl}méthyl)-2-pyrrolidinone
le 1-[4-(1-azétidinylcarbonyl)phényl]-3-(trifluoro-méthyl)-1,4,6,7-tétrahydropyranno[4,3-c]pyrazole
le N-éthyl-N-méthyl-4-[3-(trifluorométhyl)-6,7-dihydro-pyranno[4,3-c]pyrazole-1(4H)-yl]benzamide
le 1-[4-(1-azétidinylcarbonyl)-3-fluorophényl]-3-(trifluorométhyl)-1,4,5,7-tétrahydropyranno[3,4-c]pyrazole
le 1-{4-[(3,3-difluoro-1-azétidinyl)carbonyl]-3-fluoro-phényl}-3-(trifluorométhyl)-1,4,5,7-tétrahydropyranno[3,4-c]pyrazole
le 1-{4-[(3,3-difluoro-1-azétidinylcarbonyl]phényl}-3-(trifluorométhyl)-1,4,6,7-tétrahydropyranno[4,3-c]pyrazole
le 1-{4-[(3,3-difluoro-1-azétidinyl)carbonyl]phényl}-3-(trifluorométhyl)-1,4,5,7-tétrahydropyranno[3,4-c]pyrazole
le N-({4-[3-(trifluorométhyl)-6,7-dihydropyranno[4,3-c]pyrazole-1(4H)-yl]phényl)méthyl)méthanesulfonamide
le 1-{4-[(1,1-dioxido-2-isothiazolidinyl)méthyl]phényl}-3-(trifluorométhyl)-1,4,5,7-tétrahydropyranno[3,4-c]pyrazole
le N-({4-[3-(trifluorométhyl)-4,7-dihydropyranno[3,4-c]pyrazole-1(5H)-yl]phényl}méthyl)méthanesulfonamide
le 1-{4-[(1,1-dioxido-2-isothiazolidinyl)méthyl]phényl}-6-méthyl-3-(trifluorométhyl)-1,4,6,7-tétrahydro-1H-pyrazolo-[3,4-c]pyridine
le 1-[2-fluoro-4-(1-pyrrolidinylcarbonyl)phényl]-3-(trifluorométhyl)-1,4,6,7-tétrahydropyranno[4,3-c]pyrazole
la 1-({3-fluoro-4-[3-(trifluorométhyl)-4,7-dihydro-pyranno [3,4-c]pyrazole-1(5H)-yl]phényl}méthyl)-2-pyrrolidinone
la 1-({3-fluoro-4-[3-(trifluorométhyl)-6,7-dihydro-pyranno [4,3-c]pyrazole-1(4H)-yl]phényl}méthyl)-2-pyrrolidinone
le 1-{4-[(2-oxo-1-pyrrolidinyl)méthyl]phényl}-3-(tri-fluorométhyl)-1,4,5,7-tétrahydro-6H-pyrazolo[3,4-c]pyridine-6-carbaldéhyde
le N-(tétrahydro-2-furannylméthyl)-4-[3-(trifluoro-méthyl)-6,7-dihydropyranno[4,3-c]pyrazole-1(4H)-yl]benzène-sulfonamide
la 1-({2-fluoro-4-[3-(trifluorométhyl)-6,7-dihydro-pyranno [4,3-c]pyrazole-1(4H)-yl]phényl}méthyl)-2-pyrrolidinone
le 1-({2-fluoro-4-[3-(trifluorométhyl-6,7-dihydro-pyranno[4,3-c]pyrazole-1(4H)-yl]phényl}carbonyl)-3-azétidinol
le chlorhydrate de 1-({2-fluoro-4-[3-(trifluoro-méthyl)-4,5,6,7-tétrahydro-1H-pyrazolo[3,4-c]pyridine-1-yl]-phényl}méthyl)-2-pyrrolidinone
le 1-{4-[(3,3-difluoro-1-azétidinyl)carbonyl]-3-fluoro-phényl)-3-(trifluorométhyl)-1,4,6,7-tétrahydropyranno[4,3-c]pyrazole
le 1-{3-fluoro-4-[2-oxo-2-(1-pyrrolidinyl)éthyl]-phényl)-3-(trifluorométhyl-1,4,6,7-tétrahydropyranno[4,3-c]pyrazole
le 1-[3-fluoro-4-(1-pyrrolidinylcarbonyl)phényl]-3-(trifluorométhyl)-1,4,6,7-tétrahydropyranno[4,3-c]pyrazole
la 1-({4-[6-acétyl-3-(trifluorométhyl)-4,5,6,7-tétra-hydro-1H-pyrazolo[3,4-c]pyridine-1-yl-2-fluorophényl)méthyl)-2-pyrrolidinone
le 1-{3-fluoro-4-[2-oxo-2-(1-pyrrolidinyl)éthyl]-phényl}-3-(trifluorométhyl)-1,4,5,7-tétrahydropyranno[3,4-c]pyrazole

la 1-({2,6-difluoro-4-[3-(trifluorométhyl)-4,7-dihydro-pyranno [3,4-c]pyrazole-1 (5H) -yl]phényl}méthyl)-2-pyrrolidinone
le chlorhydrate de 1-[4-(1-pyrrolidinylcarbonyl)-phényl]-3-(trifluorométhyl)-4,5,6,7-tétrahydro-1H-pyrazolo-[3,4-c]pyridine
le1-{4-[(3,3-difluoro-1-azétidinyl)carbonyl]phényl}-3-(trifluorométhyl)-4,5,6,7-tétrahydro-1H-pyrazolo[3,4-c]-pyridine
le 1-[4-(1-azétidinylcarbonyl)phényl]-6-méthyl-3-(tri-fluorométhyl)-4,5,6,7-tétrahydro-1H-pyrazolo[3,4-c]pyridine
la 1-({4-[6-acétyl-3-(trifluorométhyl)-4,5,6,7-tétra-hydro-1H-pyrazolo[3,4-c]pyridine-1-yl]phényl}méthyl)-2-pyrrolidinone
ou un de ses sels ou produits de solvatation.

**9.** Composé suivant l'une quelconque des revendications 1 à 8, destiné à être utilisé comme médicament.

**10.** Composé suivant l'une quelconque des revendications 1 à 8, destiné à être utilisé dans le traitement de la schizophrénie.

**11.** Composition pharmaceutique comprenant un composé tel que défini dans l'une quelconque des revendications 1 à 8, et au moins un support ou diluant pharmaceutiquement acceptable.

**12.** Produit combiné, comprenant un composé tel que défini dans l'une quelconque des revendications 1 à 8 et un agent antipsychotique.

**13.** Utilisation d'un composé tel que défini dans l'une quelconque des revendications 1 à 8, ou d'une composition telle que définie dans la revendication 11, ou bien d'un produit tel que défini dans la revendication 12, dans la production d'un médicament destiné au traitement ou à la prévention de la schizophrénie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006015828 A
- ES 2159479


### Non-patent literature cited in the description


- **Watkins JC ; Krogsgaard-Larsen P ; Honore T.** *Trends Pharmacol Sci,* 1990, vol. 11, 25-33
- **Conn PJ ; Pinn JP.** *Ann Rev Pharmacol Toxicol,* 1997, vol. 37, 205-237
- **Dingledine R ; Borges K ; Bowie, Traynelis S.** *TRENDS PHARMACOL SCI,* 1999, vol. 51, 7-61
- **Sommer B ; Keinanen K ; Verdoon TA ; Wisden W ; Burnashev N ; Herb A ; Kohler M ; Takagi T ; Sakmann B ; Seeburg PH.** *Science,* 1990, vol. 249, 1580-1585
- **Burnachev N ; Monyer H ; Seeburg PH ; Sakmann B.** *Neuron,* 1992, vol. 8, 189-198
- **Weiss JH ; Sensi SL.** *Trends in Neurosci,* 2000, vol. 23, 365-371
- **Bliss TVP ; Collingridge GL.** *Nature,* 1993, vol. 361, 31-9
- **Arai A ; Guidotti A ; Costa E ; Lynch G.** *Neurore-port,* 1996, vol. 7, 2211-5
- **Staubli U ; Perez Y ; Xu F ; Rogers G ; Ingvar M ; Stone-Elander S ; Lynch G.** *Proc Natl Acad Sci,* 1994, vol. 91, 11158-11162
- **Zivkovic I ; Thompson DM ; Bertolino M ; Uzunov D ; DiBella M ; Costa E ; Guidotti A.** *JPET,* 1995, vol. 272, 300-309
- **Lebrun C ; Pilliere E ; Lestage P.** *Eu J Pharmacol,* 2000, vol. 401, 205-212
- **Thompson DM ; Guidotti A ; DiBella M ; Costa E.** *Proc Natl Acad Sci,* 1995, vol. 92, 7667-7671
- **Ingvar M ; Ambros-Ingerson J ; Davis M ; Granger R ; Kessler M ; Rogers GA ; Schehr RS ; Lynch G.** *Exp Neurol,* 1997, vol. 146, 553-559
- **Greene T.W.** Protective groups in organic synthesis. Wiley, 1981
- Liebigs Annalen Der Chemie. 1984, vol. 11, 1759-1882
- Diagnostic and Statistical Manual of Mental Disorders. American Psychiatric Association